# EUROPÄISCHE PATENTSCHRIFT

(11) **EP 0 237 929 B1**
(45) Veröffentlichungstag und Bekanntmachung des Hinweises auf die Patenterteilung: **02.06.1993**
(21) Anmeldenummer: 87103469.0
(22) Anmeldetag: 11.03.1987
(51) Int. Cl.: C07C 205/16, C07D 213/50, C07D 265/28, C07D 241/38, C07D 233/64, C07C 255/50, C07D 417/00, C07D 471/04

(54) **3,5-Disubstituierte Pyrocatecholderivate**
3,5-Disubstituted pyrocatechol derivatives
Dérivés du pyrocatéchol substitués en 3,5

(30) Priorität: 11.03.1986 CH 980/86; 09.01.1987 CH 62/87
(43) Veröffentlichungstag der Anmeldung: 23.09.1987
(73) Patentinhaber: F. HOFFMANN-LA ROCHE AG, 4002 Basel (CH)
(72) Erfinder: Bernauer, Karl Prof. Dr., CH-4104 Oberwil (CH); Borgulya, Janos, Dr., CH-4058 Basel (CH); Bruderer, Hans, Dr., CH-4105 Biel-Benken (CH); Da Prada. Mosé, Prof. Dr., CH-4125 Riehen (CH); Zürcher, Gerhard, CH-4058 Basel (CH)
(74) Vertreter: Lederer, Franz, Dr.

(56) Entgegenhaltungen:
- EP-A- 0 142 283
- GB-A- 2 038 819
- Chemical Abstracts, Band 92, Nr. 17, 28. April 1980, Colombus, Ohio, USA; S. Umemura et al. "3-Cyanocatechol derivatives", Seite 560, Zusammenfassung Nr. 146 461x
- Chemical Abstracts, Band 98, Nr. 7, 14. Februar 1983, Colombus, Ohio, USA; P. Traxler et al.: "A genetic approach to the biosynthesis of the rifamycin-chromophore in Norcadia mediterranei. V. Studies on the biogenetic origin of 3-substituents", Seite 360, Zusammenfassung Nr. 50 111f
- Chemical Abstracts, Band 98, Chemical Substance Index, A-B, Jänner-Juni 1983, Colombus, Ohio, USA
- Registry Handbook, Chemical Abstracts Service, 1965-1971, Colombus, Ohio, USA

## Beschreibung

Die Chatecholderivate der allgemeinen Formel 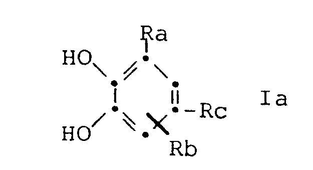
worin Ra Nitro oder Cyano, Rb Wasserstoff oder Halogen, Rc Halogen, Nitro, Cyano oder die Gruppe -(A)ₙ-(Q)ₘ-R¹ oder -(A)ₙ-Q-R², A gegebenenfalls durch niederes Alkyl substituiertes Vinylen, n die Zahl 0 oder 1, m die zahl 0 oder 1, R¹ die Gruppe -COR³, eine carbocyclische, aromatische Gruppe oder eine über ein Kohlenstoffatom gebundene, aromatische oder partiell ungesättigte, heterocyclische Gruppe, R² Wasserstoff oder einen gegebenenfalls substituierten, gesättigten oder partiell ungesättigten, niederen Kohlenwasserstoffrest, R³ Hydroxy, Amino, einen über ein Sauerstoffatom oder eine Imino- oder niedere Alkyliminogruppe gebundenen, gegebenenfalls substituierten, gesättigten oder partiell ungesättigten, niederen Kohlenwasserstoffrest oder eine über ein Ringstickstoffatom gebundene, gesättigte. N-haltige heterocyclische Gruppe, Q die Gruppe -CO- oder >C=N-(Z)ₚ-R⁴, Z ein Sauerstoffatom oder eine Iminogruppe, p die Zahl 0 oder 1 und R⁴ Wasserstoff oder einen gegebenenfallS substituierten und gegebenenfalls über eine Carbonylgruppe gebundenen, gesättigten oder partiell ungesättigten, niederen Kohlenwasserstoffrest bedeuten,
die unter physiologischen Bedingungen hydrolysierbaren Ester- und Aetherderivate und die pharmazeutisch annehmbaren Salze davon besitzen wertvolle pharmakologische Eigenschaften. Diese Verbindungen hemmen insbesondere das Enzym Catechol-O-Methyl-Transferase (COMT), ein lösliches. Magnesium-abhängiges Enzym, das die Uebertragung der Methylgruppe von S-Adenosylmethionin auf ein Catecholsubstrat katalysiert, wobei die entsprechenden Methyläther gebildet werden. Geeignete Substrate, welche durch COMT O-methyliert und damit desaktiviert werden, sind beispielsweise extraneuronale Catecholamine und exogen verabreichte therapeutische Wirkstoffe mit Catecholstruktur.

Die obigen Verbindungen der Formel Ia können demnach bei der Verhütung oder Bekämpfung von Krankheiten verwendet werden, bei welchen eine Desaktivierung von extraneuronalen Catecholaminen durch COMT eine Rolle spielt, beispielsweise bei der Verhütung oder Bekämpfung von Depressionen. In diesem Fall können die Verbindungen der obigen Formel Ia als Einzelverbindungen oder in Kombination mit anderen therapeutischen Wirkstoffen, welche den Krankheitsverlauf günstig beeinflussen, verwendet werden. Die Verbindungen der Formel Ia können aber auch als Comedikation zu anderen therapeutischen Wirkstoffen verwendet werden.

Die Verbindungen der Formel Ia können aber auch verwendet werden, um die Verhütung oder Bekämpfung von Krankheiten mit therapeutischen Wirkstoffen, welche eine Catecholstruktur aufweisen, zu verbessern. Als Beispiel sei die Behandlung der Parkinson-Krankheit und von Parkinsonismus mit L-Dopa, einem therapeutischen Wirkstoff mit Catecholstruktur, erwähnt. In solchen Fällen können die Verbindungen der Formel Ia in Form einer Comedikation oder als Kombinationspräparate verwendet werden.

Eine weitere Möglichkeit der Verwendung der obigen Verbindungen der Formel Ia bietet sich auf dem Gebiet der Diagnostik. Nach Verabreichung von [¹⁸F]-6-Fluor-L-Dopa kann [¹⁸F]--Dopamin im Hirn mit Hilfe der Positronenemissionstomographie sichtbar gemacht werden. Durch Zusatz einer Verbindung der obigen Formel Ia wird die COMT gehemmt und damit die Bildung von [¹⁸F]-3-O-Methyldopa verhindert. In Abwesenheit eines COMT-Hemmers, würde das gebildete [¹⁸F]-3-O-Methyldopa ins Hirn penetrieren und zu einem stark erhöhten Background führen, was die Diagnostik stark erschweren würde.

Die obige Formel Ia umfasst sowohl bekannte als auch neue Verbindungen. Die an sich bekannten Verbindungen der Formel Ia fallen unter die allgemeine Formel 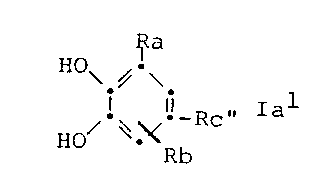
worin Ra Nitro oder Cyano, Rb Wasserstoff oder Halogen, Rc'' Nitro, Cyano oder die Gruppe -(A)ₙ-COOH oder -(A)ₙ-Q-H, A gegebenenfalls durch niederes Alkyl substituiertes Vinylen, n die Zahl 0 oder 1, Q die Gruppe -CO- oder >C=N-(Z)ₚ-R⁴, Z ein Sauerstoffatom oder eine Iminogruppe, p die Zahl 0 oder 1 und R⁴ Wasserstoff oder einen gegebenenfalls substituierten und gegebenenfalls über eine Carbonylgruppe gebundenen, gesättigten oder partiell ungesättigten, niederen Kohlenwasserstoffrest bedeuten, wobei Ra Nitro bedeutet. wenn Rc'' Cyano oder Nitro bedeutet,
unter physiologischen Bedingungen hydrolysierbare Ester- und Aetherderivate und pharmazeutisch annehmbare Salze davon.

Ähnliche Phenylethylamine werden in der EP-A-142 283 offenbart. In der GB-A-2 038 819 wird das 3,5-Dinitropyrocatechol im Zusammenhang mit der Herstellung von Explosivstoffen beschrieben. In Chem. Abstr. 92, 146 461x werden u.a. das 5-Chlor-2,3-dihydroxybenzonitril und das 4,5-Dichlor-2,3-dihydroxybenzonitril offenbart. In J. Antibiot. 35, 1361-1366 (1982) wird die Herstellung und Reduktion von 3,4-Dihydroxy-5-nitrobenzoesäure beschrieben. Chem. Abstr. 29, 5442¹ (1935) betrifft die Herstellung von 3,4-Dihydroxy-5-nitro-Zimtsäure.

Bei den neuen Verbindungen der Formel Ia handelt es sich um die Verbindungen der allgemeinen Formel 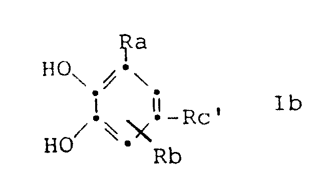
worin Ra Nitro oder Cyano, Rb Wasserstoff oder Halogen, Rc' Nitro, Cyano oder die Gruppe -(A)ₙ-(Q)ₘ-R¹¹ oder -(A)ₙ-Q-R²¹, A gegebenenfalls durch niederes Alkyl substituiertes Vinylen, n die Zahl 0 oder 1, m die Zahl 0 oder 1, R¹¹ die Gruppe -COR³¹, eine carbocyclische, aromatische Gruppe oder eine über ein Kohlenstoffatom gebundene, aromatische oder partiell ungesättigte, heterocyclische Gruppe, R²¹ einen gegebenenfalls substituierten, gesättigten oder partiell ungesättigten, niederen Kohlenwasserstoffrest, R³¹ Hydroxy, Amino, einen über ein Sauerstoffatom oder eine Imino- oder niedere Alkyliminogruppe gebundenen, gegebenenfalls substituierten, gesättigten oder partiell ungesättigten, niederen Kohlenwasserstoffrest oder eine über ein Ringstickstoffatom gebundene, gesättigte, N-haltige heterocyclische Gruppe, Q die Gruppe -CO- oder >C=N-(Z)ₚ-R⁴, Z ein Sauerstoffatom oder eine Iminogruppe, p die Zahl 0 oder 1 und R⁴ Wasserstoff oder einen gegebenenfalls substituierten und gegebenenfalls über eine Carbonylgruppe gebundenen, gesättigten oder partiell ungesättigten, niederen Kohlenwasserstoffrest bedeuten, wobei Ra Cyano bedeutet, wenn Rc' Cyano oder Nitro bedeutet, und R³¹ eine von Hydroxy verschiedene Bedeutung hat, wenn m die Zahl 0 bedeutet,
und um die unter physiologischen Bedingungen hydrolysierbaren Ester- und Aetherderivate und um die pharmazeutisch annehmbaren Salze davon.

Gegenstand der vorliegenden Erfindung sind: Die obigen Verbindungen der Formel Ia und die erwähnten Derivate davon zur Verwendung als therapeutische Wirkstoffe; Arzneimittel auf der Basis dieser Verbindungen und Derivate; die Herstellung derartiger Arzneimittel; die Verwendung der fraglichen Verbindungen und Derivate bei der Verhütung oder Bekämpfung von Krankheiten; die Verwendung der fraglichen Verbindungen und Derivate zur Herstellung von Arzneimitteln, welche gegebenenfalls im Sinne einer erwünschten Nebenwirkung das Enzym COMT hemmen; die Verbindungen der obigen Formel Ib und die erwähnten Derivate davon als solche; die Herstellung dieser Verbindungen und Derivate; sowie Zwischenprodukte für deren Herstellung.

Der Ausdruck "nieder" bezeichnet Reste und Verbindungen mit höchstens 7, vorzugsweise höchstens 4 Kohlenstoffatomen. Der Ausdruck "Alkyl" für sich allein genommen oder in Zusammensetzungen, wie "Alkylgruppe" , "Alkoxy", "Alkylthio" und "Alkylimino", bezeichnet geradkettige oder verzweigte, gesättigte Kohlenwasserstoffreste, wie Methyl, Aethyl, Propyl, Isopropyl, n-Butyl, s-Butyl, i-Butyl und t-Butyl. Der Ausdruck "gesättigter oder partiell ungesättigter, niederer Kohlenwasserstoffrest" bezeichnet offenkettige und cyclische Gruppen und Kombinationen davon. Beispiele für gesättigte und partiell ungesättigte niedere Kohlenwasserstoffreste sind: Niedere Alkylgruppen wie sie oben definiert sind; niedere Alkenylgruppen, wie 2-Propenyl, 2-Butenyl, 3-Butenyl und 2-Methyl-2-propenyl; gegebenenfalls durch niedere Alkylgruppen substituierte C₃₋₇-Cylcoalkyl- und C₈₋₁₀-Bicycloalkylgruppen, wie Cyclopropyl, Cyclopentyl, 2-Methylcyclopentyl, Cyclohexyl und 3-Methylcyclohexyl; gegebenenfalls durch niedere Alkylgruppen substituierte niedere Cycloalkenylgruppen, wie 3-Cyclopentenyl, 1-Methyl-3-cyclopentenyl und 3-Cyclohexenyl; durch niedere Cycloalkyl- oder Cycloalkenylgruppen substituierte niedere Alkyl- oder Alkenylgruppen, wie Cyclopropylmethyl, Cyclopropyläthyl, Cyclopentylmethyl, Cyclohexylmethyl, 2-Cyclohexenylmethyl und 3-Cyclopropyl-2-propenyl. Die niederen Alkenylgruppen enthalten vorzugsweise 2-4 Kohlenstoffatome; die Cycloalkyl- und Cycloalkenylgruppen enthalten vorzugsweise 3-6 Kohlenstoffatome.

Als Substituenten für die obigen niederen Kohlenwasserstoffreste kommen die folgenden in Frage: Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino und niederes Alkylthio. Die gesättigten oder partiell ungesättigten, niederen Kohlenwasserstoffreste sind vorzugsweise unsubstituiert oder mono- oder disubstituiert.

Der Ausdruck "Aryl" bezeichnet carbocyclische aromatische Gruppen, und zwar vorzugsweise mono- oder bicyclische Gruppen. Besonders bevorzugte carbocyclische aromatische Gruppen sind Phenyl- und Naphthylgruppen, insbesondere Phenylgruppen. Diese Gruppen sind gegebenenfalls substituiert durch: Halogen, Trifluormethyl, Nitro, Amino, mono- oder di(niederes Alkyl)amino, niederes Alkyl, niederes Alkoxy, niederes Alkylthio, niederes Alkanoyl, niederes Alkoxycarbonyl, Carboxy, Hydroxy, Cyano, niederes Alkanoyloxy, Carbamoyl, mono- oder di(niederes Alkyl)carbamoyl, niederes Alkylendioxy, niederes Alkanoylamino oder niederes Alkoxycarbonylamino. Die carbocyclischen aromatischen Gruppen sind vorzugsweise unsubstituiert oder mono- oder disubstituiert.

Der Ausdruck "aromatische oder partiell ungesättigte, heterocyclische Gruppe" bezeichnet vorzugsweise eine mono-, di- oder tricyclische, aromatische oder partiell ungesättigte, heterocyclische Gruppe mit bis zu 5 Heteroatomen aus der Gruppe, bestehend aus Stickstoff, Schwefel und Sauerstoff. Diese heterocyclischen Gruppen enthalten vorzugsweise 1-4 Stickstoffatome und/oder ein Sauerstoff- oder Schwefelatom. Sie sind vorzugsweise mono- oder bicyclisch. Die Heteroatome sind vorzugsweise auf einen oder zwei Ringe verteilt, wobei Stickstoffatome gleichzeitig auch Bestandteil von 2 Ringen sein können. Die heterocyclischen Gruppen sind vorzugsweise aromatisch. Sie können substituiert sein, wobei sie in diesem Fall vorzugsweise mono-, di- oder trisubstituiert sind. Als Substituenten kommen in Frage: Halogen, Trifluormethyl, Nitro, Carboxy, Amino, Arylamino, niederes Alkyl, niederes Alkoxy, Hydroxy, niederes Alkoxycarbonyl, niederes Alkanoyl, niederes Alkanoyloxy, Oxo, niederes Alkylendioxy, Mercapto, niederes Alkylthio, niederes Alkylamino, di(niederes Alkyl)amino, C₃₋₇-Cycloalkylamino, C₈₋₁₀-Bicycloalkylamino, niederes Alkanoylamino, niederes Alkoxycarbonylamino, Carbamoyl, mono- oder di(niederes Alkyl)carbamoyl, Cyano, Aryl, Aryl-niederes Alkyl, Aryl-niederes Alkylamino, Heteroaryl, Heteroaryl-niederes Alkyl, Heteroarylamino und C₃₋₇-Cycloalkyl. Die monocyclischen heterocyclischen Gruppen sind vorzugsweise 5- oder 6-gliedrig und enthalten höchstens vier Heteroatome. Die bicyclischen heterocyclischen Gruppen sind vorzugsweise 8- bis 10-gliedrig, wobei die einzelnen Ringe vorzugsweise 5- oder 6-gliedrig sind.

Als Beispiele für derartige heterocyclischen Gruppen seien die folgenden erwähnt: Pyridyl, Pyrazinyl, Triazinyl, Thiadiazinyl, Thiazolyl, Oxazolyl, Oxadiazolyl, Pyrazolyl, Tetrazolyl, Imidazolyl, Thienyl, Chinolinyl, Isochinolinyl, Dihydroisochinolinyl, Benzoxazinyl, Chinoxalinyl, Benzopyranyl, Benzimidazolyl, Indolyl, Imidazothiazolyl, Imidazothiadiazolyl, Imidazopyridyl, Benzothiazinyl, Benzochinoxalinyl und Imidazobenzothiazolyl.

Der Ausdruck "Heteroaryl" bezeichnet aromatische, heterocyclische Gruppen wie sie oben definiert sind.

Der Ausdruck "eine über eine Ringstickstoffatom gebundene, gesättigte, N-haltige heterocyclische Gruppe" bezeichnet vorzugsweise einen 3- bis 7-gliedrigen, vorzugsweise 4- bis 6-gliedrigen, gesättigten N-Heterocyclus, der neben dem besagten Stickstoffatom noch ein Sauerstoff-, Schwefel- oder Stickstoffatom als zweites Heteroatom enthalten kann. Diese gesättigten N-Heterocyclen können noch mono- oder disubstituiert sein durch: niederes Alkyl, Hydroxy, niederes Alkoxy, niederes Alkanoyloxy, niederes Hydroxyalkyl, niederes Alkoxyalkyl, niederes Alkanoyloxyalkyl, niederes Alkoxycarbonyl, niederes Alkanoyl, Carbamoyl, mono- oder di(niederes Alkyl)carbamoyl, Oxo und/oder niederes Alkylendioxy.

Als Beispiele für derartige N-haltige heterocyclische Gruppen seien die folgenden erwähnt: 4-Morpholinyl, 1-Pyrrolidinyl und 1-Azetidinyl.

Bei den unter physiologischen Bedingungen hydrolysierbaren Ester- und Aetherderivaten handelt es sich vorzugsweise um Verbindungen der Formel Ia, worin mindestens eines der beiden phenolischen Hydroxygruppen durch eine niedere Fettsäure acyliert oder durch eine niedere 1-Alkoxycarbonyloxy-1-alkyl-, niedere 1-Alkanoyloxy-1-alkyl- oder durch eine niedere 2-Oxo-1-alkylgruppe veräthert ist.

Der Substituent Ra bedeutet vorzugsweise Nitro. Der Substituent Rb befindet sich vorzugsweise in der p-Stellung zum Substituenten Ra und bedeutet vorzugsweise Wasserstoff, Chlor oder Fluor, wobei die Bedeutungsmöglichkeit Wasserstoff besonders bevorzugt ist. Der Substituent Rc' bedeutet vorzugsweise die Gruppe -CO-R¹¹, wobei R¹¹ eine aromatische, einkernige, carbocyclische Gruppe oder eine über ein Kohlenstoffatom gebundene, aromatische, einkernige heterocyclische Gruppe mit 1-3 Stickstoffatomen als Heteroringglieder bedeutet. In einer besonders bevorzugten Ausführungsform bedeutet R¹¹ eine gegebenenfalls durch Halogen, Trifluormethyl, Cyano, Hydroxy oder niederes Alkyl mono- oder disubstituierte Phenylgruppe oder eine Pyridylgruppe.

Im Rahmen der vorliegenden Erfindung ganz besonders bevorzugte Verbindungen sind:
3,4-Dihydroxy-5-nitrobenzophenon,
2'-Fluor-3,4-dihydroxy-5-nitrobenzophenon und
3,4-Dihydroxy-5-nitrophenyl-4-pyridylketon.

Die Verbindungen der Formel Ib, die unter physiologischen Bedingungen hydrolysierbaren Ester- und Aetherderivate und die pharmazeutisch annehmbaren Salze davon können erfindungsgemäss dadurch hergestellt werden, dass man
a) in einer Verbindung der allgemeinen Formel 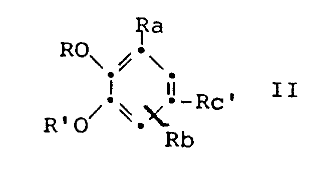 worin eines der Symbole R und R' niederes Alkyl und das andere Wasserstoff oder niederes Alkyl bedeuten, und Ra, Rb und Rc' obige Bedeutung besitzen,
die niedere(n) Alkyläthergruppe(n) spaltet, oder
b) eine Verbindung der allgemeinen Formel 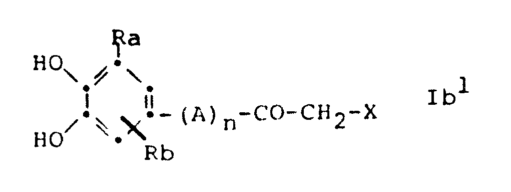 worin X eine Abgangsgruppe bedeutet, und Ra, Rb, A und n obige Bedeutung besitzen,
mit einem Thioamid, Thioharnstoff, Thiocarbonsäurehydrazid, Thiosemicarbazid, Amidin, Guanidin, Amidrazon, Aminoguanidin, cyclischen Amidin, 1,2-Diamin, 1,2-Aminothiol oder einem 1,2-Aminoalkohol umsetzt und das erhaltene Cyclokondensationsprodukt erwünschtenfalls dehydriert, oder
c) eine Verbindung der allgemeinen Formel 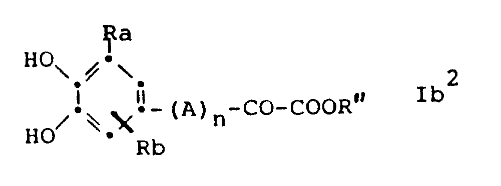 worin R'' niederes Alkyl bedeutet, und Ra, Rb, A und n obige Bedeutung besitzen,
mit einem 1,2-Diamin, 1,2-Aminothiol, 1,2-Aminoalkohol, Semicarbazid, Thiosemicarbazid, Amidrazon oder einem Aminoguanidin umsetzt und das erhaltene Cyclokondensationsprodukt erwünschtenfalls dehydriert, oder
d) eine Verbindung der obigen Formel Ib¹ mit einer β-Aminocarbonylverbindung umsetzt, oder
e) in einer Verbindung der allgemeinen Formel 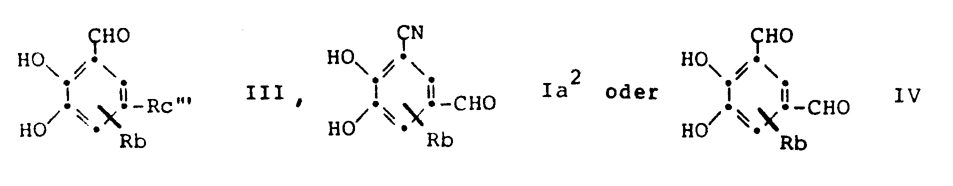 worin Rc''' Nitro, Cyano oder die Gruppe -(A)ₙ-R¹² und R¹² die Gruppe -COR³¹, eine carbocyclische, aromatische Gruppe oder eine über ein Kohlenstoffatom gebundene, aromatische oder partiell ungesättigte, heterocyclische Gruppe bedeuten, und Ra, Rb, A, n und R³¹ obige Bedeutung besitzen,
oder in einem Di-O-niederen Alkanoylderivat davon die Carboxaldehydgruppe(n) in die Cyanogruppe(n) überführt, oder
f) ein Di-O-niederes Alkanoylderivat einer Carbonsäure der allgemeinen Formel 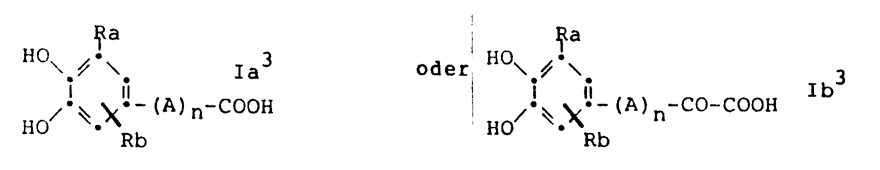 worin Ra, Rb, A und n obige Bedeutung besitzen,
in Gegenwart eines Kondensationsmittels oder ein reaktives Derivat eines Di-O-niederen Alkanoylderivates einer Carbonsäure der Formel Ia³ oder Ib³ mit einer Verbindung der allgemeinen Formel

HO-R⁵ V oder HNR⁶R⁷ VI

worin R⁵ einen gegebenenfalls substituierten, gesättigten oder partiell ungesättigten, niederen Kohlenwasserstoffrest, R⁶ Wasserstoff oder niederes Alkyl und R⁷ Wasserstoff oder einen gegebenenfalls substituierten, gesättigten oder partiell ungesättigten, niederen Kohlenwasserstoffrest oder R⁶ und R⁷ zusammen mit dem Stickstoffatom eine gesättigte N-haltige heterocyclische Gruppe bedeuten,
umsetzt, oder
g) eine Verbindung der obigen Formel Ib² oder der allgemeinen Formel 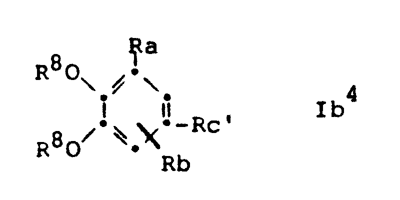 worin R⁸ niederes Alkanoyl bedeutet, und Ra, Rb und Rc' obige Bedeutung besitzen,
hydrolysiert, oder
h) eine Verbindung der allgemeinen Formel 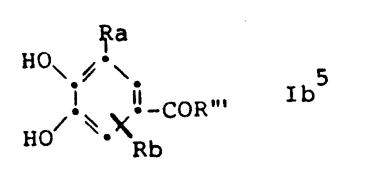 worin Ra und Rb obige Bedeutung besitzen, und R''' Wasserstoff oder niederes Alkyl bedeutet,
oder ein Di-O-niederes Alkanoylderivat davon in Gegenwart eines sekundären Amins mit einer Verbindung der allgemeinen Formel

CH₃CO-R²³ VII

worin R²³ einen gegebenenfalls substituierten, gesättigten oder partiell ungesättigten, niederen Kohlenwasserstoffrest bedeutet,
umsetzt, oder
i) eine Verbindung der allgemeinen Formel 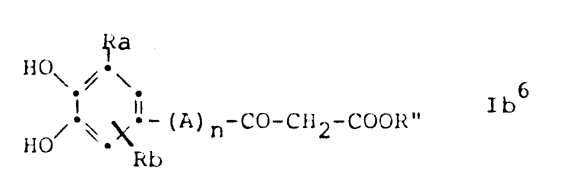 worin Ra, Rb, A, n und R'' obige Bedeutung besitzen,
mit einem Hydrazin oder einem Amidin umsetzt, oder
j) eine Verbindung der obigen Formel Ib, worin m die Zahl 1 und Q die Gruppe -CO- bedeuten,
mit einer Verbindung der allgemeinen Formel

H₂N-(Z)ₚ-R⁴ VIII

worin Z, p und R⁴ obige Bedeutung besitzen, umsetzt, und erwünschtenfalls
k) eine Verbindung der obigen Formel Ib in ein unter physiologischen Bedingungen hydrolysierbares Ester- oder Aetherderivat oder in ein pharmazeutisch annehmbares Salz davon überführt.

Gemäss Verfahrensvariante a) können die Verbindungen der Formel Ib dadurch hergestellt werden, dass man in einer Verbindung der Formel II die Aethergruppe(n) spaltet. Diese Aetherspaltung kann nach an sich bekannten und jedem Fachmann geläufigen Methoden durchgeführt werden. Die Aetherspaltung kann beispielsweise durch Behandeln mit Bromwasserstoff in einem geeigneten Lösungsmittel bewerkstelligt werden. Geeignete Lösungsmittel sind beispielsweise Wasser, Essigsäure und Mischungen davon. Man arbeitet vorzugsweise bei erhöhter Temperatur, beispielsweise in einem Temperaturbereich von etwa 100°C bis zur Siedetemperatur des Reaktionsgemisches. vorzugsweise verwendet man 48-proz. Bromwasserstoffsäure oder Mischungen davon mit Essigsäure.

Die Aetherspaltung kann auch durch Behandeln mit Bortribromid in einem geeigneten Lösungsmittel bei Temperaturen von etwa -60°C bis etwa Raumtemperatur bewerkstelligt werden. Geeignete Lösungsmittel sind insbesondere halogenierte niedere Kohlenwasserstoffe, wie Methylenchlorid, Chloroform und dergleichen. Weitere geeignete Methoden sind: Behandeln mit Pyridiniumhydrochlorid bei Temperaturen von etwa 150°C bis etwa 250°C und Behandeln mit Natriumjodid/Siliciumtetrachlorid in einem inerten organischen Lösungsmittel bei erhöhter Temperatur, beispielsweise bei der Rückflusstemperatur der Reaktionsmischung. Für das letzte Verfahren geeignete Lösungsmittel sind beispielsweise Acetonitril, aromatische Kohlenwasserstoffe wie Benzol oder Toluol, Mischungen davon und dergleichen.

Gemäss Verfahrensvariante b) können Verbindungen der allgemeinen Formel 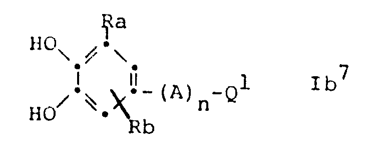
hergestellt werden, worin Ra, Rb, A und n obige Bedeutung besitzen, und Q¹ eine Gruppe der Formel 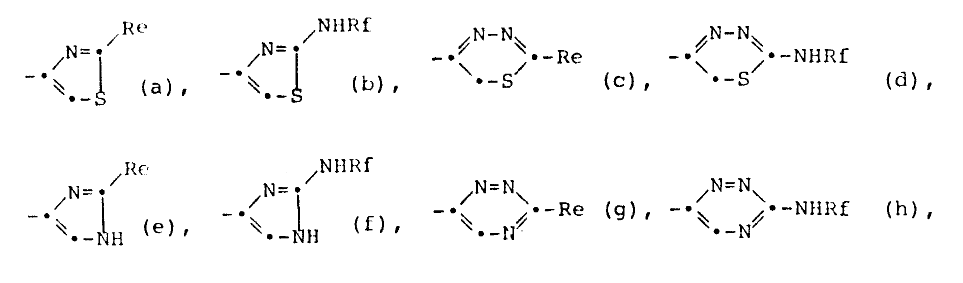 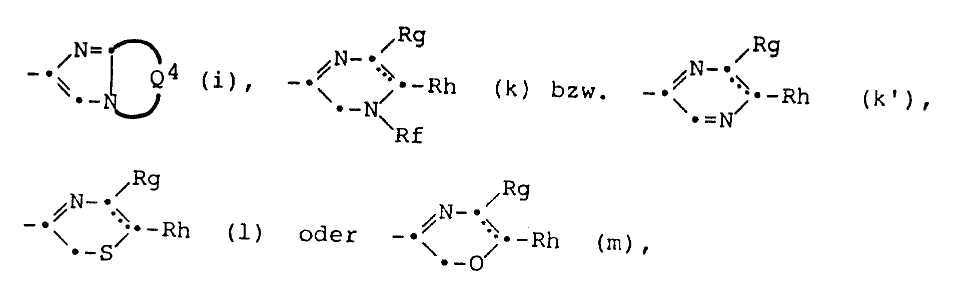
Re Wasserstoff, niederes Alkyl, C₃₋₇-Cycloalkyl, Aryl, Heteroaryl, Aryl-niederes Alkyl oder Heteroaryl-niederes Alkyl, Rf Wasserstoff, Aryl, Aryl-niederes Alkyl, niederes Alkyl, niederes Alkoxycarbonyl, Heteroaryl, Heteroaryl-niederes Alkyl, C₈₋₁₀-Bicycloalkyl oder C₃₋₇-Cycloalkyl, Rg und Rh je Wasserstoff, Cyano, niederes Alkyl, C₃₋₇-Cycloalkyl, Aryl, Aryl-niederes Alkyl, Heteroaryl oder Heteroaryl-niederes Alkyl, oder Rg und Rh zusammen mit den beiden Kohlenstoffatomen, an welche sie gebunden sind, eine carboxyclische aromatische Gruppe oder eine aromatische oder partiell ungesättigte, heterocyclische Gruppe, die gestrichelte Linie eine fakultative Bindung und Q⁴ zusammen mit dem Kohlenstoff- und dem Stickstoffatom eine aromatische oder partiell ungesättigte, heterocyclische Gruppe, welche mindestens ein Stickstoffatom als Heteroringglied enthaltet, bedeuten.

Geeignete Lösungsmittel für diesen Verfahrensaspekt sind niedere Alkohole, wie Aethanol, n-Butanol, n-Hexanol und Aethylenglykol, offenkettige und cyclische Aether, welche noch freie Hydroxygruppen enthalten können, wie Tetrahydrofuran, Dioxan, t-Butylmethyläther Aethylenglykoldimethyläther, Diäthylenglykoldimethyläther, Aethylenglykolmonomethyläther und Diäthylenglykolmonomethyläther, Acetonitril, Dimethylformamid, Dimethylacetamid und Dimethylsulfoxid. Die gewünschte Reaktion kann auch ohne Lösungsmittel durch trockenes Erwärmen der Reaktionspartner bewerkstelligt werden. Die Reaktion wird vorzugsweise bei erhöhter Temperatur, beispielsweise in einem Bereich von etwa 50°C bis 150°C, durchgeführt, wobei man vorzugsweise bei der Siedetemperatur der Lösungsmittels arbeitet, sofern man in Gegenwart einer Lösungsmittels arbeitet und der Siedepunkt im vorerwähnten Bereich liegt.

Gemäss Verfahrensvariante c) können Verbindungen der allgemeinen Formel 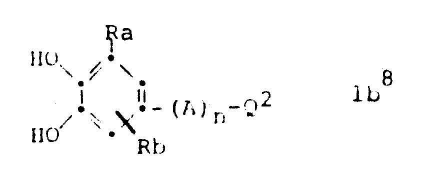
hergestellt werden, worin Ra, Rb, A und n obige Bedeutung besitzen und Q² eine Gruppe der Formel 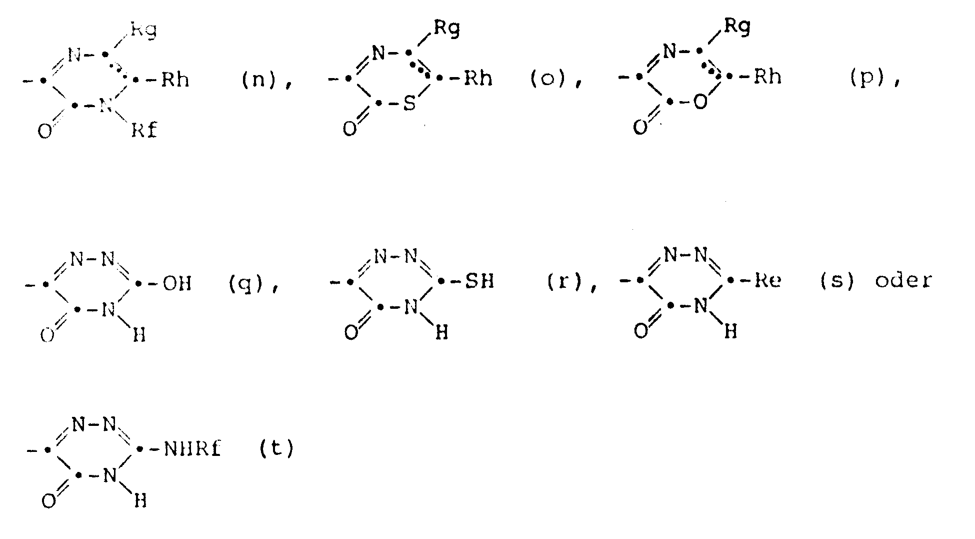
bedeutet, wobei Re, Rf, Rg, Rh und die gestrichelte Linie obige Bedeutung besitzen.

Die Umsetzung gemäss Verfahrensvariante c) kann unter den gleichen Reaktionsbedingungen durchgeführt werden wie die Verfahrensvariante b).

Gemäss Verfahrensvariante d) können Verbindungen der allgemeinen Formel 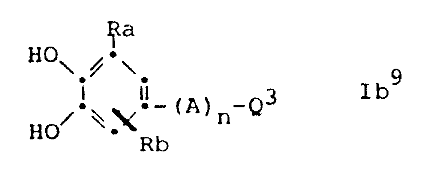
hergestellt werden, worin Q³ die Gruppe der Formel 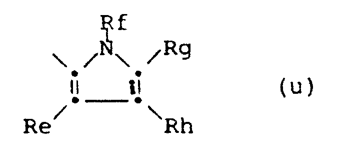
bedeutet, und Ra, Rb, Re, Rf, Rg, Rh, A, n und die gestrichelte Linie obige Bedeutung besitzen.

Auch die Verfahrensvariante d) kann unter den gleichen Reaktionsbedingungen durchgeführt werden wie die Verfahrensvariante b).

Gemäss Verfahrensvariante e) können Verbindungen der Formel Ib hergestellt werden, worin Ra Cyano, Rc' Nitro, Cyano oder die Gruppe -(A)ₙ-R¹² und R¹² die Gruppe -COR³¹, eine carbocyclische, aromatische Gruppe oder eine über ein Kohlenstoffatom gebundene, aromatische oder partiell ungesättigte, heterocyclische Gruppe bedeuten, und Rb, A, n und R³¹ obige Bedeutung besitzen. Die Ueberführung der Carboxaldehydgruppe(n) in die Cyanogruppe(n) kann nach an sich bekannten und jedem Fachmann geläufigen Methoden erfolgen. Man kann beispielsweise eine Verbindung der Formel Ia², III oder IV mit Hydroxylamin-O-sulfonsäure bei erhöhter Temperatur behandeln, wobei man vorzugsweise Wasser als Lösungsmittel verwendet. Die Reaktion kann in einem Temperaturbereich von etwa 50°C bis etwa 100°C durchgeführt werden.

Gemäss Verfahrensvariante f) können Di-O-niedere Alkanoylderivate von Verbindungen der Formel Ib hergestellt werden, worin Rc' die Gruppe -(A)ₙ-(CO)ₘ-COR³² und R³² Amino, einen über ein Sauerstoffatom oder eine Imino- oder niedere Alkyliminogruppe gebundenen, gegebenenfalls substituierten, gesättigten oder partiell ungesättigten, niederen Kohlenwasserstoffrest oder eine über ein Ringstickstoffatom gebundene, gesättigte, N-haltige heterocyclische Gruppe bedeuten, und A, n und m obige Bedeutung besitzen. Auch diese Reaktion kann nach an sich bekannten und jedem Fachmann geläufigen Methoden durchgeführt werden. Niedere Alkylester können beispielsweise hergestellt werden, indem man die Carbonsäure in Gegenwart einer Säure mit dem entsprechenden niederen Alkohol behandelt, wobei man als Lösungsmittel vorzugsweise den entsprechenden niederen Alkohol verwendet. Geeignete Säuren sind beispielsweise Mineralsäuren, wie Chlorwasserstoff, und organische Sulfonsäuren, wie p-Toluolsulfonsäure. Die Reaktionstemperatur liegt vorzugsweise in einem Bereich von Raumtemperatur bis zur Siedetemperatur des gewählten Lösungsmittels.

Die übrigen Ester und die Amide werden vorzugsweise ausgehend von reaktiven Carbonsäurederivaten hergestellt. Geeignete reaktive Carbonsäurederivate sind beispielsweise die entsprechenden Carbonsäurehalogenide, insbesondere die Carbonsäurechloride, entsprechende Carbonsäureanhydride und gemischte Anhydride (z.B. mit Trifluoressigsäure und organischen Sulfonsäuren, wie Mesitylensulfonsäure und p-Toluolsulfonsäure), entsprechende Carbonsäureimidazolide und dergleichen. Man arbeitet zweckmässigerweise in Gegenwart eines säurebindenden Mittels und in einem inerten organischen Lösungsmittel. Geeignete säurebindende Mittel sind beispielsweise tertiäre Amine, wie Triäthylamin und Pyridin. Bei der Herstellung von Amiden kann als säurebindendes Mittel auch überschüssiges Amin der Formel VI verwendet werden. Geeignete Lösungsmittel sind beispielsweise offenkettige und cyclische Aether, wie Tetrahydrofuran, Diäthyläther, t-Butylmethyläther, Dioxan, Aethylenglykol, Dimethyläther oder dergleichen, halogenierte Kohlenwasserstoffe, wie Methylenchlorid, Chloroform und 1,2-Dichloräthan, Acetonitril und Dimethylformamid.

Auch die Hydrolyse von Verbindungen der Formel Ib⁴ zu den entsprechenden Catecholderivaten gemäss Verfahrensvariante g) kann nach an sich bekannten und jedem Fachmann geläufigen Methoden durchgeführt werden. Die Hydrolyse kann beispielsweise durch Behandeln mit einem Alkalimetallhydroxid, wie Natrium- oder Kaliumhydroxid, in einem geeigneten Lösungsmittel durchgeführt werden. Geeignete Lösungsmittel sind beispielsweise niedere Alkohole, wie Methanol, und Wasser oder Mischungen davon. Die Hydrolyse kann beispielsweise in einem Temperaturbereich von etwa 0°C bis zur Siedetemperatur des Lösungsmittels durchgeführt werden, wobei man jedoch vorzugsweise bei Raumtemperatur arbeitet.

Gemäss Verfahrensvarianate h) können Verbindungen der allgemeinen Formel 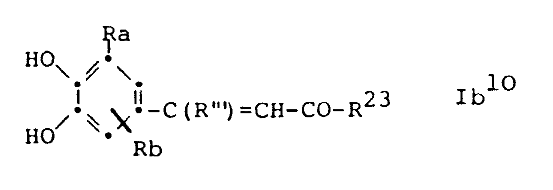
worin Ra, Rb, R''' und R²³ obige Bedeutung besitzen, und die entsprechenden Di-O-niederen Alkanoylderivate davon hergestellt werden. Als sekundäres Amin verwendet man vorzugsweise cyclische Amine, wie Pyrrolidin, Piperidin, Morpholin und Thiomorpholin. Geeignete Lösungsmittel für dieses Verfahren sind beispielsweise offenkettige und cyclische Aether, wie Tetrahydrofuran, Diäthyläther, t-Butylmethyläther, Dioxan, Aethylenglykol und Dimethyläther, halogenierte Kohlenwasserstoffe, wie Methylenchlorid, Chloroform und 1,2-Dichloräthan, Acetonitril und Dimethylformamid. Die Reaktionstemperatur liegt zweckmässigerweise in einem Bereich von etwa 0°C bis zur Siedetemperatur des gewählten Lösungsmittels, wobei man vorzugsweise bei Raumtemperatur arbeitet. In einer besonders bevorzugten Ausführungsform wird die Reaktion in Gegenwart einer Säure, vorzugsweise einer Carbonsäure, wie Essigsäure, durchgeführt.

Gemäss Verfahrensvariante i) können Verbindungen der allgemeinen Formel 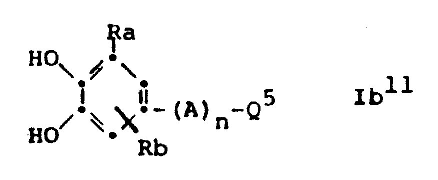
hergestellt werden, worin Q⁵ die Gruppe 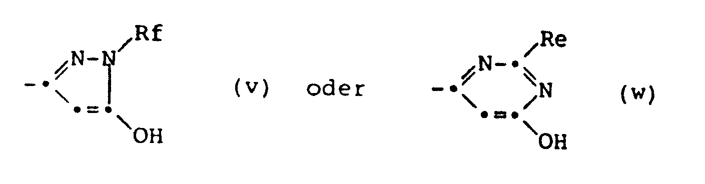
bedeutet, und Ra, Rb, Re, Rf, A und n obige Bedeutung besitzen.
Geeignete Lösungsmittel für dieses Verfahren sind beispielsweise niedere Alkohole, wie Methanol und Aethanol, offenkettige und cyclische Aether, wie Tetrahydrofuran, Diäthyläther, t-Butylmethyläther, Dioxan, Aethylenglykol und Dimethyläther, Acetonitril und Dimethylformamid. Man arbeitet vorzugsweise bei erhöhter Temperatur, beispielsweise in einem Bereich von etwa 50°C bis zur Siedetemperatur des gewählten Lösungsmittels, wobei man vorzugsweise bei der Siedetemperatur des gewählten Lösungsmittels arbeitet.

Gemäss Verfahrensvariante j) können Verbindungen der allgemeinen Formel 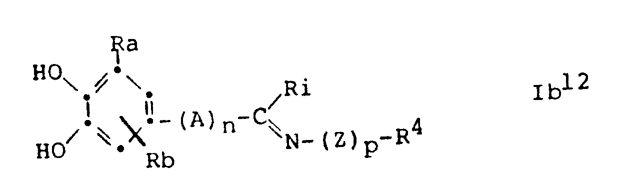
hergestellt werden, worin Ri die Gruppe -COR³¹, eine carbocyclische, aromatische Gruppe oder eine über ein Kohlenstoffatom gebundene, aromatische oder partiell ungesättigte, heterocyclische Gruppe oder einen gegebenenfalls substituierten, gesättigten oder partiell ungesättigten, niederen Kohlenwasserstoffrest bedeutet, und Ra, Rb, R³¹, R⁴, A, Z, n und p obige Bedeutung besitzen.
Auch dieses Verfahren kann nach an sich bekannten und jedem Fachmann geläufigen Methoden durchgeführt werden. Geeignete Lösungsmittel sind beispielsweise niedere Alkohole, wie Methanol und Aethanol, Dimethylformamid und Wasser. Die Reaktion wird zweckmässigerweise bei Raumtemperatur durchgeführt.

Gemäss Verfahrensvariante k) können die Verbindungen der Formel Ib in unter physiologischen Bedingungen hydrolysierbare Ester- oder Aetherderivate übergeführt werden. Geeignete, unter physiologischen Bedingungen hydrolysierbare Esterderivate sind insbesondere die Verbindungen der Formel Ib, worin mindestens eines der beiden phenolischen Hydroxygruppen durch eine niedere Fettsäure acyliert ist. Diese können nach an sich bekannten und jedem Fachmann geläufigen Methoden hergestellt werden. In einer bevorzugten Ausführungsform wird die Acylierung mit dem entsprechenden niederen Fettsäureanhydrid in Gegenwart einer katalytischen Menge einer starken Säure durchgeführt, wobei man als Lösungsmittel vorzugsweise überschüssiges Fettsäureanhydrid verwendet. Geeignete Säuren sind beispielsweise Schwefelsäure und organische Sulfonsäuren, wie p-Toluolsulfonsäure.

Geeignete unter physiologischen Bedingungen hydrolysierbare Aetherderivate sind beispielsweise Verbindungen der Formel Ib, worin mindestens eines der beiden phenolischen Hydroxygruppen durch eine niedere 1-Alkoxycarbonyloxy-1-alkyl-, niedere 1-Alkanoyloxy-1-alkyl- oder durch eine niedere 2-Oxo-1-alkylgruppe veräthert ist. Die Verätherung kann nach an sich bekannten und jedem Fachmann geläufigen Methoden durchgeführt werden. Man kann beispielsweise eine Verbindung der Formel Ib mit einem niederen 1-Alkoxycarbonyloxy-1-alkyl-, einem niederen 1-Alkanoyloxy-1-alkyl- oder einem niederen 2-Oxo-1-alkylhalogenid umsetzen, wobei diese Verätherung zweckmässigerweise in Gegenwart einer Base durchgeführt wird. Als Halogenide kommen insbesondere die Jodide in Frage. Geeignete Basen sind beispielsweise Alkalimetallhydroxide und Alkalimetallcarbonate, wie Natriumhydroxid und Natriumcarbonat.

Gemäss Verfahrensvariante k) können Verbindungen der obigen Formel Ib auch in pharmazeutisch annehmbare Salze übergeführt werden. Als Salze kommen insbesondere Salze mit pharmazeutisch annehmbaren Basen in Betracht. Als Beispiele solcher Salze seien die Alkalimetallsalze, wie die Natrium- und Kaliumsalze erwähnt. Diese Salze können nach an sich bekannten und jedem Fachmann geläufigen Methoden hergestellt werden.

Die verschiedenen als Ausgangsstoffe verwendeten Verbindungen sind bekannt oder können nach an sich bekannten Methoden hergestellt werden. Die nachfolgenden Beispiele enthalten detaillierte Angaben betreffend die Herstellung dieser Ausgangsstoffe.

Wie bereits eingangs erwähnt, hemmen die Verbindungen der Formel Ia das Enzym CONT. Diese Wirkung kann wie folgt quantitativ erfasst werden: Man inkubiert Rattenleber-Homogenate in Gegenwart eines geeigneten Substrates wie in J. Neurochem. 38, 191-195 (1982) beschrieben und misst die COMT-Aktivität. In einer zweiten Versuchsreihe wird die Inkubation in Gegenwart einer Verbindung der Formel Ia durchgeführt. Aus der Differenz der ermittelten COMT-Aktivitäten lässt sich dann die IC₅₀ berechnen. Die IC₅₀ wird in nMol/l angegeben und ist diejenige Konzentration in der Inkubationsmischung, welche notwendig ist, um die COMT-Aktivität um 50% herabzusetzen. In der nachfolgenden Tabelle sind die IC₅₀-Werte für einige Verbindungen der Formel Ia angegeben. Diese Tabelle enthält zudem Angaben über die akute Toxizität dieser Verbindungen (DL₅₀ in mg/kg bei einmaliger oraler Verabreichung an Mäusen).

| Verbindung der Formel Ia | IC₅₀ in nMol/l | DL₅₀ in mg/kg p.o. |
|---|---|---|
| 3,4-Dihydroxy-5-nitrophenyl-2-pyridylketon | 53,4 | 1250-2500 |
| 3,4-Dihydroxy-5-nitrophenyl-3-pyridylketon | 47,0 | 1000-2000 |
| 3,4-Dihydroxy-5-nitrophenyl-4-pyridylketon | 67,0 | 1000-2000 |
| 3,4-Dihydroxy-5-nitrobenzoesäure-n-butylester | 20,0 | 312- 625 |
| 3,4-Dihydroxy-5-nitrozimtsäure-n-butylester | 25,9 | 2500-5000 |
| 3,4-Dihydroxy-5-nitrophenylglyoxylsäureäthylester | 48,1 | 1250-2500 |
| 3,4-Dihydroxy-5-nitrobenzophenon | 48,0 | 500-1000 |
| 3,5-Dinitropyrocatechol | 36,9 | 500-1000 |
| 2'-Fluor-3,4-dihydroxy-5-nitrobenzophenon | 42,0 | 312- 625 |

Die beschriebenen Stoffe können als Heilmittel, z.B. in Form pharmazeutischer Präparate zur enteralen oder parenteralen Applikation, Verwendung finden. Die Verbindungen der Formel Ia können beispielsweise peroral, z.B. in Form von Tabletten, Lacktabletten, Dragees, Hart- und Weichgelatinekapseln, Lösungen, Emulsionen oder Suspensionen, rektal, z.B. in Form von Suppositorien, oder parenteral, z.B. in Form von Injektionslösungen, verabreicht werden.

Die Herstellung der pharmazeutischen Präparate kann in jedem Fachmann geläufiger Weise dadurch erfolgen, dass man die Verbindungen der Formel Ia, gegebenenfalls in Kombination mit anderen therapeutisch wertvollen Stoffen, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Als Trägermaterialien eignen sich sowohl anorganische als auch organische Trägermaterialien. So kann man für Tabletten, Lacktabletten, Dragees und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Träger beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glukose. Für Injektionslösungen eignen sich als Trägermaterialien beispielsweise Wasser, Alkohole, Polyole, Glyzerin und pflanzliche Oele. Für Suppositorien eignen sich als Trägermaterialien beispielsweise natürliche oder gehärtete Oele, Wachse, Fette und halbflüssige oder flüssige Polyole.

Als pharmazeutische Hilfsstoffe kommen die üblichen Konservierungsmittel, Lösungsvermittler, Stabilisierungsmittel, Netzmittel, Emulgiermittel, Mittel zur geschmacklichen Verbesserung, wie Süssmittel und Aromatisierungsmittel, Färbemittel, Salze zur Veränderung des osmotischen Druckes, Puffer, Ueberzugsmittel und Antioxidantien in Frage.

Die Dosierung der beschriebenen Stoffe kann, abhängig von der zu behandelnden Krankheit, dem Alter und dem individuellen Zustand des Patienten und von der Applikationsweise, innerhalb weiter Grenzen variieren und ist natürlich in jedem einzelnen Fall den individuellen Gegebenheiten anzupassen. Bei der Verbesserung der Behandlung der Parkinson-Krankheit und von Parkinsonismus mit L-Dopa kommt für den erwachsenen Patienten eine tägliche Dosis von etwa 25 mg bis etwa 1000 mg, insbesondere etwa 100 mg bis etwa 300 mg in Betracht. Je nach Dosierung ist es dabei zweckmässig, die Tagesdosis in mehreren Dosierungseinheiten zu verabreichen.

Die erfindungsgemässen pharmazeutischen Präparate enthalten zweckmässigerweise etwa 25 mg bis etwa 300 mg, vorzugsweise etwa 50 mg bis etwa 150 mg einer Verbindung der Formel Ia oder eines unter physiologischen Bedingungen hydrolysierbaren Ester- oder Aetherderivates oder eines pharmazeutisch annehmbaren Salzes davon.

Die nachfolgenden Beispiele sollen die vorliegende Erfindung näher erläutern, ihren Umfang jedoch in keiner Weise beschränken. Sämtliche Temperaturen sind in Celsiusgraden angegeben.

### Beispiel 1

a) 17,1 g (86,7 mMol) 4-Hydroxy-3-methoxy-5-nitrobenzaldehyd werden mit 170 ml konstantsiedender Bromwasserstoffsäure versetzt und während 3,5 Stunden unter Rückfluss erhitzt. Nach dem Abkühlen wird der ausgefallene Niederschlag abgesaugt, zweimal mit Eiswasser gewaschen und in Essigester aufgenommen. Die organische Phase wird zweimal mit je 50 ml Kochsalzlösung gewaschen, über Magnesiumsulfat getrocknet und im Wasserstrahlvakuum eingedampft. Die erhaltenen Kristalle werden in Methylenchlorid aufgenommen worauf man über der zehnfachen Menge Kieselgel filtriert. Das erhaltene Material wird aus Essigester/Isopropylether kristallisiert. Man erhält 3,4-Dihydroxy-5-nitrobenzaldehyd in Form gelber Kristalle vom Smp. 142-143°.
b) Man gibt zu einer Lösung von 1,83 g (10 mMol) 3,4-Dihydroxy-5-nitrobenzaldehyd in 25 ml Wasser eine Lösung von 1,7 g (15 mMol) Hydroxylamin-O-Sulfonsäure in 6 ml Wasser, rührt anschliessend während 3,5 Stunden bei 65°, kühlt ab, saugt den ausgefallene Niederschlag ab und nimmt diesen in Essigester auf. Die organische Phase wird über Natriumsulfat getrocknet und im Wasserstrahlvakuum eingedampft. Die erhaltenen Kristalle werden aus Essigester/n-Hexan umkristallisiert. Man erhält 3,4-Dihydroxy-5-nitrobenzonitril in Form gelber Kristalle vom Smp. 194-195°.

### Beispiel 2

aa) Zu 4,1 g 4-(Benzyloxy)-3-methoxy-brombenzol, gelöst in 40 ml Tetrahydrofuran, werden bei -70° innert 10 Min. 10 ml tert. Butyllithiumlösung (1,4 M in Hexan) zugetropft. Nach 2 Std. Rühren bei -70° wird 1 ml Pyridin-3-carbaldehyd innert 5 Min. zugegeben. Das Reaktionsgemisch wird 1 Std. bei -70° und 2 Std. bei 0° gerührt und auf 100 ml 1N-Salzsäure gegossen. Es wird dreimal mit je 50 ml Aether extrahiert. Die vereinigten Aetherphasen werden mit 100 ml 1 N-Salzsäure und 20 ml Wasser gewaschen. Die vereinigten Wasserphasen werden mit wässriger Ammoniaklösung alkalisch gestellt und dreimal mit je 100 ml Methylenchlorid extrahiert. Die vereinigten Methylenchloridphasen werden über Natriumsulfat getrocknet und eingedampft. Man erhält alpha-[4-(Benzyloxy)-3-methoxyphenyl]-3-pyridinmethanol als Oel.
ab) In analoger Weise erhält man bei Verwendung von Pyridin-4-carbaldehyd das alpha-[4-(Benzyloxy)-3-methoxyphenyl]-4-pyridinmethanol als Oel.
ba) 3.2 g alpha-[4-(Benzyloxy)-5-methoxyphenyl]-3-pyridinmethanol, suspendiert in 50 ml Wasser, werden mit 2.5 g Kaliumpermanganat versetzt, worauf man während 30 Min. bei 90° rührt. Nach Zusatz von weiteren 1.0 g Kaliumpermanganat und Rühren während weiteren 30 Min. bei 90° wird auf Raumtemperatur abgekühlt und zweimal mit je 150 ml Essigester extrahiert. Die vereinigten Essigesterphasen werden mit Kochsalzlösung gewaschen, über Natriumsulfat getrocknet und eingedampft. Der so erhaltene Rückstand wird an 50 g Kieselgel mit Essigester chromatographiert. Man erhält nach Umkristallisieren aus Methylenchlorid/Hexan 4-(Benzyloxy)-3-methoxyphenyl 3-pyridylketon vom Smp. 76°.
bb) In analoger Weise erhält man aus alpha-[4-(Benzyloxy)-3-methoxyphenyl)-4-pyridinmethanol das 4-(Benzyloxy)-3-methoxyphenyl 4-pyridylketon vom Smp. 85-87° (Methylenchlorid/Hexan).
ca) Zu 20 g 4-(Benzyloxy)-3-methoxyphenyl 3-pyridylketon, gelöst in 200 ml Methylenchlorid, werden bei 10° 50 ml 33-proz. Bromwasserstoffsäure in Essigsäure innert 15 Min. zugetropft. Nach 3 Std. Rühren bei 20° wird das Reaktionsgemisch auf ein Gemisch von 100 ml konz. wässrigem Ammoniak und Eis gegossen. Das pH wird durch Zugabe von Essigsäure auf 6 eingestellt. Die Methylenchloridphase wird abgetrennt: die wässrige Phase wird noch zweimal mit je 100 ml Methylenchlorid extrahiert. Die vereinigten Methylenchloridphasen werden über Natriumsulfat getrocknet und eingedampft. Der Rückstand wird aus Methylenchlorid/Hexan umkristallisiert. Man erhält 4-Hydroxy-3-methoxyphenyl 3-pyridylketon vom Smp. 150-151°.
cb) In analoger Weise erhält man aus 4-(Benzyloxy)-3-methoxyphenyl 4-pyridylketon das 4-Hydroxy-3-methoxyphenyl 4-pyridylketon vom Smp. 215-218° (Acetonitril).
da) Zu 1,15 g 4-Hydroxy-3-methoxyphenyl 3-pyridylketon, gelöst in 15 ml Essigsäure, werden 0,38 ml 65-proz. Salpetersäure bei Raumtemperatur zugetropft. Nach 2 Std. Rühren wird das Reaktionsgemisch auf 120 ml Eiswasser gegossen, worauf man mit konz. Ammoniak auf pH 5 stellt und den gebildeten Niederschlag abfiltriert. Der so erhaltene Rückstand wird in 20 ml Acetonitril unter Rückfluss erwärmt, worauf man erneut abfiltriert. Man erhält 4-Hydroxy-3-methoxy-5-nitrophenyl-3-pyridylketon als braune Kristalle vom Smp. 193° .
db) In analoger Weise erhält man aus 4-Hydroxy-3-methoxyphenyl-4-pyridylketon das 4-Hydroxy-3-methoxy-5-nitrophenyl-4-pyridylketon vom Smp. 240°.
e) 3.5 g 4-Hydroxy-3-methoxy-5-nitrophenyl-3-pyridylketon, gelöst in 70 ml 48-proz. wässriger Bromwasserstoffsäure, werden während 18 Std. bei 100° gerührt. Anschliessend wird das Reaktionsgemisch unter vermindertem Druck eingedampft. Der Rückstand wird aus Wasser umkristallisiert. Man erhält 3,4-Dihydroxy-5-nitrophenyl-3-pyridylketon-Hydrobromid vom Smp. 265°.
f) In analoger Weise erhält man aus 4-Hydroxy-3-methoxy-5-nitrophenyl-4-pyridylketon das 3,4-Dihydroxy-5-nitrophenyl-4-pyridylketon vom Smp. 246° (aus Wasser).
g) 13,2 g 3,4-Dihydroxy-5-nitrophenyl-4-pyridylketon werden in 500 ml Methanol suspendiert und unter Rühren mit 4.88 g Methansulfonsäure versetzt. Die Suspension wird 60 Minuten unter Rückfluss erhitzt. Anschliessend wird auf 10° abgekühlt, die Kristalle werden abgenutscht und zweimal mit je 30 ml Methanol gewaschen. Man erhält 3,4-Dihydroxy-5-nitrophenyl-4-pyridylketon-methansulfonat vom Smp. 260-261° (Zers.).

### Beispiel 3

a) Eine Lösung von 2.6 g (12,2 mMol) 4-Hydroxy-3-methoxy-5-nitrobenzoesäure in 26 ml konstantsiedender Bromwasserstoffsäure wird 2 Stunden unter Rückfluss erhitzt. Nach dem Abkühlen wird das Lösungsmittel im Wasserstrahlvakuum abdestilliert. Der kristalline Rückstand wird aus 50 ml Wasser in der Siedehitze umkristallisiert. Man erhält 3,4-Dihydroxy-5-nitrobenzoesäure in Form gelber Kristalle vom Smp. 224-226°.
b) Man vesetzt 1,0 g (5 mMol) 3,4-Dihydroxy-5-nitrobenzoesäure mit 20 ml methanolischer Salzsäurelösung, rührt während 3 Stunden bei 45° und nimmt den Rückstand nach Entfernen des Lösungsmittels in Methylenchlorid auf. Die organische Phase wird mit Kochsalzlösung gewaschen, über Natriumsulfat getrocknet und eingedampft. Das erhaltene kristalline Produkt wird in Methylenchlorid aufgenommen und über der zehnfachen Menge Kieselgel filtriert. Das erhaltene Material wird aus Essigester/n-Hexan umkristallisiert. Man erhält 3,4-Dihydroxy-5-nitrobenzoesäuremethylester in Form gelber Kristalle vom Smp. 144-145°.
   In analoger Weise erhält man ausgehend von der 3,4-Dihydroxy-5-nitrobenzoesäure die folgenden Ester:
c) 3,4-Dihydroxy-5-nitrobenzoesäure-ethylester vom Smp. 106-107° (aus Essigester/n-Hexan),
d) 3,4-Dihydroxy-5-nitrobenzoesäure-n-butylester vom Smp. 73-74° (aus Methylenchlorid) und
e) 3,4-Dihydroxy-5-nitrobenzoesäure-n-hexylester vom Smp. 44-45° (aus Isopropylether).

### Beispiel 4

a) Zu 10,0 g 3,4-Dimethoxy-5-nitrobenzaldehyd, gelöst in 150 ml Tetrahydrofuran, werden bei -20° innert 15 Min. 25 ml 2 M-Phenyllithiumlösung (in Benzol/Aether (7:3)) zugetropft, und das Reaktionsgemisch wird 1 Std. bei 0° und 2 Std. bei 20° gerührt. Anschliessend wird mit 150 ml 2 N-Schwefelsäure versetzt und dreimal mit 150 ml Aether extrahiert. Die vereinigten Aetherphasen werden mit Kochsalzlösung gewaschen, über Natriumsulfat getrocknet und eingedampft. Der so erhaltene Rückstand wird an 180 g Kieselgel mit Methylenchlorid chromatographiert. Man erhält 3,4-Dimethoxy-5-nitrobenzohydrol als amorphen Festkörper.
b) 2.5 g 3,4-Dimethoxy-5-nitrobenzohydrol, gelöst in 50 ml Methylenchlorid, werden mit 2,2 g Pyridiniumchlorochromat versetzt, worauf man während 2 Std. bei Raumtemperatur rührt. Anschliessend werden die unlöslichen Bestandteile abfiltriert. Das Filtrat wird eingedampft, und der Rückstand wird an 60 g Kieselgel mit Methylenchlorid chromatographiert. Dabei erhält man nach Kristallisieren aus Methylenchlorid/Hexan 3,4-Dimethoxy-5-nitrobenzophenon vom Smp. 78-80°.
c) 0,5 g 3,4-Dimethoxy-5-nitrobenzophenon werden in einem Gemisch von 4 ml Essigsäure und 4 ml 48-proz. wässriger Bromwasserstoffsäure während 30 Std. bei 110° gerührt. Anschliessend wird das Reaktionsgemisch zur Trockne eingedampft. Der Rückstand wird in Methylenchlorid aufgenommen. Es wird mit Wasser gewaschen, über Natriumsulfat getrocknet und eingedampft. Nach Umkristalisieren aus Methlyenchlorid/Hexan erhält man 3,4-Dihydroxy-5-nitrobenzophenon vom Smp. 132°.

### Beispiel 5

a) Man suspendiert 4,9 g (0,2 Mol) Magnesium in 15 ml absolutem Ethanol und erwärmt nach Zugabe von 1 ml Tetrachlorkohlenstoff bis die Reaktion anläuft. Dann wird eine Lösung von 31,8 g (0,2 Mol) Malonsäurediethylester in 19,9 ml absolutem Ethanol und 80 ml absolutem Toluol unter Rühren so zugetropft, dass die Temperatur zwischen 50° und 60° liegt. Anschliessend wird 1 Stunde bei dieser Temperatur weitergerührt, worauf das Reaktionsgemisch auf -5° abgekühlt und eine Lösung von 49,3 g (0,2 Mol) 3,4-Dimethoxy-5-nitrobenzoylchlorid (Smp. 82-85°) in 300 ml absolutem Toluol und 50 ml absolutem Tetrahydrofuran so zugetropft wird, dass die Temperatur -5° nicht übersteigt. Anschliessend wird über Nacht bei Raumtemperatur weitergerührt. Nach Abdestillieren des Lösungsmittels wird der Rückstand in 500 ml Essigester gelöst. Unter Rühren und Eiskühlung wird mit einer eiskalten Lösung von 12 ml konzentrierter Schwefelsäure in 80 ml Wasser versetzt. Die organische Phase wird mit Kochsalzlösung gewaschen, über Magnesiumsulfat getrocknet und eingedampft. Das erhaltene Oel wird an der zehnfachen Menge Kieselgel mit Methylenchlorid chromatographiert. Das erhaltene kristalline Material wird aus Isopropylether umkristallisiert. Man erhält 3,4-Dimethoxy-5-nitrobenzoylmalonsäurediethylester in Form hellbeiger Kristalle vom Smp. 70°.
b) Man löst 19.0 g (51,4 mMol) 3,4-Dimethoxy-5-nitrobenzoylmalonsäurediethylester in 100 ml Eisessig, gibt 5 Tropfen konzentrierter Schwefelsäure dazu und erhitzt während 16 Stunden unter Rückfluss. Die Essigsäure wird im Wasserstrahlvakuum bei 60° abdestilliert, und der Rückstand wird dreimal mit je 250 ml Toluol versetzt, wobei jeweils eingedampft wird. Der kristalline Rückstand wird mit Essigester extrahiert. Die organische Phase wird mit Wasser gewaschen, über Magnesiumsulfat getrocknet und eingedampft. Die erhaltenen Kristalle werden an der 30-fachen Menge Kieselgel mit Toluol chromatographiert. Das erhaltene kristalline Material wird aus Isopropylether umkristallisiert. Man erhält 3',4'-Dimethoxy-5'-nitroacetophenon in Form gelblicher Kristalle vom Smp. 86-87°.
c) Man versetzt 2 g (8,9 mMol) 3',4'-Dimethoxy-5'-nitroacetophenon mit 30 ml konstantsiedender Bromwasserstoffsäure und rührt während 2,5 Stunden bei 140°. Nach dem Abkühlen wird auf 200 ml Eiswasser gegossen und dreimal mit je 100 ml Essigester extrahiert. Die organische Phase wird zweimal mit je 25 ml Kochsalzlösung gewaschen, über Natriumsulfat getrocknet und eingedampft. Das erhaltene Produkt wird mit Essigester über der 20-fachen Menge Kieselgel filtriert. Durch Umkristallisieren des erhaltenen Materials aus Wasser erhält man 3',4'-Dihydroxy-5'-nitroacetophenon in Form gelber Kristalle vom Smp. 159-160°.
   Die gleiche Verbindung erhält man ausgehend von 4'-Hydroxy-3'-methoxy-5'-nitroacetophenon durch Behandeln mit Bromwasserstoffsäure in der Siedehitze.

### Beispiel 6

a) Man löst 100 g (0,8 Mol) Guajacol in 136,4 g (0.86 Mol) Isobuttersäureanhydrid, versetzt mit 120 g (0,88 Mol) wasserfreiem Zinkchlorid (wobei alles in Lösung geht), erhitzt das Reaktionsgemisch auf 155° und kühlt nach drei Minuten ab. Der Rückstand wird zunächst einer Wasserdampfdestillation unterzogen, um die leicht flüchtigen Anteile zu entfernen, und dann dreimal mit je 500 ml Ether extrahiert. Die organische Phase wird zweimal mit je 250 ml Wasser, einmal mit 150 ml gesättigter Bicarbonatlösung und nochmals mit 250 ml Wasser gewaschen, über Natriumsulfat getrocknet und im Wasserstrahlvakuum eingedampft. Das erhaltene braune Harz wird im Hochvakuum destilliert. Das Destillat vom Sdp. 105-120° (6,67 Pa) wird in Ether gelöst, worauf man bis zur beginnenden Kristallisation mit n-Hexan versetzt. Die erhaltenen Kristalle werden aus Ether/Hexan umkristallisiert. Man erhält 4'-Hydroxy-3'-methoxy-2-methyl-propiophenon in Form farbloser Kristalle vom Smp. 86-87°.
b) Man löst 15,0 g (77,2 mMol) 4'-Hydroxy-3'-methoxy-2-methyl-propiophenon in 300 ml Eisessig und tropft unter Rühren innert 15 Minuten 7,65 ml 50,5-proz. Salpetersäure (11,2 N) in 40 ml Eisessig dazu. Nach 15 Minuten wird das Reaktionsgemisch auf Eiswasser gegossen, und die ausgefallenen Kristalle werden abgenutscht, mit Wasser gewaschen und in Methylenchlorid gelöst. Die Lösung wird über Natriumsulfat getrocknet und eingedampft. Das erhaltene Rohprodukt wird in Methylenchlorid aufgenommen und über 100 g Kieselgel filtriert. Die so erhaltenen Kristalle werden aus Methylenchlorid/n-Hexan umkristallisiert. Man erhält 4'-Hydroxy-3'-methoxy-2-methyl-5'-nitropropiophenon in Form gelber Kristalle vom Smp. 85-87°.
c) Man versetzt 8,0 g (33,4 mMol) 4'-Hydroxy-3'-methoxy-2-methyl-5'-nitropropiophenon mit 64 g Pyridinhydrochlorid und rührt während 45 Minuten bei 180°. Nach dem Abkühlen wird das Reaktionsgemisch auf 500 ml Eiswasser gegossen, worauf man mit 20 ml 3N-Salzsäure sauer stellt und mit Methylenchlorid extrahiert. Die organische Phase wird mit Wasser gewaschen, über Natriumsulfat getrocknet und im Wasserstrahlvakuum eingedampft. Nach Kristallisieren aus Methylenchlorid/n-Hexan erhält man 3',4'-Dihydroxy-2-methyl-5'-nitropropiophenon in Form gelber Kristalle vom Smp. 98-99°.

### Beispiel 7

a) Man löst 100 g (805,5 mMol) Guajacol in 136,4 g (86,2 mMol) Buttersäureanhydrid, versetzt mit 120 g (880 mMol) Zinkchlorid, erhitzt wie in Beispiel 6.a angegeben während 3 Minuten und arbeitet dann wie dort beschrieben auf. Das nach Hochvakuumdestillation erhaltene Rohprodukt wird mit Toluol an 600 g Kieselgel chromatographiert. Nach Umkristallisieren aus Ether/n-Hexan erhält man 4'-Hydroxy-3'-methoxy-butyrophenon in Form farbloser Kristalle vom Smp. 40-41°.
b) Zu einer Lösung von 12,7 g (65,4 mMol) 4'-Hydroxy-3'-methoxybutyrophenon in 250 ml Eisessig werden unter Rühren innerhalb von 10 Minuten 6,5 ml 11,2 N-Salpetersäure zugetropft. Man rührt anschliessend während 15 Minuten, giesst das Reaktionsgemisch auf Eiswasser, saugt den ausgefallenen Niederschlag ab, wäscht diesen mit Eiswasser und nimmt in Methylenchlorid auf. Die Methylenchloridlösung wird über 50 g Kieselgel filtriert. Das erhaltene Material wird aus Methylenchlorid/n-Hexan umkristallisiert. Man erhält 4'-Hydroxy-3'-methoxy-5'-nitrobutyrophenon in Form gelber Kristalle vom Smp. 92-93°.
c) Man versetzt 10,2 g (42,6 mMol) 4'-Hydroxy-3'-methoxy-5'-nitrobutyrophenon mit 80 g Pyridinhydrochlorid und rührt während 40 Minuten bei 200°. Nach dem Abkühlen wird das Reaktionsgemisch auf 500 ml Eiswasser gegossen. Man versetzt mit 30 ml 3 N-Salzsäure und extrahiert mit Methylenchlorid. Die organische Phase wird über Natriumsulfat getrocknet und eingedampft. Das erhaltene Rohprodukt wird mit Methylenchlorid an 150 g Kieselgel chromatographiert. Das erhaltene Material wird aus Methylenchlorid/n-Hexan umkristallisiert. Man erhält 3',4'-Dihydroxy-5'-nitrobutyrophenon in Form gelber Kristalle vom Smp. 88-90°.

### Beispiel 8

a) Man löst 2,25 g (10 mMol) 3,4-Dihydroxy-5-nitrozimtsäure in 50 ml Methanol und leitet in diese Lösung während 10 Minuten Salzsäuregas ein. Nach 1 Stunde werden 50 ml Isopropylether dazugegeben, und der ausgefallene Niederschlag wird abgesaugt und mit Isopropylether gewaschen. Nach Umkristallisieren aus Methanol/Ether erhält man 3,4-Dihydroxy-5-nitrozimtsäuremethylester in Form gelber Kristalle vom Smp. 186-187°.
b) In analoger Weise erhält man aus 3,4-Dihydroxy-5-nitrozimtsäure und butanolischer Salzsäurelösung den 3,4-Dihydroxy-5-nitrozimtsäure-n-butylester in Form gelblicher Kristalle vom Smp. 129-130°.

### Beispiel 9

Man löst 5,0 g (13,5 mMol) 3,4-Dimethoxy-5-nitrobenzoylmalonsäurediethylester in 50 ml absolutem Methylenchlorid. Nach Abkühlen auf -20° wird unter Rühren eine Lösung von 16,9 g (67,5 mMol) Bortribromid in 30 ml Methylenchlorid so zugetropft, dass die Temperatur -20° nicht übersteigt. Anschliessend wird über Nacht bei Raumtemperatur gerührt. Nach Zugabe von 80 ml Ethanol wird während 30 Minuten bei Raumtemperatur gerührt, und anschliessend wird das Lösungsmittel im Wasserstrahlvakuum abdestilliert. Der Rückstand wird mit Wasser versetzt und mit Methylenchlorid extrahiert. Die organische Phase wird mit Wasser gewaschen, über Natriumsulfat getrocknet und eingedampft. Das erhaltene Rohprodukt wird mit Essigester über 50 g Kieselgel filtriert. Der erhaltene kristalline Rückstand wird aus Methylenchlorid/n-Hexan umkristallisiert. Man erhält 3,4-Dihydroxy-5-nitro-benzoylessigsäureethylester in Form gelber Kristalle vom Smp. 141-142°.

### Beispiel 10

a) Eine Lösung von 1,49 g (12,3 mMol) 2-Phenylethylamin in 100 ml Methylenchlorid wird mit 1,26 g Triethylamin versetzt. Unter Rühren wird eine Lösung von 3,0 g 3,4-Dimethoxy-5-nitrobenzoylchlorid in 100 ml Methylenchlorid zugetropft, worauf man während 15 Minuten weiterrührt. Die organische Phase wird dann zweimal mit je 50 ml Eiswasser extrahiert, über Natriumsulfat getrocknet und im Wasserstrahlvakuum eingedampft. Nach Umkristallisieren aus Methylenchlorid/n-Hexan erhält man 3,4-Dimethoxy-5-nitro-N-phenethylbenzamid in Form hellbeiger Nadeln vom Smp. 121-122°.
b) 3,6 g (10,9 mMol) 3,4-Dimethoxy-5-nitro-N-phenethylbenzamid werden mit 36 ml Phosphoroxychlorid unter Stickstoffatmosphäre während 96 Stunden unter Rückfluss erhitzt. Nach Abdestillieren des überschüssigen Phosphoroxychlorids im Wasserstrahlvakuum bei 60° wird dreimal mit je 100 ml Toluol versetzt, wobei das Lösungsmittel jeweils abdestilliert wird. Der Rückstand wird in Methylenchlorid aufgenommen. Die organische Phase wird mit Wasser gewaschen, über Natriumsulfat getrocknet und im Wasserstrahlvakuum eingedampft. Das erhaltene rote Harz wird mit Methylenchlorid/Essigester (1:1) an 120 g Kieselgel chromatographiert. Man erhält 1-(3,4-Dimethoxy-5-nitrophenyl)-3,4-dihydroisochinolin in Form eines gelben Harzes.
c) Man vesetzt 1,4 g (4,5 mMol) 1-(3,4-Dimethoxy-5-nitrophenyl)-3,4-dihydroisochinolin mit 15 ml konstantsiedender Bromwasserstoffsäure und erhitzt unter Stickstoffatmosphäre während 1,5 Stunden unter Rückfluss zum Sieden. Nach Abdestillieren der Bromwasserstoffsäure im Wasserstrahlvakuum wird der kristalline Rückstand aus Aceton umkristallisiert. Man erhält 5-(3,4-Dihydro-1-isochinolinyl)-3-nitropyrocatechol Hydrobromid in Form gelber Kristalle vom Smp. >250° (Zersetzung).

### Beispiel 11

a) Man versetzt 5,0 g (27,7 mMol) 4-Hydroxy-5-methoxy-isophthalaldehyd mit 50 ml konstantsiedender Bromwasserstoffsäure und erhitzt unter Argonatmosphäre während 3 Stunden unter Rückfluss und Rühren zum Sieden. Nach dem Abkühlen werden 50 ml Eiswasser zugegeben und der ausgefallene Niederschlag wird abgesaugt und mit Wasser gewaschen. Das Rohprodukt wird in Essigester aufgenommen und über 50 g Aluminiumoxid (Aktivitätsstufe II), filtriert. Das erhaltene kristalline Material wird aus Essigester/n-Hexan umkristallisiert. Man erhält 4,5-Dihydroxyisophthalaldehyd in Form von schwach orangen Kristallen vom Smp. 201-202°.
b) Zu einer Lösung von 2,0 g (12,0 mMol) 4,5-Dihydroxyisophthalaldehyd in 20 ml Wasser wird unter Rühren bei 30° eine Lösung von 3,27 g (28,9 mMol) Hydroxylamin-O-sulfonsäure in 12 ml Wasser zugetropft, worauf man während 10 Stunden bei 65° hält. Nach dem Abkühlen wird der ausgefallene Niederschlag abgesaugt, mit Wasser gewaschen und in Essigester aufgenommen. Die organische Phase wird mit Wasser gewaschen, über Natriumsulfat getrocknet und im Wasserstrahlvakuum eingedampft. Nach Umkristallisieren aus Essigester erhält man 4,5-Dihydroxyisophthalonitril in Form gelber Kristalle, welche sich oberhalb 300° zersetzen.

### Beispiel 12

a) Zu einer Lösung von 112.5 g 2-Brom-4'-hydroxy-3'-methoxyacetophenon in 560 ml Eisessig tropft man unter Rühren und innerhalb von 30 Minuten bei 20-25° eine Lösung von 38 g rauchender Salpetersäure (96 %) in 50 ml Eisessig dazu. Dabei scheiden sich gelbbraune Kristalle ab. Nach 90 Minuten wird das Reaktionsgemisch auf 300 g Eis gegossen. Die Kristalle werden abgenutscht, mit 1000 ml Wasser gewaschen und in 1000 ml Methylenchlorid gelöst. Man wäscht die organische Phase mit gesättigter Kochsalzlösung, trocknet über Natriumsulfat, filtriert und dampft das Filtrat bei 50° im Wasserstrahlvakuum bis zur beginnenden Kristallisation ein. Das auf Raumtemperatur abgekühlte Kristallisat wird abgenutscht und mit wenig Methylenchlorid gewaschen. Man erhält 2-Bromo-4'-hydroxy-3'-methoxy-5'-nitroacetophenon vom Smp. 147-149°

### b) Methode A:

ba) Man versetzt eine Suspension von 580,1 mg 2-Bromo-4'-hydroxy-3'-methoxy-5'-nitroacetophenon in 10 ml Aethanol mit 443,8 mg Selendioxid und erhitzt während 71 Stunden unter Rückfluss. Nachher wird das Reaktionsgemisch vom Selen abfiltriert und eingedampft. Man löst den Rückstand in Methylenchlorid, wäscht mit gesättigter Natriumchloridlösung, trocknet über Natriumsulfat, filtriert und dampft ein. Man erhält 4-Hydroxy-3-methoxy-5-nitrophenylglyoxylsäureethylester vom Smp. 165-167° (aus Aethanol).
   In analoger Weise erhält man:
bb) Aus 2-Brom-4'-hydroxy-3'-methoxy-5'-nitroacetophenon and n-Butanol den 4-Hydroxy-3-methoxy-5-nitrophenylglyoxylsäure-n-butylester vom Smp. 105-107° (aus Aethanol) und
bc) aus 2-Brom-4'-hydroxy-3'-methoxy-5'-nitroacetophenon und n-Hexanol den 4-Hydroxy-3-methoxy-5-nitrophenylglyoxylsäurehexylester vom Smp. 103-105° (aus n-Hexanol/Petroläther).

### c) Methode B:

ca) Man versetzt eine Suspension von 29.01 g 2-Bromo-4'-hydroxy-3'-methoxy-5'-nitroacetophenon in 300 ml tert. Butanol mit 27,74 g Selendioxid und erhitzt während 18 Stunden unter Rückfluss zum Sieden. Das heisse Reaktionsgemisch wird unter Nachwaschen mit Methylenchlorid durch ein Filtrierhilfsmittel aus Diatomenerde genutscht. Man dampft das Filtrat ein und schlemmt den Rückstand in 150 ml heissem Methylenchlorid auf. Der kristalline Niederschlag wird abgesaugt und mit wenig Methylenchlorid gewaschen. Man erhält 4-Hydroxy-3-methoxy-5-nitrophenylglyoxylsäure vom Smp. 169-171°.
   Man löst 2,42 g 4-Hydroxy-3-methoxy-5-nitrophenylglyoxylsäure in 25 ml trockenem N,N-Dimethylformamid, versetzt bei Zimmertemperatur mit 50 mg 4-Dimethylaminopyridin und 920 mg trockenem Methanol, kühlt anschliessend mit einem Eisbad auf 0° ab und gibt 2,27 g N,N-Dicyclohexylcarbodiimid dazu. Nach 10 Minuten wird das Eisbad entfernt, und das Reaktionsgemisch wird noch eine Stunde bei Raumtemperatur gerührt. Anschliessend wird eingedampft. Der Rückstand wird in Essigester gelöst, worauf man von ungelöstem Harnstoff abfiltriert, das Filtrat viermal mit Wasser wäscht, über Natriumsulfat trocknet, filtriert und eindampft. Man erhält 4-Hydroxy-3-methoxy-5-nitrophenylglyoxylsäuremethylester vom Smp. 155-157° (aus Methylenchlorid/Aether).
   In analoger Weise erhält man:
cb) Aus 4-Hydroxy-3-methoxy-5-nitrophenylglyoxylsäure und Aethanol den 4-Hydroxy-3-methoxy-5-nitrophenylglyoxylsäureäthylester vom Smp. 165-167° (aus Aethanol) und
cc) aus 4-Hydroxy-3-methoxy-5-nitrophenylglyoxylsäure und Isopropanol den 4-Hydroxy-3-methoxy-5-nitrophenylglyoxylsäure-i-propylester vom Smp. 99-101° (aus Isopropanol).
d) Man versetzt eine Suspension von 17,2 g 4-Hydroxy-3-methoxy-5-nitrophenylglyoxylsäureäthylester in 100 ml trockenem Acetonitril und 100 ml trockenem Toluol mit 10,53 g Natriumjodid und 11,9 g Siliciumtetrachlorid und erhitzt während 47 Stunden unter Rückfluss. Dann dampft man das Reaktionsgemisch ein und destilliert den Rückstand sechsmal mit je 200 ml Toluol ab. Man verteilt den erhaltenen Rückstand zwischen Wasser und Aether und filtriert durch ein Filtrierhilfsmittel aus Diatomenerde. Man wäscht die ätherische Phase viermal mit gesättigter Kochsalzlösung, trocknet über Natriumsulfat, filtriert und dampft ein. Der ölige Rückstand wird dreimal mit Aether und Aktivkohle behandelt. Man erhält 3,4-Dihydroxy-5-nitrophenylglyoxylsäureäthylester vom Smp. 77-79° (aus Aether/n Hexan).
   In analoger Weise erhält man:
e) Aus 4-Hydroxy-3-methoxy-5-nitrophenylglyoxylsäuremethylester den 3,4-Dihydroxy-5-nitrophenylglyoxylsäuremethylester als gelbes Destillat bei 145-150° und 10,67 Pa,
f) aus 4-Hydroxy-3-methoxy-5-nitrophenylglyoxylsäureisopropylester den 3,4-Dihydroxy-5-nitrophenylglyoxylsäureisopropylester als gelbes Destillat bei 155-160° und 12,0 Pa,
g) aus 4-Hydroxy-3-methoxy-5-nitrophenylglyoxylsäure-n-butylester den 3,4-Dihydroxy-5-nitrophenylglyoxylsäure-n-butylester als gelbes Destillat bei 160-165° und 10,67 Pa und
h) aus 4-Hydroxy-3-methoxy-5-nitrophenylglyoxylsäure-n-hexylester den 3,4-Dihydroxy-5-nitrophenylglyoxylsäurehexylester als gelbes Destillat bei 165-170° und 12.0 Pa.

### Beispiel 13

Man löst 236,1 mg 3,4-Dihydroxy-nitrophenylglyoxylsäure-n-hexylester in 15 ml Aethanol und versetzt mit 7,59 ml 0,1 N-Natriumhydroxydlösung. Nach einer Stunde wird die rötlichgelbe Lösung eingedampft. Das erhaltene Natriumsalz des 3,4-Dihydroxy-5-nitrophenylglyoxylsäure-n-hexylesters wird aus Wasser kristallisiert und hat einen Smp. von ∼ 300°.

### Beispiel 14

Eine Lösung von 3,18 g 3,4-Dihydroxy-5-nitrophenylglyoxylsäure-n-hexylester in 47,5 ml Aethanol wird mit 1,11 g O-Methylhydroxylamin-hydrochlorid, 1.95 g Natriumacetat und 2,5 ml Wasser versetzt und 5 Stunden unter Rückfluss zum Sieden erhitzt. Nachher wird das Reaktionsgemisch eingedampft, und der Rückstand wird mit Aether und Wasser versetzt. Die organische Phase wird mit gesättigter Natriumchloridlösung gewaschen, über Natriumsulfat getrocknet, filtriert und eingedampft. Der Rückstand wird an Kieselgel mit Methylenchlorid chromatographiert. Man erhält ein 7:3-Gemisch von E- und Z-3,4-Dihydroxy-5-nitrophenylglyoxylsäure-n-hexylester O-methyloxim als ein rötliches Oel; 80 MHz-NMR-Spektrum (CDCl₃): Signal für O-Methyl bei 3,96 und 4,05 ppm.

### Beispiel 15

Man versetzt eine Lösung von 5,1 g 3,4-Dihydroxy-5-nitrophenylglyoxylsäureäthylester in trockenem Methylenchlorid bei -10° innerhalb von 15 Minuten tropfenweise mit 25 g Bortribromid. Man rührt dann eine Stunde bei -10° und anschliessend 17 Stunden bei Raumtemperatur. Nachher dampft man das Reaktionsgemisch ein, versetzt den Rückstand vorsichtig mit Wasser und rührt noch 30 Minuten bei 50°. Nach dem Abkühlen auf Zimmertemperatur nutscht man den flockigen Niederschlag ab. Man säuert die wässrige Phase mit 10 ml 1N-Salzsäure an, extrahiert viermal mit Aether, wäscht die vereinigten organischen Auszüge viermal mit gesättigter Natriumchloridlösung, trocknet über Natriumsulfat, filtriert und dampft ein. Das Rohprodukt wird dreimal nacheinander in Aether durch ein Filtrierhilfsmittel aus Diatomenerde filtriert. Man erhält 3,4-Dihydroxy-5-nitrophenylglyoxylsäure vom Smp. 172-174° (aus Isopropyläther).

### Beispiel 16

a) Ein Gemisch aus 3,93 g 3,4-Dihydroxy-5-nitrophenylglyoxylsäure-n-hexylester und 1,38 g 2-Aminophenol werden bei 120° unter Rühren zum Schmelzen gebracht. Die Schmelze kristallisiert nach 5 Minuten. Nach 2 Stunden wird abgekühlt und aus Methanol umkristallisiert. Man erhält 3-(3,4-Dihydroxy-5-nitrophenyl)-2H-1,4-benzoxazin-2-on vom Smp. 202-204°.
   In analoger Weise erhält man:
b) Aus 3,4-Dihydroxy-5-nitrophenylglyoxylsäure-n-hexylester und 2-Amino-p-kresol das 3-(3,4-Dihydroxy-5-nitrophenyl)-6-methyl-2H-1,4 -benzoxazin-2-on vom Smp. 233-235° (aus Methanol/Methylenchlorid),
c) aus 3,4-Dihydroxy-5-nitrophenylglyoxylsäure-n-hexylester und 2-Amino-4-propylphenol das 3-(3,4-Dihydroxy-5-nitrophenyl)-6-propyl-2H-1,4-benzoxazin-2-on vom Smp. 200-202° (aus Methanol),
d) aus 3,4-Dihydroxy-5-nitrophenylglyoxylsäure-n-hexylester und 3-Amino-4-hydroxybenzoesäure das 3-(3,4-Dihydroxy-5-nitrophenyl)-2-oxo-2H-1,4-benzoxazin-6-carbonsäure vom Smp. 286-287° (aus Aceton/Petroläther),
e) aus 3,4-Dihydroxy-5-nitrophenylglyoxylsäure-n-hexylester und 2-Amino-4-chlorphenol das 6-Chlor-3-(3,4-dihydroxy-5-nitrophenyl)-2H-1,4-benzoxazin-2-on vom Smp. 241-243° (aus Methanol),
f) aus 3,4-Dihydroxy-5-nitrophenylglyoxylsäure-n-hexylester und 2-Amino-4,6-dichlorphenol das 6,8-Dichlor-3-(3,4-dihydroxy-5-nitrophenyl)-2H-1,4-benzoxazin-2-on vom Smp. 237-239° (aus Aethanol/Aether) und
g) aus 3,4-Dihydroxy-5-nitrophenylglyoxylsäure-n-hexylester und 2-Amino-5-nitrophenol das 3-(3,4-Dihydroxy-5-nitrophenyl)-7-nitro-2H-1,4-benzoxazin-2-on vom Smp. 253-255° (aus Acetonitril/Aethanol).

### Beispiel 17

a) Man erwärmt ein Gemisch aus 396,0 mg 3,4-Dihydroxy-5-nitrophenylglyoxylsäure-n-hexylester und 137,6 mg 1,2-Phenylendiamin während 60 Minuten auf 120°. Nachher wird in Methanol aufgeschlämmt, abgenutscht und aus N,N-Dimethylformamid/Wasser umkristallisiert. Man erhält 3-(3,4-Dihydroxy-5-nitrophenyl)-2(1H)-chinoxalinon vom Smp. >300°.
   In analoger Weise erhält man:
b) Aus 3,4-Dihydroxy-5-nitrophenylglyoxylsäure-n-hexylester und N-Methyl-1,2-phenylendiamin das 1-Methyl-3-(3,4-dihydroxy-5-nitrophenyl)-2(1H)-chinoxalinon vom Smp. 271-273° (aus Methanol),
c) aus 3,4-Dihydroxy-5-nitrophenylglyoxylsäure-n-hexylester und N-Propyl-1,2-phenylendiamin das 1-Propyl-3-(3,4-dihydroxy-5-nitrophenyl)-2(1H)-chinoxalinon vom Smp. 183-185° (aus Methanol),
d) aus 3,4-Dihydroxy-5-nitrophenylglyoxylsäure-n-hexylester und 4,5-Dimethyl-1,2-phenylendiamin das 3-(3,4-Dihydroxy-5-nitrophenyl)-6,7-dimethyl-2(1H)-chinoxalinon vom Smp. >300° (aus N,N-Dimethylformamid/Wasser),
e) aus 3,4-Dihydroxy-5-nitrophenylglyoxylsäure-n-hexylester und 4,5-Dichlor-1,2-phenylendiamin das 6,7-Dichlor-3-(3,4-dihydroxy-5-nitrophenyl)-2(1H)-chinoxalinon vom Smp. >300° (aus N,N-Dimethylformamid/Wasser),
f) aus 3,4-Dihydroxy-5-nitrophenylglyoxylsäure-n-hexylester und 3-Chlor-5-trifluoromethyl-1,2-phenylendiamin ein 1:1-Gemisch von 8(und 5)-Chlor-3-(3,4-dihydroxy-5-nitrophenyl)-6-(und 7)-trifluormethyl-2(1H)-chinoxalinon vom Smp. >300° (aus N,N-Dimethylformamid/Wasser),
g) aus 3,4-Dihydroxy-5-nitrophenylglyoxylsäure-n-hexylester und 4-Methoxy-1,2-phenylendiamin ein 1:1-Gemisch von 3-(3,4-Dihydroxy-5-nitrophenyl)-6(und 7)-methoxy-2(1H)-chinoxalinon vom Smp. >300° (aus Aethanol/Aether),
h) aus 3,4-Dihydroxy-5-nitrophenylglyoxylsäure-n-hexylester und 4-Nitro-1,2-phenylendiamin ein 1:1-Gemisch von 3-(3,4-Dihydroxy-5-nitrophenyl)-6(und 7)-nitro-2(1H)-chinoxalinon vom Smp. >300° (aus N,N-Dimethylformamid/Wasser) und
i) aus 3,4-Dihydroxy-5-nitrophenylglyoxylsäure-n-hexylester und N-Hexyl-1,2-phenylendiamin das 1-Hexyl-3-(3,4-dihydroxy-5-nitrophenyl)-2H-chinoxalinon vom Smp. 152-154° (aus Methanol).

### Beispiel 18

Eine Lösung von 1,07 g 3,4-Dihydroxy-5-nitrophenylglyoxylsäure-n-hexylester und 696,3 mg 2,3-Diaminonaphthalin in 3 ml 1-Hexanol wird 3 Stunden unter Rückfluss zum Sieden erhitzt. Das Reaktionsgemisch wird dann abgekühlt und mit Methanol verdünnt. Das rohe Produkt wird abgenutscht und aus N,N-Dimethylformamid/Wasser umkristallisiert. Man erhält 3-(3,4-Dihydroxy-5-nitrophenyl)benzo[g]chinoxalin-2(1H)-on vom Smp. >300°.

### Beispiel 19

Eine Suspension von 2.05 g 3,4-Dihydroxy-5-nitrophenylglyoxylsäure-n-hexylester und 600,8 mg Thiosemicarbazid wird 30 Minuten bei 90° intensiv gerührt. Dann wird auf 40° abgekühlt und mit einer Lösung von 870,1 mg Natriumhydroxyd in 15 ml Wasser versetzt. Anschliessend erwärmt man die Lösung während 30 Minuten auf 90°, kühlt das Reaktionsgemisch auf Raumtemperatur ab und versetzt tropfenweise mit 2 ml konz. Salzsäure. Das auskristallisierte Produkt wird abgenutscht und dann in Essigester gelöst. Man wäscht mit gesättigter Kochsalzlösung, trocknet über Natriumsulfat, filtriert und dampft ein. Man erhält 6-(3,4-Dihydroxy-5-nitrophenyl)-3-mercapto-1,2,4-triazin -5(4H)-on vom Smp. 282-284° (aus Aethanol).

### Beispiel 20

aaa) Man versetzt eine Suspension von 2,9 g 2-Brom-4'-hydroxy-3'-methoxy-5'-nitroacetophenon und 761,2 mg Thioharnstoff in 170 ml Aethanol bei 60° mit 820,3 mg Natriumacetat und rührt während 6 Stunden. Dann dampft man das Reaktionsgemisch ein, versetzt den Rückstand mit 170 ml Wasser und erwärmt während 30 Minuten auf 60°. Nach dem Abkuhlen nutscht man das Produkt ab und wäscht es mit Wasser. Man erhält 4-(2-Amino-4-thiazolyl)-2-methoxy-6-nitrophenol vom Smp. 248-250° (aus Aethanol).
   In analoger Weise erhält man:
aab) Aus 2-Brom-4'-hydroxy-3'-methoxy-5'-nitroacetophenon und N-Phenylthioharnstoff das 4-(2-Anilino-4-thiazolyl)-2-methoxy-6-nitrophenol vom Smp. 185-187° (aus Aether).
aba) Zu einer Lösung von 4,88 g 6-Amino-4'-(trifluormethyl)-hexananilid-hydrochlorid in 70 ml Wasser gibt man 50 ml 1,2-Dichloräthan und 3,56 g Calciumcarbonat. Die Suspension versetzt man innerhalb von 60 Minuten unter Rühren bei Raumtemperatur mit einer Lösung von 2,6 g Thiophosgen in 1,7 ml Toluol. Nach 16 Stunden nutscht man den Niederschlag ab und wäscht die organische Phase mit 1N-Salzsäure und Wasser. Nach Trocknen über Natriumsulfat und Filtrieren dampft man ein. Man erhält rohes 4'-(Trifluormethyl)hexananilid-6-isothiocyanat.
abb) Man versetzt eine Lösung von 5,2 g rohem 4'-(Trifluormethyl)hexananilid-6-isothiocyanat in 60 ml Aethanol mit 100 ml konz. Ammoniaklösung. Nach 30 Minuten dampft man das Reaktionsgemisch ein. Man löst den Rückstand in Essigester, wäscht mit Wasser, trocknet über Natriumsulfat, filtriert und dampft ein. Man erhält 1-[5-[(α,α,α-Trifluor-p-tolyl)carbamoyl]pentyl] -2-thioharnstoff vom Smp. 140-142° (aus Aethanol).
abc) Man versetzt eine Suspension von 666,7 mg 1-[5-[α,α,α-Trifluor-p-tolyl)carbamoyl]pentyl] -2-thioharnstoff und 580.2 mg 2-Brom-4'-hydroxy-3'-methoxy-5'-nitroacetophenon in 50 ml Aethanol bei 60° mit 164.2 mg Natriumacetat. Dabei entsteht eine rötlichgelbe Lösung. Nach 90 Minuten dampft man das Reaktionsgemisch ein, versetzt den Rückstand mit Wasser, nutscht den Niederschlag ab und wäscht viermal mit je 10 ml Wasser. Man erhält 6-[[4-(4-Hydroxy-3-methoxy-5-nitrophenyl)-2-thiazolyl]amino] -4'-(trifluormethyl)hexananilid vom Smp. 160-162° (aus Aethanol).
ac) Eine Lösung von 29.0 g 2-Brom-4'-hydroxy-3'-methoxy-5'-nitroacetophenon und 13.5 g 2-Aminoacetophenon in 250 ml trockenem N,N-Dimethylformamid wird bei 90° 16 Stunden gerührt. Das Reaktionsgemisch wird dann zu ca. zwei Drittel eingedampft und auf Eis gegossen. Die abgeschiedenen Kristalle werden abgenutscht und in Methylenchlorid gelöst. Man wäscht mit gesättigter Kochsalzlösung, trocknet über Natriumsulfat, filtriert und dampft ein. Man erhält 2-(4-Hydroxy-3-methoxy-5-nitrobenzoyl)-3-methylindol vom Smp. 195-197° (aus Methylenchlorid/Methanol).
ada) Man versetzt eine Suspension von 14.5 g 2-Brom-4'-hydroxy-3'-methoxy-5'-nitroacetophenon und 5,41 g 1,2-Phenylendiamin in 350 ml Methanol mit 4.92 g Natriumacetat und erhitzt das Gemisch unter Rückfluss während 22 Stunden zum Sieden. Dann dampft man das Reaktionsgemisch ein, löst den Rückstand in Methylenchlorid, wäscht diese Lösung viermal mit Wasser, trocknet sie über Natriumsulfat, filtriert und dampft ein. Man erhält 2-(4-Hydroxy-3-methoxy-5-nitrophenyl)chinoxalin vom Smp. 195-197° (aus Methylenchlorid/Methanol).
   In analoger Weise erhält man:
adb) Aus 2-Brom-4'-hydroxy-3'-methoxy-5'-nitroacetophenon und 4,5-Dimethyl-1,2-phenylendiamin das 6,7-Dimethyl-2-(4-hydroxy-3-methoxy-5-nitrophenyl)chinoxalin vom Smp. 207-209° (aus Methylenchlorid/Methanol).
b) Man versetzt eine Suspension von 267,3 mg 4-(2-Amino-4-thiazolyl)-2-methoxy-6-nitrophenol in 10 ml trockenem Methylenchlorid bei -20° mit 1,25 g Bortribromid. Man rührt nach der Zugabe noch eine Stunde bei -20° und ohne Kühlung bei Raumtemperatur während 18 Stunden. Dann dampft man das Reaktionsgemisch ein, versetzt den Rückstand vorsichtig mit Wasser und rührt während 30 Minuten bei 50°. Nach dem Abkühlen auf Zimmertemperatur nutscht man das rohe Produkt ab und wäscht es mit wenig Wasser. Man erhält 5-(2-Amino-4-thiazolyl)-3-nitropyrocatechol-hydrobromid vom Smp. 244-246° (aus Methanol).
   In analoger Weise erhält man:
c) Aus 4-(2-Anilino-4-thiazolyl)-2-methoxy-6-nitrophenol das 5-(2-Anilino-4-thiazolyl)-3-nitropyrocatechol vom Smp. 202-204° (aus Methanol).
d) aus 6-[4-(4-Hydroxy-3-methoxy-5-nitrophenyl)-2-thiazolyl)amino] -4'-(trifluormethyl)hexananilid das 6-[[4-(3,4-Dihydroxy-5-nitrophenyl)-2-thiazolyl]amino] -4'-(trifluormethyl)hexananilid vom Smp. 214-216° (aus Methanol).
e) aus 2-(4-Hydroxy-3-methoxy-5-nitrobenzoyl)-3-methylindol das 5-Brom-2-(3,4-dihydroxy-5-nitrobenzoyl)-3-methylindol vom Smp. 265-267° (aus Methanol).
f) aus 2-(4-Hydroxy-3-methoxy-5-nitrophenyl)chinoxalin das 2-(3,4-Dihydroxy-5-nitrophenyl)chinoxalin vom Smp. 241-243° (aus Methanol) und
g) aus 6,7-Dimethyl-2-(4-hydroxy-3-methoxy-5-nitrophenyl)-chinoxalin das 6,7-Dimethyl-2-(3,4-dihydroxy-5-nitrophenyl)chinoxalin vom Smp. 274-276° (aus Methanol).

### Beispiel 21

Man erhitzt eine Mischung aus 3,12 g 2-Brom-3',4'-dihydroxy-5'-nitroacetophenon und 849,3 mg Thioacetamid werden in 40 ml Aethanol während 18 Stunden unter Rückfluss zum Sieden. Nach dem Abkühlen werden die Kristalle abgenutscht und aus Methanol/Isopropanol umkristallisiert. Man erhält 5-(2-Methyl-4-thiazolyl)-3-nitropyrocatechol-hydrobromid vom Smp. 280-282°.

### Beispiel 22

Eine Lösung von 1,38 g 2-Brom-3',4'-dihydroxy-5'-nitroacetophenon und 461 mg Thiosemicarbazid in 20 ml n-Butanol wird während 60 Minuten unter Rückfluss zum Sieden erhitzt. Nach dem Abkühlen auf Zimmertemperatur nutscht man die Kristalle ab und kristallisiert sie aus n-Butanol um. Man erhält 5-(2-Amino-6H-1,3,4-thiadiazin-5-yl)-3 -nitropyrocatechol-hydrobromid vom Smp. 265-267°.

### Beispiel 23

Eine Lösung von 1,38 g 2-Brom-3',4'-dihydroxy-5'-nitroacetophenon und 505,7 mg 2-Amino-1,3,4-thiadiazol in 25 ml n-Butanol wird während 7 Stunden unter Rückfluss zum Sieden erhitzt. Dann kühlt man das Reaktionsgemisch auf Zimmertemperatur ab und nutscht die abgeschiedenen Kristalle ab. Man erhält 5-(Imidazo[2,1-b]-1,3,4-thiadiazol-6-yl)-3-nitropyrocatechol vom Smp. 278-280° (aus Methanol).

### Beispiel 24

Eine Mischung aus 2,78 g 2-Brom-3',4'-dihydroxy-5'-nitroacetophenon, 1,01 g 2-Aminothiazol und 40 ml Aethanol wird während 23 Stunden unter Rückfluss zum Sieden erhitzt. Dann kühlt man das Reaktionsgemisch auf Zimmertemperatur ab und nutscht die Kristalle ab. Man erhält 5-(Imidazo[2,1-b]thiazol-6-yl)-3 -nitropyrocatechol-hydrobromid vom Smp. 286-288° (aus Methanol).

### Beispiel 25

Eine Mischung aus 1,95 g 2-Brom-3',4'-dihydroxy-5'-nitroacetophenon, 1.06 g 2-Aminobenzothiazol und 50 ml Aethanol wird während 17 Stunden unter Rückfluss zum Sieden erhitzt. Das Reaktionsgemisch wird dann auf Zimmertemperatur abgekühlt, worauf man die Kristalle abnutscht. Man erhält 5-(Imidazo[2,1-b]benzothiazol-2-yl)-3-nitropyrocatechol vom Smp. 303-305° (aus N,N-Dimethylformamid/Methanol).

### Beispiel 26

Eine Mischung aus 2,78 g 2-Brom-3',4'-dihydroxy-5'-nitroacetophenon, 1,51 g 2-Aminothiophenol und 50 ml Aethanol wird während einer Stunde unter Rückfluss zum Sieden erhitzt. Das Reaktionsgemisch wird dann auf Zimmertemperatur abgekühlt, worauf man die Kristalle abnutscht. Man erhält 5-(2H-1,4-Benzothiazin-3-yl)-3-nitropyrocatechol vom Smp. 302-304° (aus N,N-Dimethylformamid/Methanol).

### Beispiel 27

Eine Mischung aus 987,5 mg 2-Brom-3',4'-dihydroxy-5'-nitroacetophenon und 1.01 g 2-Aminopyridin wird bei 110° zum Schmelzen gebracht. Nach 30 Minuten versetzt man mit 15 ml Aethanol und erhitzt während 3 Stunden unter Rückfluss zum Sieden. Dann wird das Reaktionsgemisch auf Raumtemperatur abgekühlt, und die Kristalle werden abgenutscht. Man erhält 5-(Imidazo[1,2-a]pyridin-3-yl)-3-nitropyrocatechol vom Smp. 250-252° (aus N,N-Dimethylformamid/Methanol).

### Beispiel 28

a) Zu einer Lösung von 10,7 g 4-Hydroxy-3-methoxy-5-nitrobenzoesäure in 500 ml trockenem Tetrahydrofuran gibt man 8,9 g 1,1'-Carbonyldiimidazol und erwärmt anschliessend das Reaktionsgemisch während 5 Stunden auf 65-70°. Dann kühlt man es auf Zimmertemperatur ab und tropft innert 15 Minuten eine Lösung von 21,6 g 6-Aminohexyl-t-butylcarbamat in 50 ml trockenem Tetrahydrofuran dazu. Man erwärmt dann das Reaktionsgemisch während 18 Stunden auf 65-70°, dampft ein, schlemmt den Rückstand in Essigester auf, nutscht ab und chromatographiert das abgenutschte Material mit Aceton/Methylenchlorld (3:1) an Kieselgel. Man erhält [6-(4-Hydroxy-5-nitro-m-anisamido)hexyl]-t-butylcarbamat vom Smp. 145-147° (aus Isopropanol).
b) Zu einer Lösung von 4,1 g [6-(4-Hydroxy-5-nitro-m-anisamido)hexyl]-t-butylcarbamat in 80 ml Eisessig gibt man bei Zimmertemperatur 5,8 ml Bromwasserstoffsäure in Eisessig (∼33-proz.). Man rührt während 2 Stunden, nutscht die ausgefallenen Kristalle ab und wäscht sie mit Aether. Man erhält N-(6-Aminohexyl)-4-hydroxy-5-nitro-m-anisamid-hydrobromid vom Smp. 207-209° (aus Isopropanol).
c) Zu einer Suspension von 392,3 mg N-(6-Aminohexyl)-4-hydroxy-5-nitro-m-anisamid hydrobromid in 25 ml trockenem Methylenchlorid gibt man bei -20° 1,25 g Bortribromid dazu. Nach der Zugabe rührt man während einer Stunde bei -20° und anschliessend während 17 Stunden bei Raumtemperatur. Dann dampft man das Reaktionsgemisch ein, versetzt den Rückstand mit 10 ml Wasser und rührt während einer Stunde bei Raumtemperatur. Nach Abdampfen des Wassers chromatographiert man den Rückstand mit Toluol/Aethanol (1:1) an Sephadex LH 20. Man erhält N-6-Aminohexyl-3,4-dihydroxy-5-nitrobenzamid-hydrobromid vom Smp. 205-207° (aus Aethanol).

### Beispiel 29

aa) Eine Lösung von 4,93 g 5-Nitrovanillin und 2,7 g 1,2-Phenylendiamin in 45 ml Methanol und 15 ml Nitrobenzol wird unter Rückfluss zum Sieden erhitzt. Nach 15 Minuten beginnen aus der roten Lösung Kristalle auszufallen. Nach 18 Stunden wird das Reaktionsgemisch auf Raumtemperatur abgekühlt und mit 60 ml Methanol verdünnt. Die Kristalle werden abgenutscht und mit Methanol gewaschen. Man erhält 4-(2-Benzimidazolyl)-2-methoxy-6-nitrophenol vom Smp. 198-200° (aus N,N-Dimethylformamid/Methanol).
   In analoger Weise erhält man:
ab) Aus 5-Nitrovanilin und 4,5-Dichlor-1,2-phenylendiamin das 4-(5,6-Dichlor-2-benzimidazolyl)-2-methoxy-6-nitrophenol vom Smp. 258-260° (aus N,N-Dimethylformamid/Aether).
b) Eine Suspension von 860,1 mg 4-(2-Benzimidazolyl)-2-methoxy-6-nitrophenol in 10 ml Eisessig und 10 ml 48-proz. Bromwasserstoffsäure wird während 72 Stunden unter Rückfluss zum Sieden erhitzt. Dann wird eingedampft, und der Rückstand wird viermal mit je 50 ml Toluol versetzt, das man jeweils wieder abdestilliert. Man erhält 5-(2-Benzimidazolyl)-3-nitropyrocatechol vom Smp. >300° (aus Aceton/Wasser).
   In analoger Weise erhält man:
c) Aus 4-(5,6-Dichlor-2-Benzimidazolyl)-2-methoxy-6-nitrophenol das 5-(5,6-Dichlor-2-benzimidazolyl)-3-nitropyrocatechol vom Smp. 282-284° (aus Aceton/Wasser).

### Beispiel 30

Man versetzt eine Suspension von 29,0 g 2-Brom-4'-hydroxy-3'-methoxy-5'-nitroacetophenon in 700 ml trockenem Methylenchlorid bei -20° innerhalb von 30 Minuten mit einer Lösung von 125,3 g Bortribromid in 300 ml trockenem Methylenchlorid. Man rührt nach der Zugabe noch eine Stunde bei -20° und 16 Stunden bei Raumtemperatur, dampft dann ein, versetzt den Rückstand unter Eiskühlung vorsichtig mit Wasser und rührt während 30 Minuten bei 50°. Nach dem Abkühlen extrahiert man mit Aether, wäscht die ätherische Phase mit Wasser, trocknet über Natriumsulfat, filtriert und dampft ein. Man erhält 2-Brom-3',4'-dihydroxy-5'-nitroacetophenon vom Smp. 138-140° (aus Methylenchlorid).

### Beispiel 31

a) Man erwärmt eine Lösung von 3,6 g 3,4-Dihydroxy-5-nitrophenylglyoxylsäureäthylester in 30 ml Essigsäureanhydrid in Gegenwart einer katalytischen Menge an konz. Schwefelsäure während 30 Minuten bei 110°, kühlt auf Raumtemperatur ab, giesst das Reaktionsgemisch auf 150 ml Wasser und rührt während 60 Minuten. Man extrahiert mit Aether, wäscht mit gesättigter Natriumchloridlösung, trocknet die vereinigten organischen Auszüge über Natriumsulfat, filtriert und dampft ein. Man erhält 3,4-Diacetoxy-5-nitrophenylglyoxylsäureäthylester vom Smp. 87-89° (aus Aether/Petroläther).
   In analoger Weise erhält man:
b) Aus 3-(3,4-Dihydroxy-5-nitrophenyl)-2H-1,4-benzoxazin-2-on das 3-(3,4-Diacetoxy-5-nitrophenyl)-2H-1,4-benzoxazin-2-on vom Smp. 186-188° (aus Methylenchlorid/Methanol).
c) aus 3-(3,4-Dihydroxy-5-nitrophenyl)-2(1H)-chinoxalinon das 3-(3,4-Diacetoxy-5-nitrophenyl)-2(1H)-chinoxalinon vom Smp. 241-243° (aus Methylenchlorid/Methanol),
d) aus 3,4-Dihydroxy-5-nitrobenzophenon das 3,4-Diacetoxy-5-nitrobenzophenon vom Smp. 141-143° (aus Methylenchlorid/Aether).
e) aus 2'-Fluor-3,4-dihydroxy-5-nitrobenzophenon das 3,4-Diacetoxy-2'-fluor-5-nitrobenzophenon vom Smp. 122-124° (aus Aether) und
f) aus 3,4-Dihydroxy-5-nitrophenyl-4-pyridylketon das 3,4-Diacetoxy-5-nitrophenyl-4-pyridylketon vom Smp. 148-150° (aus Methylenchlorid/Aether).

### Beispiel 32

a) Man erwärmt eine Lösung von 2.0 g 3,5-Dinitropyrocatechol in 25 ml Propionsäureanhydrid in Gegenwart einer katalytischen Menge an konz. Schwefelsäure während 18 Stunden bei 110°, destilliert das überschüssige Anhydrid bei 70° im Hochvakuum (1,33 Pa) ab, löst den Rückstand in Methylenchlorid, wäscht mit Wasser, trocknet über Natriumsulfat, filtriert und dampft ein Man erhält 1,2-Dipropionyloxy-3,5-dinitrobenzol vom Smp. 74-76° (aus Methylenchlorid/Petroläther).
   In analoger Weise erhält man:
b) Aus 3-(3,4-Dihydroxy-5-nitrophenyl)-6-methyl-2H-1,4-benzoxazin -2-on das 3-(3,4-Dipropionyloxy-5-nitrophenyl)-6-methyl -2H-1,4-benzoxazin-2-on vom Smp. 158-160° (aus Methylenchlorid),
c) aus 6-Chlor-3-(3,4-dihydroxy-5-nitrophenyl)-2H-1,4-benzoxazin-2-on das 5-(6-Chlor-2-oxo-2H-1,4-benzoxazin-3-yl)-3-nitro-o-phenylen -dipropionat vom Smp. 148-150° (aus Methylenchlorid/Aether),
d) aus 3-(3,4-Dihydroxy-5-nitrophenyl)-7-nitro-2H-1,4 -benzoxazin-2-on das 3-Nitro-5-(7-nitro-2-oxo-2H-1,4-benzoxazin-3-yl) -o-phenylen-dipropionat vom Smp. 177-179° (aus Methanol),
e) aus 3-(3,4-Dihydroxy-5-nitrophenyl)-2-oxo-2H-1,4-benzoxazin -6-carbonsäure die 3-[3,4-Bis(propionyloxy)-5-nitrophenyl]-2-oxo-2H-1,4 -benzoxazin-6-carbonsäure vom Smp. 192-194° (aus Methylenchlorid),
f) aus 3-Nitro-5-(2-chinoxalinyl)pyrocatechol das 3-Nitro-5-(2-chinoxalinyl)-o-phenylen-dipropionat vom Smp. 152-154° (aus Methylenchlorid/Aether),
g) aus 5-(2-Benzimidazolyl)-3-nitropyrocatechol das 5-(2-Benzimidazolyl)-3-nitro-o-phenylen-dipropionat vom Smp. 179-181° (aus Aether).
h) aus 6,7-Dichlor-3-(3,4-dihydroxy-5-nitrophenyl)-2(1H) -chinoxalinon das 5-(6,7-Dichlor-3,4-dihydro-3-oxo-2-chinoxalinyl)-3-nitro -o-phenylen-dipropionat vom Smp. 260-262° (aus Methylenchlorid),
i) aus 5-Brom-2-(3,4-dihydroxy-5-nitrobenzoyl)-3-methylindol das 5-Brom-2-(3,4-dipropionyloxy-5-nitrobenzoyl)-3-methylindol vom Smp. 196-198° (aus Aether) und
j) aus 6-(3,4-Dihydroxy-5-nitrophenyl)-3-mercapto-1,2,4-triazin -5(4H)-on das 3-Nitro-5-(2,3,4,5-tetrahydro-5-oxo-3-thioxo-as-triazin -6-yl)-o-phenylen-dipropionat vom Smp. 237-239° (aus Aether).

### Beispiel 33

a) Man erwärmt eine Lösung von 1.09 g 3,4-Dihydroxy-5-nitrophenylglyoxylsäureäthylester in 6 ml Isobuttersäureanhydrid in Gegenwart einer katalytischen Menge an konz. Schwefelsäure während 17 Stunden bei 110°. Dann wird das Reaktionsgemisch zehnmal mit je 10 ml Toluol versetzt, wobei man jeweils bei 80° und 18,7 mbar eindampft. Der ölige Rückstand wird im Kugelrohr bei 175-180° und 8,0 Pa destilliert. Man erhält 3,4-Diisobutyryloxy-5-nitrophenylglyoxylsäureäthylester.
   In analoger Weise erhält man:
b) Aus 3,5-Dinitropyrocatechol das 1,2-Diisobutyryloxy-3,5-dinitrobenzol vom Smp. 78-80° (aus Aether),
c) aus 3-(3,4-Dihydroxy-5-nitrophenyl)-6-methyl-2H-1,4-benzoxazin -2-on das 3-(3,4-Diisobutyryloxy-5-nitrophenyl)-6-methyl-2H -1,4-benzoxazin-2-on vom Smp. 142-144° (aus Aether).
d) aus 2-(3,4-Dihydroxy-5-nitrophenyl)chinoxalin das 2-(3,4-Diisobutyryloxy-5-nitrophenyl)chinoxalin vom Smp. 155-157° (aus Aether),
e) aus 1-Methyl-3-(3,4-dihydroxy-5-nitrophenyl)-2(1H) -chinoxalinon das 1-Methyl-3-(3,4-diisobutyryloxy-5-nitrophenyl)-2(1H)-chinoxalinon vom Smp. 138-140° (aus Aether),
f) aus 5-(Imidazo[2,1-b]-1,3,4-thiadiazol-6-yl)-3-nitropyrocatechol das 5-(Imidazo[2,1-b]-1,3,4-thiadiazol-6-yl)-3-nitro -o-phenylen-diisobutyrat vom Smp. 169-171° (aus Methylenchlorid),
g) aus 2-Brom-3',4'-dihydroxy-5'-nitroacetophenon das 5-(Bromoacetyl)-3-nitro-o-phenylen-diisobutyrat vom Smp. 56-58° (aus Methylenchlorid/Hexan),
h) aus 5-(2-Amino-4-thiazolyl)-3-nitropyrocatechol-hydrobromid das 3-Nitro-5-(2-isobutyramido-4-thiazolyl) -o-phenylen-diisobutyrat vom Smp. 157-159° (aus Methylenchlorid/Aether),
i) aus 5-(2-Amino-6H-1,3,4-thiadiazin-5-yl)-3-nitropyrocatechol-hydrobromid das 3-Nitro-5-(2-isobutyramido-4-isobutyryl-1,3,4-thiadiazin -5-yl)-o-phenylen-diisobutyrat vom Smp. 177-179° (aus Aether),
j) aus 3-(3,4-Dihydroxy-5-nitrophenyl)-6-propyl -2H-1,4-benzoxazin-2-on das 3-Nitro-5-(2-oxo-6-propyl-2H-1,4-benzoxazin -3-yl)-o-phenylen-diisobutyrat vom Smp. 131-133° (aus Methanol),
k) aus 5-(Imidazo[1,2-a]pyridin-3-yl)-3-nitropyrocatechol das 5-(Imidazo[1,2-a]pyridin-3-yl)-3-nitro-o-phenylen-diisobutyratvom Smp. 137-139° (aus Aether),
l) aus 5-(Imidazo[2.1-b]benzothiazol-2-yl)-3-nitropyrocatechol das 5-(Imidazo[2.1-b]benzothiazol-2-yl)-3-nitro -o-phenylen-diisobutyrat vom Smp. 197-199° (aus Methylenchlorid/Aether) und
m) aus 3,4-Dihydroxy-5-nitrophenyl-2-pyridylketon-hydrobromid das 3-Nitro-5-(2-pyridylcarbonyl)-o-phenylen-diisobutyrat vom Smp. 83-85° (aus Aether/Hexan).

### Beispiel 34

a) Man erwärmt eine Lösung von 613,0 mg 3,4-Dihydroxy-5-nitrophenylglyoxylsäureäthylester in 4 ml Pivaloylsäureanhydrid in Gegenwart einer katalytischen Menge an konz. Schwefelsäure während 17 Stunden auf 100°, verdünnt die abgekühlte Lösung mit Aether, wäscht mit gesättigter Natriumchloridlösung, trocknet über Natriumsulfat, filtriert und dampft ein. Der Rückstand wird zehnmal mit je 10 ml Toluol versetzt, wobei man jeweils wieder eindampft. Man erhält nach Kugelrohrdestillation (Luftbad) bei 175-180° und 4,0 Pa 3,4-Dipivaloyloxy-5-nitrophenylglyoxylsäureäthylester .
   In analoger Weise erhält man:
b) Aus 3-(3,4-Dihydroxy-5-nitrophenyl)-6-methyl-2H-1,4 -benzoxazin-2-on das 3-(3,4-Dipivaloyloxy-5-nitrophenyl)-6-methyl-2H-1,4-benzoxazin-2-on vom Smp. 180-182° (aus Aether),
c) aus 3,4-Dihydroxy-5-nitrobenzophenon das 3,4-Dipivaloyloxy-5-nitrobenzophenon vom Smp. 101-103° (aus t-Butylmethyläther) und
d) aus 2'-Fluor-3,4-dihydroxy-5-nitrobenzophenon das 3,4-Dipivaloyloxy-2'-fluorbenzophenon vom Smp. 74-76° (aus Petroläther ts.).

### Beispiel 35

Man erwärmt eine Lösung von 1,7 g 3-(3,4-Dihydroxy-5-nitrophenyl)-2(1H)-chinoxalinon in 17 ml Oenantsäureanhydrid in Gegenwart einer katalytischen Menge an konz. Schwefelsäure während 17 Stunden auf 110°, destilliert dann das überschüssige Anhydrid im Hochvakuum ab, löst den Rückstand in Methylenchlorid, wäscht die organische Lösung mit Wasser, trocknet über Natriumsulfat, filtriert und dampft ein. Man erhält 5-(1,2-Dihydro-2-oxo-3-chinoxalinyl)-3-nitro -o-phenylen-diheptanoat vom Smp. 186-188° (aus Methylenchlorid/Aether).

### Beispiel 36

a) Zu einer Lösung von 1,35 g 2-Bromo-3',4'-dihydroxy-5'-nitroacetophenon in 25 ml Alkohol gibt man 1,26 g Natriumacetat und erhitzt unter Rückfluss zum Sieden. Nach 6 Stunden wird das Reaktionsgemisch vom abgeschiedenen Natriumbromid abfiltriert und eingedampft. Der Rückstand wird in Essigester gelöst. Man wäscht mit gesättigter Kochsalzlösung, trocknet über Natriumsulfat, filtriert und dampft bei 50° ein. Man erhält (3,4-Dihydroxy-5-nitrobenzoyl)methylacetat vom Smp. 166-168° (aus Essigester/Aether).
   In analoger Weise erhält man:
b) Aus 2-Brom-3',4'-dihydroxy-5'-nitroacetophenon und Natriumisobutyrat das (3,4-Dihydroxy-5-nitrobenzoyl)methylisobutyrat vom Smp. 120-122° (aus Essigester/Aether) und
c) aus 2-Brom-3',4'-dihydroxy-5'-nitroacetophenon und 3,4-Dihydroxy-5-nitrophenylglyoxylsäure-natriumsalz das 3,4-Dihydroxy-5-nitrophenacyl(3,4-dihydroxy-5-nitrobenzoyl)format vom Smp. 224-226° (aus Methanol/Essigester).

### Beispiel 37

Man versetzt eine Suspension von 2,91 g 2-Brom-3',4'-dihydroxy-5'-nitroacetophenon mit 1,31 g Thionicotinamid in 50 ml Alkohol und erhitzt während 2 Stunden unter Rückfluss zum Sieden. Nach dem Abkühlen auf Raumtemperatur nutscht man die Kristalle ab und kristallisiert sie aus N,N-Dimethylformamid/Alkohol um. Man erhält 3-Nitro-5-[2-(3-pyridyl)-4-thiazolyl]pyrocatechol vom Smp. 279-281°.

### Beispiel 38

Eine Lösung von 5,52 g 2-Brom-3',4'-dihydroxy-5'-nitroacetophenon und 2,7 g 2-Aminoacetophenon in 100 ml trockenem N,N-Dimethylformamid wird bei 90° während 24 Stunden gerührt. Man dampft das Reaktionsgemisch ein, löst den Rückstand in Essigester, wäscht mit Wasser, trocknet über Natriumsulfat, filtriert und dampft ein. Man erhält 2-(3,4-Dihydroxy-5-nitrobenzoyl)-3-methylindol vom Smp. 212-214° (aus n-Butanol).

### Beispiel 39

Man versetzt eine Suspension von 7,32 g 2-Brom-3',4'-dihydroxy-5'-nitroacetophenon mit 4,06 g 1-(3-Pyridinyl)-2-thioharnstoff in 100 ml n-Butanol und erhitzt während 3 Stunden unter Rückfluss zum Sieden. Nach dem Abkühlen auf Raumtemperatur nutscht man die Kristalle ab und kristallisiert sie aus n-Butanol um. Man erhält 3-Nitro-5-[2-(3-pyridylamino)-4 -thiazolyl]pyrocatechol-hydrobromid vom Smp. >300°.

### Beispiel 40

Man versetzt eine Suspension von 6,35 g 2-Brom-3',4'-dihydroxy-5'-nitroacetophenon mit 4,68 g 1-(3-Chinolinyl)-2-thioharnstoff in 150 ml n-Butanol und erhitzt während 3 Stunden unter Rückfluss zum Sieden. Nach dem Abkühlen auf Raumtemperatur nutscht man die Kristalle ab und kristallisiert sie aus n-Butanol um. Man erhält 3-Nitro-5-[2-(3-chinolinylamino)-4 -thiazolyl]pyrocatechol-hydrobromid vom Smp. >300°.

### Beispiel 41

Man versetzt eine Suspension von 8.28 g 2-Brom-3',4'-dihydroxy-5'-nitroacetophenon mit 6.37 g rac-1-(2-exo-Bornyl)-2-thioharnstoff in 100 ml n-Butanol und erhitzt während 3 Stunden unter Rückfluss zum Sieden. Nach dem Abkühlen auf Raumtemperatur nutscht man die Kristalle ab und kristallisiert sie aus n-Butanol um. Man erhält rac-3-Nitro-5-[2-(2-exo-bornylamino)-4-thiazolyl] -pyrocatechol-hydrobromid vom Smp. 262-264°.

### Beispiel 42

Man versetzt 0.875 ml Pyrrolidin in 35 ml Tetrahydrofuran bei 5° mit 0,605 ml Essigsäure und anschliessend mit 1.73 g 3,4-Dihydroxy-5-nitrobenzaldehyd und 2,87 g 6-Oxo-4'-(trifluoromethyl)heptananilid und rührt während 56 Stunden unter Argon bei 23°. Der nach dem Eindampfen des Reaktionsgemisches erhaltene Rückstand wird zwischen Aethylacetat und 1N-Natronlauge verteilt. Man stellt die vereinigten Natronlaugeauszüge mit konz. Salzsäure sauer, extrahiert mit Aethylacetat, wäscht die vereinigten Aethylacetatauszüge mit gesättigter Kochsalzlösung, trocknet über Natriumsulfat, dampft ein und chromatographiert den Rückstand an 120 g Kieselgel mit Methylenchlorid/Methanol (91:9). Nach Umkristallisieren aus Aethylacetat/Petroläther erhält man (E)-8-(3,4-Dihydroxy-5-nitrophenyl)-6-oxo-4' -(trifluoromethyl)-7-octenanilid vom Smp. 194-197°.

### Beispiel 43

a) 26,0 g 2-Chlor-3-hydroxy-p-anisaldehyd werden in 400 ml Essigsäureanhydrid und 5 ml Pyridin gelöst. Man rührt 8 Stunden bei 80°, dampft anschliessend das Reaktionsgemisch ein, verteilt den Rückstand zwischen Eiswasser und Methylenchlorid, trocknet die organische Phase über Natriumsulfat, dampft ein und kristalisiert den Rückstand aus Methylenchlorid/Petroläther um. Man erhält 2-Chlor-3-formyl-6-methoxyphenyl-acetat vom Smp. 48-50°.
b) 38 g 2-Chlor-3-formyl-6-methoxyphenyl-acetat werden bei -5° bis -10° innert 15 Minuten portionenweise in 150 ml 98-proz. Salpetersäure eingetragen. Nach 30 Minuten Rühren bei -5° wird das Reaktionsgemisch auf 1,5 l Eiswasser gegossen und dreimal mit 500 ml Methylenchlorid extrahiert. Die vereinigten organischen Phasen werden mit Eiswasser gewaschen, über Natriumsulfat getrocknet und eingedampft. Der Rückstand wird aus Aether kristallisiert. Man erhält 2-Chlor-3-formyl-6-methoxy-5-nitrophenyl-acetat vom Smp. 84-85°.
c) 35,8 g 2-Chlor-3-formyl-6-methoxy-5-nitrophenyl-acetat werden in 300 ml Methanol gelöst. Nach Zugeben von 145 ml 1N-Natronlauge wird 1 Stunde bei 23° gerührt. Nach Abdampfen des Methanols verdünnt man den Rückstand mit Eiswasser, stellt mit 2N-Salzsäure sauer und extrahiert zweimal mit je 400 ml Aethylacetat. Die organischen Phasen werden mit gesättigter Kochsalzlösung gewaschen, über Natriumsulfat getrocknet und eingedampft. Der Rückstand wird aus Methylenchorid/Petroläther umkristallisiert. Man erhält 2-Chlor-3-hydroxy-5-nitro-p-anisaldehyd vom Smp. 130°.
d) Man löst 1,3 g 2-Chlor-3-hydroxy-5-nitro-p-anisaldehyd in 80 ml Methylenchlorid, versetzt mit 0,82 ml Bortribromid, rührt während 18 Stunden bei 23°, versetzt das Reaktionsgemisch unter Eiskühlung mit 5 ml Methanol, dampft ein, trocknet den Rückstand im Hochvakuum, digeriert in Wasser, filtriert und kristallisiert aus Acetonitril um. Man erhält 2-Chlor-3,4-dihydroxy-5-nitrobenzaldehyd vom Smp. 193-195°.

### Beispiel 44

a) Man rührt eine Mischung aus 30 g 2-Chlor-3-hydroxy-p-anisaldehyd und 230 ml Aethanol in Gegenwart von 12,3 g Hydroxylaminhydrochlorid während 4 Stunden bei 70°, dampft anschliessend das Reaktionsgemisch ein, trocknet den Rückstand im Hochvakuum und kristallisiert aus Methanol/Wasser um. Man erhält 2-Chlor-3-hydroxy-p-anisaldehyd-oxim vom Smp. 174-176°.
b) 21 g 2-Chlor-3-hydroxy-p-anisaldehyd-oxim werden zusammen mit 400 ml Essigsäureanhydrid während 20 Stunden unter Rückfluss erhitzt. Hierauf dampft man das Reaktionsgemisch ein, versetzt den Rückstand mit 300 ml Eiswasser, rührt 1 Stunde, dekantiert, verteilt den so erkalteten Rückstand zwischen Methylenchlorid und Wasser, trocknet die organische Phase über Natriumsulfat, dampft ein, trocknet den Rückstand im Hochvakuum, chromatographiert diesen an 200 g Kieselgel mit Methylenchlorid und kristallisiert aus Methylenchlorid/Petroläther um. Man erhält 2-Chlor-3-cyano-6-methoxyphenyl-acetat vom Smp. 97-99°.
c) In Analogie zu den Beispielen 43b und 43c erhält man aus 2-Chlor-3-cyano-6-methoxyphenyl-acetat das 2-Chlor-3-hydroxy-5-nitro-p-anisonitril vom Smp. 157-159° (Methylenchlorid/Hexan).
d) In Analogie zu Beispiel 43d erhält man aus 2-Chlor-3-hydroxy-5-nitro-p-anisonitril 2-Chlor-3,4-dihydroxy-5-nitro-benzonitril vom Smp. 180° (Acetonitril).

### Beispiel 45

a) 5,5 g α-Chlor-2-fluor-3,4-dimethoxytoluol und 4,05 g Kaliumacetat werden in 50 ml Dimethylformamid während 25 Stunden bei 80° gerührt. Anschliessend wird das Reaktionsgemisch auf 150 ml Eiswasser gegossen und mit Aether extrahiert. Die Aetherphasen werden mit Kochsalzlösung gewaschen, über Natriumsulfat getrocknet und eingedampft. Man erhält 2-Fluor-3,4-dimethoxybenzyl-acetat als Oel.
b) 5,4 g 2-Fluor-3,4-dimethoxybenzyl-acetat werden zusammen mit 50 ml Methanol und 50 ml 1 N-Natronlauge während 1,5 Stunden auf 80° erwärmt. Nach Abdampfen des Methanols wird der Rückstand mit Methylenchlorid extrahiert. Die vereinigten Extrakte werden mit Wasser gewaschen, über Natriumsulfat getrocknet und eingedampft. Man chromatographiert den Rückstand an 80 g Kieselgel mit Methylenchlorid/Methanol (95:5). Man erhält 2-Fluor-3,4-dimethoxybenzylalkohol als Oel.
c) 3,0 g 2-Fluor-3,4-dimethoxybenzylalkohol und 5,0 g Mangandioxid werden zusammen mit 50 ml Benzol während 1 Stunde unter Rückfluss erwärmt. Anschliessend werden die unlöslichen Anteile unter Waschen mit Methylenchlorid abfiltriert. Das Filtrat wird eingedampft, und der Rückstand wird aus Methylenchlorid/Hexan umkristallisiert. Man erhält 2-Fluor-3,4-dimethoxybenzaldehyd vom Smp. 52-54°.
d) 4.4 g 2-Fluor-3,4-dimethoxybenzaldehyd und 1,83 g Hydroxylaminhydrochlorid werden zusammen mit 30 ml Aethanol 5 Stunden unter Rückfluss erwärmt. Anschliessend wird das Reaktionsgemisch eingedampft, und der im Hochvakuum bei 23° getrocknete Rückstand wird in 40 ml Phosphoroxychlorid eingetragen. Nach 2,5 Stunden Rühren bei 23° dampft man das Reaktionsgemisch ein, behandelt den Rückstand mit Eiswasser, filtriert den dabei gebildeten Niederschlag ab, wäscht mit Wasser, nimmt in Methylenchlorid auf, trocknet die Methylenchloridlösung über Natriumsulfat und dampft ein. Durch Umkristallisieren des Rückstandes aus Methylenchlorid/Hexan erhält man 2-Fluor-3,4-dimethoxybenzonitril vom Smp. 64-65°.
e) Man löst 2,0 g 2-Fluor-3,4-dimethoxybenzonitril in 60 ml Methylenchlorid, versetzt mit 1,1 ml Bortribromid, rührt 1 Stunde bei 23°, versetzt anschliessend mit weiteren 1,0 ml Bortribromid und rührt weitere 80 Minuten bei 23°. Hierauf wird das Reaktionsgemisch auf 100 ml eiskalte gesättigte Natriumhydrogencarbonatlösung gegossen, worauf man zweimal mit 300 ml Aether extrahiert, die vereinigten Aetherphasen zweimal mit Kochsalzlösung wäscht, über Natriumsulfat trocknet, eindampft und den Rückstand an 50 g Kieselgel mit Methylenchlorid und Methylenchlorid/Methanol (97:3) chromatographiert. Man erhält 2-Fluor-3-hydroxy-p-anisonitril vom Smp. 198-200°.
f) 0,9 g 2-Fluor-3-hydroxy-p-anisonitril werden in 10 ml Essigsäureanhydrid und 0,5 ml Pyridin gelöst, worauf man 2 Stunden bei 120° gerührt. Anschliessend wird das Reaktionsgemisch eingedampft, und der Rückstand wird zwischen Eiswasser und Methylenchlorid verteilt. Die organische Phase wird über Natriumsulfat getrocknet und eingedampft, und der Rückstand wird aus Methylenchlorid/Hexan umkristallisiert. Man erhält 3-Cyano-2-fluor-6-methoxyphenyl-acetat vom Smp. 90-91°.
g) 0.8 g 3-Cyano-2-fluor-6-methoxyphenyl-acetat werden in drei Portionen bei -15° in 5 ml 96-proz. Salpetersäure eingetragen, worauf man 1 Stunde bei -10° rührt. Hierauf wird das Reaktionsgemisch auf 50 g Eis gegossen. Man extrahiert zweimal mit je 70 ml Aethylacetat. Die vereinigten organischen Phasen werden mit gesättigter Natriumchloridlösung gewaschen, über Natriumsulfat getrocknet und eingedampft. Der so erhaltene Rückstand wird in 50 ml 1 N-Natriumcarbonatlösung und 50 ml Methanol gelöst, worauf man 1 Stunde bei 23° rührt, und das Methanol abdestilliert. Die zurückbleibende wässrige Phase wird bei 0° mit konz. Salzsäure auf pH 2 gestellt und zweimal mit 100 ml Aethylacetat extrahiert. Die vereinigten organischen Phasen werden mit gesättigter Natriumchloridlösung gewaschen, über Natriumsulfat getrocknet und eingedampft, und der Rückstand wird an 45 g Kieselgel mit Methylenchlorid/Methanol (97:3) chromatographiert. Man erhält nach Umkristallisieren aus Aether/Hexan 2-Fluor-3-hydroxy-5-nitro-p-anisonitril vom Smp. 112-113°.
h) 550 mg 2-Fluor-3-hydroxy-5-nitro-p-anisonitril werden in 30 ml Methylenchlorid gelöst, worauf man mit 0,44 ml Bortribromid versetzt. Nach 6 Stunden Rühren bei 23° werden weitere 0,1 ml Bortribromid dazugegeben, worauf man noch 1,5 Stunden bei 23° rührt. Hierauf werden bei -15° 3 ml Aethanol dazugegeben. Das Reaktionsgemisch wird eingedampft, und der Rückstand wird bei 23° im Hochvakuum getrocknet. Durch Umkristallisieren aus Aether/Hexan erhält man 2-Fluor-3,4-dihydroxy-5-nitrobenzonitril vom Smp. 154°.

### Beispiel 46

a) 9,5 g 3,4-Dimethoxy-5-nitrobenzoesäure werden in 95 ml Thionylchlorid suspendiert. Man rührt 1 Stunde bei 80°. Durch zweimaliges Eindampfen unter Zusatz von absolutem Toluol erhält man 10 g 3,4-Dimethoxy-5-nitrobenzoesäurechlorid, welches in 100 ml Tetrahydrofuran gelöst wird. Diese Lösung wird zu 300 ml 28-proz. wässrigem Ammoniak zugetropft. Anschliessend wird 2 Stunden bei 40° gerührt und auf 5° abgekühlt. Der kristalline Niederschlag wird abfiltriert. Man erhält 3,4-Dimethoxy-5-nitrobenzamid vom Smp. 182-185°.
b) Unter Eiskühlung werden 2,46 g Chlor in ein Gemisch aus 8.0 g Natriumhydroxid, 50 ml Wasser und 30 g Eis eingeleitet. Hierauf werden 6,5 g 3,4-Dimethoxy-5-nitrobenzamid, suspendiert in 5 ml Tetrahydrofuran, langsam dazugegeben. Man erwärmt das Reaktionsgemisch innert 30 Minuten auf 70°, rührt 1 Stunde bei 70°, kühlt auf 5° ab und filtriert die ausgefallenen Kristalle ab. Man erhält 3,4-Dimethoxy-5-nitroanilin vom Smp. 129-131°. Durch Extraktion des Filtrates mit Aethylacetat. Trocknen des Auszuges über Natriumsulfat. Einengen und Kristallisieren des Rückstandes unter Zusatz von Aether kann eine weitere Portion 3,4-Dimethoxy-5-nitroanilin erhalten werden.
c) 10 g fein pulverisiertes 3,4-Dimethoxy-5-nitroanilin werden in 15 ml 12 N-Salzsäure und 40 ml Wasser suspendiert, worauf man 1 Stunde bei 30° rührt. Hierauf werden bei -5° 3,8 g Natriumnitrit, gelöst in 20 ml Wasser, innert 15 Minuten dazugetropft. Man rührt 30 Minuten bei -5° und tropft die kalte Diazoniumsalzlösung innert 45 Minuten zu 100 ml Pyridin von 40°. Anschliessend wird 1 Stunde bei 70° gerührt. Das Reaktionsgemisch wird eingedampft, und der Rückstand wird in 300 ml Aethylacetat aufgenommen. Es wird dreimal mit je 200 ml 2 N-Salzsäure ausgezogen. Die saure Wasserphase wird mit konz. Ammoniak auf pH 9 gestellt und mit Methylenchlorid extrahiert. Die vereinigten Methylenchloridextrakte werden über Natriumsulfat getrocknet und eingedampft, und der Rückstand wird an 250 g Kieselgel mit Aethylacetat chromatographiert. Man erhält nach Kristallisieren aus Methylenchlorid/Hexan 2-(3,4-Dimethoxy-5-nitrophenyl)pyridin vom Smp. 89-90°.
d) 1,5 g 2-(3,4-Dimethoxy-5-nitrophenyl)pyridin werden in 30 ml 48 proz. wässriger Bromwasserstoffsäure gelöst. Das Reaktionsgemisch wird 16 Stunden bei 100° und 18 Stunden bei 23° gerührt. Der dabei gebildete Niederschlag wird abfiltriert und aus Methanol/Aether umkristallisiert. Man erhält 3-Nitro-5-(2-pyridyl)pyrocatechol-Hydrobromid vom Smp. 239-240°.

### Beispiel 47

a) 2,76 ml 2-Brompyridin, gelöst in 30 ml absolutem Tetrahydrofuran, werden bei -60° innert 30 Minuten mit 19.2 ml 1.6 M n-Butyllithium-Lösung (in Hexan) versetzt, worauf man 30 Minuten bei -60° rührt. Dann werden 6,0 g 3,4-Dimethoxy-5-nitrobenzaldehyd, gelöst in 50 ml Tetrahydrofuran, innert 30 Minuten bei -40° dazugetropft. Das Reaktionsgemisch wird innert 2 Stunden auf 0° erwärmt, auf Eiswasser gegossen und mit Aethylacetat extrahiert. Die vereinigten Extrakte werden über Natriumsulfat getrocknet und eingedampft, und der Rückstand wird an 200 g Kieselgel mit Aethylacetat chromatographiert. Man erhält α-(3,4-Dimethoxy-5-nitrophenyl)-2-pyridinmethanol als braunes Oel.
b) Zu 4,0 g α-(3,4-Dimethoxy-5-nitrophenyl)-2-pyridinmethanol, gelöst in 100 ml Aceton, werden unter stetigem Erhitzen unter Rückfluss über einen Zeitraum von 2,5 Stunden 7 g Mangandioxid portionenweise dazugegeben. Hierauf wird weitere 2 Stunden unter Rückfluss erhitzt. Anschliessend werden die Mangansalze abfiltriert, und der nach dem Eindampfen erhaltene Rückstand wird aus Aether/Hexan umkristallisiert. Man erhält 3,4-Dimethoxy-5-nitrophenyl-2-pyridylketon vom Smp. 113°.
c) 1,5 g 3,4-Dimethoxy-5-nitrophenyl-2-pyridylketon, gelöst in 30 ml 48 proz. Bromwasserstoffsäure, werden 18 Stunden bei 100° gerührt. Hierauf wird das Reaktionsgemisch eingedampft, und der Rückstand wird aus Acetonitril/Methanol umkristallisiert. Man erhält 3,4-Dihydroxy-5-nitrophenyl-2-pyridylketon-Hydrobromid vom Smp. 213°.

### Beispiel 48

a) Zu 2,25 g 3',4'-Dimethoxy-5'-nitroacetophenon, gelöst in 50 ml Aethanol, werden 11,3 g Zinndichlorid-Dihydrat dazugefügt, worauf man 30 Minuten bei 75° rührt. Hierauf wird das Reaktionsgemisch auf 100 g Eis gegossen, mit ca. 300 ml gesättigter Natriumhydrogencarbonatlösung neutralisiert und mit 150 ml Methylenchlorid versetzt. Man filtriert und trennt die Methylenchloridphase ab. Diese wird über Natriumsulfat getrocknet und eingedampft, und der Rückstand wird aus Aether/Petroläther umkristallisiert. Man erhält 5'-Amino-3',4'-dimethoxyacetophenon vom Smp. 63-65°.
b) Zu 14,0 g 5'-Amino-3',4'-dimethoxyacetophenon, gelöst in 155 ml 1 N-Salzsäure, wird bei 0° innert 20 Minuten eine Lösung von 5,2 g Natriumnitrit in 20 ml Wasser dazugetropft. Nach 30 Minuten Rühren bei -2° wird die kalte Diazoniumsalzlösung innert 30 Minuten bei 5-10° zu einer Lösung von 8,7 g Kupfer(I)cyanid und 5,45 g Kaliumcyanid in 60 ml Wasser dazugetropft. Nach beendeter Zugabe werden 200 ml Methylenchlorid zugesetzt, und das Reaktionsgemisch wird 3 Stunden bei 23° gerührt und dann filtriert. Die organische Phase wird abgetrennt, mit Wasser gewaschen, über Natriumsulfat getrocknet und eingedampft. Der Rückstand wird aus Methylenchlorid/Petroläther umkristallisiert. Man erhält 5'-Cyano-3',4'-dimethoxyacetophenon vom Smp. 125-126°.
c) 3,75 g Aluminiumpulver und 28,5 g Jod werden in 160 ml absolutem Benzol 2 Stunden unter Rückfluss erhitzt. Bei 20° werden dann 3,0 g 5'-Cyano-3',4'-dimethoxyacetophenon und 0.5 g Tetra-n-butylammoniumjodid zugesetzt, worauf man 1 Stunde unter Rückfluss erhitzt. Hierauf wird bei 20° mit 50 g Eis versetzt und filtriert. Der Rückstand wird mit Aethylacetat gewaschen. Die Phasen werden getrennt, und die wässrige Phase wird noch zweimal mit Aethylacetat ausgezogen. Die vereinigten organischen Phasen werden mit 20-proz. Natriumthiosulfatlösung gewaschen, über Natriumsulfat getrocknet und eingedampft. Der dabei erhaltene Rückstand wird in 20 ml Essigsäureanhydrid und 0,5 ml Pyridin gelöst und 6 Stunden bei 120° gerührt. Anschliessend wird das Gemisch eingedampft, und der Rückstand wird zwischen Methylenchlorid und Eiswasser verteilt. Die Methylenchloridphase wird über Natriumsulfat getrocknet und eingedampft, und der Rückstand wird an 100 g Kieselgel mit Methylenchlorid chromatographiert. Man erhält nach Umkristallisieren aus Aether 5'-Cyano-3',4'-diacetoxyacetophenon vom Smp. 76-79°.

### Beispiel 49

0,33 g 5'-Cyano-3',4'-diacetoxyacetophenon, gelöst in 3,3 ml Methanol, werden mit 2,7 ml 1,0 N Natronlauge versetzt, und das Reaktionsgemisch wird 45 Minuten bei 23° gerührt. Anschliessend wird mit 2N-Salzsäure angesäuert, mit 5 ml gesättigter Kochsalzlösung verdünnt und zweimal mit je 30 ml Aethylacetat extrahiert. Die vereinigten Aethylacetatphasen werden über Natriumsulfat getrocknet und eingedampft. Der Rückstand wird aus Toluol/Acetonitril umkristallisiert. Man erhält 5'-Cyano-3',4'-dihydroxyacetophenon als bräunliche Kristalle, die sich oberhalb von 215° zersetzen.

### Beispiel 50

a) Zu 1,9 g 5'-Cyano-3',4'-dimethoxyacetophenon, gelöst in 30 ml Tetrahydrofuran, werden bei Raumtemperatur innert 45 Minuten 3,48 g Phenyltrimethylammoniumbromid-dibromid, gelöst in 30 ml Tetrahydrofuran, zugetropft, worauf man 30 Minuten rührt. Hierauf wird das Reaktionsgemisch auf 120 ml Eiswasser gegossen und dreimal mit 70 ml Methylenchlorid extrahiert. Die vereinigten Methylenchloridphasen werden mit 2N-Schwefelsäure gewaschen, über Natriumsulfat getrocknet und eingedampft. Der Rückstand wird an 20 g Kieselgel mit Methylenchlorid chromatographiert. Man erhält nach Umkristallisieren aus Methylenchlorid/Hexan 5-(Bromoacetyl)-2,3-dimethoxybenzonitril vom Smp. 138-141°.
b) 1,45 g 5-(Bromoacetyl)-2,3-dimethoxybenzonitril und 1,12 g Selendioxid werden in 10 ml n-Hexanol während 18 Stunden bei 120° gerührt. Nach Abkühlen auf Raumtemperatur wird mit 20 ml Methylenchlorid verdünnt und filtriert. Das Filtrat wird mit Wasser gewaschen, über Natriumsulfat getrocknet und eingedampft. Der Rückstand wird an 70 g Kieselgel mit Hexan/Aether (2:1) chromatographiert. Man erhält Hexyl-(3-cyano-4,5-dimethoxyphenyl)glyoxylat als Oel.
c) Zu 4,0 g Hexyl-(3-cyano-4,5-dimethoxyphenyl)glyoxylat, gelöst in 100 ml Methylenchlorid, werden unter Eiskühlung innert 20 Minuten 4,8 ml Bortribromid gelöst in 20 ml Methylenchlorid zugetropft, und das Reaktionsgemisch wird während 18 Stunden bei Raumtemperatur gerührt. Anschliessend werden bei -60° 40 ml Methanol zugetropft. 1 Stunde bei Raumtemperatur gerührt und eingedampft. Der Rückstand wird in Methanol aufgenommen. Es wird 10 Minuten unter Rückfluss erhitzt, zur Trockene eingedampft und im Hochvakuum getrocknet. Das so erhaltene Rohprodukt wird aus Acetonitril umkristallisiert. Man erhält Methyl-(3-cyano-4,5-dihydroxyphenyl)glyoxylat vom Smp. 252°.

### Beispiel 51

1.075 g Methyl-(3-cyano-4,5-dihydroxyphenyl)glyoxylat und 0.60 g 2-Amino-p-kresol werden in 2 ml Dimethylformamid während 70 Minuten bei 120° gerührt. Anschliessend wird auf Raumtemperatur abgekühlt und mit 15 ml Wasser verdünnt. Der Niederschlag wird abfiltriert und 6 Stunden bei 80° im Wasserstrahlvakuum getrocknet. Nach Umkristallisieren aus Acetonitril erhält man 2,3-Dihydroxy-5-(6-methyl-2-oxo-2H-1,4-benzoxazin -3-yl)benzonitril vom Smp. 278-280°.

### Beispiel 52

a) Eine Lösung von 2,5 g (10,2 mMol) 3,4-Dimethoxy-5-nitrobenzoylchlorid in 10 ml absolutem Tetrahydrofuran wird mit 1,1 ml Isocyanessigsäureäthylester und anschliessend mit einer Lösung von 3,0 ml Triäthylamin in 10 ml Tetrahydrofuran versetzt und während 48 Stunden bei Raumtemperatur gerührt. Nach Abdestillieren des Lösungsmittels wird mit Essigester/Wasser extrahiert. Das nach Eindampfen erhaltene Rohprodukt wird an der 20-fachen Menge Kieselgel mit Essigester chromatographiert. Nach Umkristallisieren aus Essigester/Hexan erhält man Aethyl-5-(3,4-dimethoxy-5-nitrophenyl)-4-oxazolcarboxylat in Form gelber Kristalle vom Smp. 109-110°.
b) 1,0 g (3,1 mMol) Aethyl-5-(3,4-dimethoxy-5-nitrophenyl)-4-oxazolcarboxylat werden mit 10 ml konstant siedender Bromwasserstoffsäure versetzt und während 2 Stunden bei 140° gerührt. Nach Abdestillieren der überschüssigen Bromwasserstoffsäure wird der gelbe Rückstand aus Aethanol/Aceton umgelöst. Man erhält 2-Amino-3',4'-dihydroxy-5'-nitroacetophenon-Hydrobromid in Form gelber Kristalle vom Smp. >250° (Zers.).

### Beispiel 53

a) 10 g (34 mMol) Aethyl-(3,4-dimethoxy-5-nitrobenzoyl)acetat werden in 100 ml Aethanol suspendiert, mit 1,7 g (37 mMol) Methylhydrazin versetzt und während 16 Stunden unter Rückfluss erhitzt. Nach Abdestillieren von ca. 50 ml Aethanol wird auf 0° abgekühlt, und der ausgefallene Niederschlag wird abgesaugt. Nach Umkristallisieren aus Aethanol erhält man 3-(3,4-Dimethoxy-5-nitrophenyl)-1-methylpyrazol-5-ol in Form gelber Kristalle vom Smp. 200-202°.
b) 2,0 g (7,2 mMol) 3-(3,4-Dimethoxy-5-nitrophenyl)-1-methylpyrazol-5-ol werden in 100 ml Methylenchlorid suspendiert. Nach Abkühlen auf -40° wird eine Lösung von 4,9 ml Bortribromid in 60 ml Methylenchlorid innert einer Stunde zugetropft. Anschliessend wird während 16 Stunden bei Raumtemperatur gerührt, auf -20° abgekühlt und innert 30 Minuten mit 100 ml Aethanol versetzt. Nach Rühren bei Raumtemperatur während einer Stunde wird das Lösungsmittel im Wasserstrahlvakuum bei 40° abdestilliert. Der Rückstand wird dreimal mit einem Gemisch von je 100 ml Aethanol/Toluol versetzt, wobei man das Lösungsmittel jeweils abdestilliert. Der Rückstand wird aus Aethanol umkristallisiert. Man erhält 5-(5-Hydroxy-1-methylpyrazol-3-yl) -3-nitropyrocatechol-Hydrobromid in Form gelber Kristalle vom Smp. >250°.

### Beispiel 54

a) 16,8 g (0,7 g-Atom) Magnesium werden mit 15 ml Aethanol versetzt, und nach Zugabe von 2 ml Tetrachlorkohlenstoff wird die Reaktion durch Erwärmen gestartet. Unter Rühren tropft man innert ca. 30 Minuten eine Lösung von 130,3 g tert.Butyläthylmalonat in 70 ml Aethanol und 600 ml absolutem Aether so zu, dass die Reaktion bei der Rückflusstemperatur abläuft. Anschliessend wird während 3 Stunden bei 50° weitergerührt, und das Lösungsmittel wird bei 40° im Wasserstrahlvakuum abdestilliert. Der Rückstand wird in 900 ml Tetrahydrofuran gelöst. Zu dieser Lösung tropft man unter Rühren bei 50° eine Lösung von 170 g (0,7 Mol) 3,4-Dimethoxy-5-nitrobenzoylchlorid in 700 ml absolutem Tetrahydrofuran zu und rührt während einer Stunde bei der Rückflusstemperatur. Das Lösungsmittel wird bei 40° im Wasserstrahlvakuum abdestilliert. Der Rückstand wird mit 1 l Aether versetzt. Unter Kühlen und Rühren gibt man 260 ml 3N-Schwefelsäure dazu und rührt während 30 Minuten. Die wässrige Phase wird zweimal mit je 600 ml Aether extrahiert. Die organische Phase wird mit Wasser neutral gewaschen, über Natriumsulfat getrocknet und eingedampft. Das erhaltene braune Oel wird mit Toluol über 1 kg Kieselgel filtriert. Das erhaltene Gemisch, bestehend aus Aethyl-tert.butyl-(3,4-dimethoxy-5-nitrobenzoyl)malonat und Aethyl-(3,4-dimethoxy-5-nitrobenzoyl)acetat, wird in 600 ml Methylenchlorid gelöst und unter Rühren innert ca. 30 Minuten mit 193 ml Trifluoressigsäure versetzt. Anschliessend wird während 2 Stunden bei 40° gerührt und dann bei 40° im Wasserstrahlvakuum eingedampft. Das erhaltene Oel wird mit Aether/Wasser extrahiert. Die organische Phase wird über Natriumsulfat getrocknet und eingedampft. Nach Lösen in Diisopropyläther/Hexan und Kühlen werden die ausgefallenen Kristalle abgesaugt und aus Diisopropyläther umkristallisiert. Man erhält Aethyl-(3,4-dimethoxy-5-nitrobenzoyl)acetat in Form schwach gelblicher Kristalle vom Smp. 67-68°.
b) 10,0 g (33,6 mMol) Aethyl-(3,4-dimethoxy-5-nitrobenzoyl)acetat werden in Analogie zu Beispiel 53a mit 4,0 g (37 mMol) Phenylhydrazin umgesetzt. Nach Umkristallisieren aus Methylenchlorid/Aethanol erhält man 3-(3,4-Dimethoxy-5-nitrophenyl)-1-phenyl-2-pyrazolin-5-on in Form gelber Kristalle vom Smp. 190-192°.
c) In Analogie zu Beispiel 53b erhält man daraus mit Bortribromid das 5-(5-Hydroxy-1-phenylpyrazol-3-yl) -3-nitropyrocatechol-Hydrobromid in Form gelber Kristalle vom Smp. >220° (Zers.).

### Beispiel 55

20,1 g Diäthyl-(3,4-dimethoxy-5-nitrobenzoyl)malonat werden in 200 ml Methylenchlorid gelöst, worauf man auf -20° abkühlt und bei dieser Temperatur unter Rühren innert 15 Minuten eine Lösung von 68,1 g Bortribromid in 120 ml Methylenchlorid zutropft. Anschliessend wird über Nacht bei Raumtemperatur gerührt. Nach Abkühlen auf -20° wird mit 300 ml Aethanol versetzt und während 30 Minuten bei Raumtemperatur gerührt. Das Lösungsmittel wird im Wasserstrahlvakuum bei 40° abdestilliert. Der Rückstand wird mit 300 ml Eiswasser und Methylenchlorid versetzt. Die organische Phase wird mit Wasser gewaschen, über Natriumsulfat getrocknet und eingedampft. Das erhaltene Rohprodukt wird an der 10-fachen Menge Kieselgel mit Toluol chromatographiert. Nach Kristallisieren aus Methylenchlorid/Hexan erhält man Aethyl-(3,4-dihydroxy-5-nitrobenzoyl)acetat in Form gelber Kristalle vom Smp. 136-137°.

### Beispiel 56

2,0 g (7,4 mMol) Aethyl-(3,4-dihydroxy-5-nitrobenzoyl)acetat werden in 50 ml Aethanol suspendiert. Nach Versetzen mit 0,4 g Hydrazinhydrat wird während 16 Stunden bei der Rückflusstemperatur gehalten. Nach Abdestillieren des Lösungsmittels wird der Rückstand zusammen mit 50 ml Essigester während 5 Minuten in der Siedehitze gehalten. Der ausgefallene Niederschlag wird abgesaugt, und das Filtrat wird auf 10 ml eingeengt. Die in der Kälte ausgefallenen Kristalle werden abgesaugt. Man erhält 5-(5-Hydroxypyrazol-3-yl)-3-nitropyrocatechol in Form oranger Kristalle vom Smp. 228° (Zers.).

### Beispiel 57

7,3 g (36,66 mMol) 3,4-Dihydroxy-5-nitrobenzoesäure werden mit 30 ml Essigsäureanhydrid versetzt, worauf man während 8 Stunden bei der Rückflusstemperatur hält. Das Reaktionsgemisch wird auf Eis gegossen. Der ausgefallene Niederschlag wird abgesaugt, mit Wasser gewaschen und in Methylenchlorid aufgenommen. Die organische Phase wird über Natriumsulfat getrocknet und eingedampft. Der erhaltene Rückstand wird aus Methylenchlorid/n-Hexan in der Kälte umkristallisiert. Man erhält 3,4-Diacetoxy-5-nitrobenzoesäure in Form farbloser Kristalle vom Smp. 126-127°.

### Beispiel 58

a) 9.7 g (32,2 mMol) 3,4-Diacetoxy-5-nitrobenzoesäure werden mit 12,5 ml Thionylchlorid versetzt, worauf man während 1,5 Stunden bei 100° rührt. Nach Abdestillieren des überschüssigen Thionylchlorids wird das 5-(Chlorcarbonyl)-2-nitro-o-phenylendiacetat destilliert, Sdp. 160° (26,7 Pa).
b) 3,2 g (10,6 mMol) 5-(Chlorcarbonyl)-2-nitro-o-phenylendiacetat werden in 50 ml Dimethylformamid gelöst. Die eiskalte Lösung wird unter Rühren mit einer Lösung von 2,2 ml Diäthylamin in 20 ml Dimethylformamid versetzt. Anschliessend wird während einer Stunde bei Raumtemperatur gerührt, worauf man das Lösungsmittel im Wasserstrahlvakuum bei 50° abdestilliert. Der erhaltene Rückstand wird mit Wasser und Methylenchlorid versetzt. Die organische Phase wird über Natriumsulfat getrocknet und eingedampft. Das erhaltene gelbe Harz wird aus Methylenchlorid/Aether kristallisiert. Man erhält N,N-Diäthyl-3,4-dihydroxy-5-nitrobenzamid in Form gelber Kristalle vom Smp. 145-146°.

### Beispiel 59

3,2 g (10,6 mMol) 5-(Chlorcarbonyl)-2-nitro-o-phenylendiacetat werden in 50 ml Dimethylformamid gelöst, worauf man bei 0-5° unter Rühren und innert 40 Minuten mit einer Lösung von 3,02 ml 2,2-Diäthylaminoäthylamin in 20 ml Dimethylformamid versetzt. Anschliessend wird während 30 Minuten bei Raumtemperatur gerührt. Das Lösungsmittel wird im Wasserstrahlvakuum bei 60° abdestilliert. Der Rückstand wird zweimal mit je 20 ml Aethanol extrahiert, in heissem Aethanol gelöst und mit einem Ueberschuss an äthanolischer Salzsäure versetzt, worauf man eindampft. Nach Umkristallisieren aus Aethanol/Essigester erhält man N-[2-(Diäthylamino)äthyl]-3,4-dihydroxy -5-nitrobenzamid-Hydrochlorid in Form gelber Kristalle vom Smp. 139° (Zers.).

### Beispiel 60

a) 10,0 g (47,4 mMol) 2,3-Dimethoxy-5-nitrobenzaldehyd werden mit 50 ml Eisessig und 50 ml konstant siedender Bromwasserstoffsäure versetzt und während 7 Stunden bei der Rückflusstemperatur gehalten. Nach Versetzen mit Eis wird der ausgefallene Niederschlag abgesaugt, mit Wasser gewaschen und in Essigester aufgenommen. Die organische Phase wird über Natriumsulfat getrocknet und eingedampft. Das erhaltene Rohprodukt wird mit Essigester über Kieselgel filtriert. Nach Kristallisieren aus Essigester/Hexan erhält man 2,3-Dihydroxy-5-nitrobenzaldehyd in Form bräunlicher Kristalle vom Smp. 226-228°.
b) 4,5 g 2,3-Dihydroxy-5-nitrobenzaldehyd werden in 75 ml Wasser suspendiert. Nach Versetzen mit 4,2 g Hydroxylamin-o-sulfonsäure wird während 16 Stunden bei 65° gerührt. Nach Abkühlen werden die ausgefallenen Kristalle abgesaugt und mit Wasser gewaschen. Das Filtrat wird mit Essigester extrahiert. Die Kristalle und die getrocknete organische Phase werden vereinigt, worauf man eindampft und den Rückstand aus Diisopropyläther umkristallisiert. Man erhält 2,3-Dihydroxy-5-nitrobenzonitril in Form gelber Kristalle vom Smp. 186-188°.

### Beispiel 61

a) 4,0 g (19,2 mMol) 3,4-Dimethoxy-5-nitro-benzonitril werden in 50 ml Dimethylformamid gelöst, worauf man mit 1,66 g Ammoniumchlorid und 2,02 g Natriumazid versetzt und während 31 Stunden bei 125° rührt. Nach jeweils 8 und 15 Stunden wird noch einmal dieselbe Menge Ammoniumchlorid und Natriumazid dazugegeben. Nach dem Abkühlen wird auf Eis gegossen. Der ausgefallene Niederschlag wird abgesaugt, mit Wasser gewaschen und getrocknet. Man erhält 2-Methoxy-6-nitro-4-(1H-tetrazol-5-yl)phenol in Form oranger Kristalle vom Smp. >240° (Zers.).
b) 4,0 g (16,9 mMol) 2-Methoxy-6-nitro-4-(1H-tetrazol-5-yl)phenol werden mit 40 ml konstant siedender Bromwasserstoffsäure versetzt, worauf man unter Stickstoffatmosphäre während 8 Stunden bei 140° rührt. Nach dem Abkühlen wird auf Eis gegossen. Der ausgefallene Niederschlag wird abgesaugt und aus Aether umkristallisiert. Man erhält 3-Nitro-5-(1H-tetrazol-5-yl)pyrocatechol in Form organger Kristalle vom Smp. >240° (Zers.).

### Beispiel 62

Zu 130 ml konz. Schwefelsäure werden unter Rühren portionenweise innert 10 Minuten insgesamgt 7,2 g (40 mMol) 3,4-Dihydroxy-5-nitrobenzonitril eingetragen, worauf man während 4 Stunden bei 50° rührt. Das Reaktionsgemisch wird auf 800 ml Eiswasser gegossen. Der ausgefallene Niederschlag wird abgesaugt, mit Wasser gewaschen und in Essigester aufgenommen. Die organische Phase wird über Natriumsulfat getrocknet und eingedampft. Nach Umkristallisieren aus Aceton/Essigester erhält man 3,4-Dihydroxy-5-nitrobenzamid in Form oranger Kristalle vom Smp. 235-236°.

### Beispiel 63

a) Zu einer Suspension von 3,6 g (82,5 mMol) einer 55-proz. Natriumhydrid-Dispersion in 50 ml absolutem Dimethylformamid tropft man unter Argonatmosphäre innert 15 Minuten eine Lösung von 11,25 g (82,6 mMol) 2-Hydroxyacetophenon in 100 ml absolutem Dimethylformamid und rührt eine Stunde bei Raumtemperatur. Nach Abkühlen auf 0° tropft man innert 20 Minuten eine Lösung von 20,3 g (82,6 mMol) 3,4-Dimethoxy-5-nitrobenzoylchlorid in 100 ml absolutem Dimethylformamid dazu und rührt bei Raumtemperatur über Nacht. Das Reaktionsgemisch wird auf Eiswasser gegossen, worauf man zweimal mit je 250 ml Essigester extrahiert. Die organische Phase wird zweimal mit je 100 ml Kochsalzlösung extrahiert, über Natriumsulfat getrocknet und eingedampft. Das erhaltene braune Oel wird in 100 ml Toluol erwärmt. Der ausgefallene Niederschlag wird abgesaugt, und das Filtrat wird an der 30-fachen Menge Kieselgel mit Toluol/Essigester (4:1) chromatographiert. Nach Umkristallisieren aus Essigester/Hexan erhält man o-Acetylphenyl-3,4-dimethoxy-5-nitrobenzoat in Form gelblicher Kristalle vom Smp. 108-109°.
b) 10,0 g (29 mMol) o-Acetylphenyl-3,4-dimethoxy-5-nitrobenzoat werden in 50 ml Pyridin gelöst. Nach Versetzen mit 8,12 g (144 mMol) pulverisiertem und getrocknetem Kaliumhydroxid wird während 5 Minuten bei 80° gerührt. Nach dem Abkühlen wird auf Eis gegossen. Die wässrige Lösung wird durch Versetzen mit 3N-Salzsäure sauer gestellt. Der ausgefallene Niederschlag wird abgesaugt. Nach Umkristallisieren aus Essigester/Hexan erhält man 1-(o-Hydroxyphenyl)-3-(3,4-dimethoxy-5-nitrophenyl) -1,3-propandion in Form gelblicher Kristalle vom Smp. 188-189°.
c) Zu einer Lösung von 500 mg (1,45 mMol) 1-(o-Hydroxyphenyl)-3-(3,4-dimethoxy-5-nitrophenyl) -1,3-propandion in 50 ml Methylenchlorid wird unter Rühren und Argonatmosphäre bei -20° eine Lösung von 1,82 g (0,7 ml) Bortribromid in 20 ml Methylenchlorid innert ca. 20 Minuten dazugetropft, worauf man über Nacht bei Raumtemperatur rührt. Nach Abkühlen auf -20° wird tropfenweise mit 25 ml Aethanol versetzt und bei 40° im Wasserstrahlvakuum eingedampft. Nach Umlösen aus Aethanol erhält man 1-(3,4-Dihydroxy-5-nitrophenyl)-3-(o-hydroxyphenyl) -1,3-propandion in Form gelber Kristalle vom Smp. 251-252°.

### Beispiel 64

a) Eine Lösung von 2,0 g (5,79 mMol) o-Acetylphenyl-3,4-dimethoxy-5-nitrobenzoat in 12,5 ml Eisessig wird mit 0,94 g Natriumacetat versetzt und während 4 Stunden bei der Rückflusstemperatur gehalten. Nach dem Abkühlen wird auf Eiswasser gegossen. Die ausgefallenen Kristalle werden abgesaugt. Nach Umkristallisieren aus Essigester/Hexan erhält man 2-(3,4-Dimethoxy-5-nitrophenyl)-4H-1-benzopyran-4-on in Form farbloser Kristalle vom Smp. 216-217°.
b) Zu einer Losung von 1,0 g (2,9 mMol) 2-(3,4-Dimethoxy-5-nitrophenyl)-4H-1-benzopyran-4-on in 100 ml Methylenchlorid werden bei -10° unter Argonatmosphäre eine Lösung von 10 ml Bortribromid in 50 ml Methylenchlorid innert 30 Minuten zugetropft, worauf man über Nacht bei Raumtemperatur rührt. Nach Abkühlen auf -20° werden 20 ml Aethanol dazugetropft. Dann wird bei 40° im Wasserstrahlvakuum eingedampft. Der erhaltene gelbe Rückstand wird mit Wasser/Essigester extrahiert. Die organische Phase wird mit Wasser gewaschen, über Natriumsulfat getrocknet und eingedampft. Nach Umkristallisieren aus Aethanol/Essigester erhält man 2-(3,4-Dihydroxy-5-nitrophenyl)-4H-1-benzopyran-4-on in Form gelber Kristalle vom Smp. >240° (Zers.).

### Beispiel 65

a) Zu 33,0 g 4-Brombenzotrifluorid (gelöst in 150 ml Tetrahydrofuran) werden bei -70° innert 20 Minuten 92 ml n-Butyllithiumlösung (1,6 M in Hexan) zugetropft. Nach 45 Minuten Rühren bei -70° werden 36 g 3-Methoxy-4-benzyloxybenzaldehyd (gelöst in 100 ml Tetrahydrofuran) zwischen -70° und -60° zugetropft. Das Reaktionsgemisch wird 2 Stunden bei -70° und 1 Stunde bei 0° gerührt, auf ein Gemisch von Eis und 100 ml 2N-Schwefelsäure gegossen und zweimal mit 500 ml Aether extrahiert. Die vereinigten Aetherphasen werden mit gesättigter Kochsalzlösung gewaschen, über Natriumsulfat getrocknet und eingedampft. Man erhält 4-(Benzyloxy)-3-methoxy-4'-(trifluoromethyl)benzhydrol, das direkt in die anschliessende Reaktionsstufe eingesetzt werden kann.
b) 52,6 g 4-(Benzyloxy)-3-methoxy-4'-(trifluoromethyl)benzhydrol (gelöst in 500 ml Methylenchlorid) werden innert 10 Minuten bei 20° mit 30,6 g Pyridiniumchlorochromat versetzt und während 2 Stunden bei 20° gerührt. Anschliessend wird der gebildete Niederschlag abfiltriert und mit Methylenchlorid gewaschen. Das Filtrat wird eingedampft, und der Rückstand wird an 150 g Kieselgel mit Methylenchlorid chromatographiert. Man erhält nach Umkristallisieren aus Methylenchlorid/Hexan 4-(Benzyloxy)-3-methoxy-4'-(trifluoromethyl)benzophenon vom Schmelzpunkt 101°.
c) Zu 20 g 4-(Benzyloxy)-3-methoxy-4'-(trifluoromethyl)benzophenon (gelöst in 150 ml Methylenchlorid) werden bei 10° 70 ml 33-proz. Bromwasserstoffsäure in Essigsäure innert 15 Minuten zugegeben. Nach 1,5 Stunden Rühren bei 20° wird das Reaktionsgemisch auf 600 ml Eiswasser gegossen; die Methylenchloridphase wird abgetrennt und die wässrige Phase wird noch zweimal mit 100 ml Methylenchlorid extrahiert. Die vereinigten Methylenchloridphasen werden mit 600 ml Wasser gewaschen, über Natriumsulfat getrocknet und eingedampft. Der Rückstand wird an 150 g Kieselgel mit Methylenchlorid chromatographiert. Man erhält nach Umkristallisieren aus Methylenchlorid/Hexan 4-Hydroxy-3-methoxy-4'-(trifluoromethyl)benzophenon vom Schmelzpunkt 97°.
d) Zu 12,8 g 4-Hydroxy-3-methoxy-4'-(trifluoromethyl)benzophenon (gelöst in 160 ml Essigsäure) werden innert 10 Minuten bei 20° 3,2 ml 65-proz. Salzpetersäure zugetropft. Nach 1,5 Stunden Rühren wird das Reaktionsgemisch auf 600 ml Eiswasser gegossen, und der gebildete Niederschlag wird abfiltriert, mit Wasser gewaschen und in Methylenchlorid gelöst. Die Methylenchloridlösung wird über Natriumsulfat getrocknet und eingedampft, und der Rückstand wird aus Methylenchlorid/Hexan umkristallisiert. Man erhält 4-Hydroxy-3-methoxy-5-nitro-4'-(trifluoromethyl)benzophenon vom Schmelzpunkt 172°.
e) 2,0 g 4-Hydroxy-3-methoxy-5-nitro-4'-(trifluoromethyl)benzophenon (gelöst in 20 ml 33-proz. Bromwasserstoffsäure in Essigsäure) werden während 18 Stunden bei 90° gerührt. Hierauf werden 20 ml 48-proz. wässrige Bromwasserstoffsäure zugesetzt, worauf man weitere 18 Stunden bei 110° rührt. Anschliessend wird das Reaktionsgemisch unter vermindertem Druck eingedampft, und der Rückstand wird aus Wasser kristallisiert. Man erhält 3,4-Dihydroxy-5-nitro-4'-(trifluoromethyl)benzophenon vom Schmelzpunkt 116-118°.

### Beispiel 66

a) 18,7 g 4-Hydroxy-3-methoxy-5-nitro-4'-(trifluoromethyl)benzophenon (gelöst in 250 ml Tetrahydrofuran) werden bei Raumtemperatur mit 27,5 ml 2N-Kaliumhydroxidlösung versetzt, worauf man eindampft. Der Rückstand wird mit 200 ml Toluol versetzt, worauf man erneut eindampft. Hierauf wird mit 400 ml Toluol 4 Stunden unter Wasserabscheidung und unter Rückfluss erwärmt. 100 ml Toluol werden abdestilliert und 10 ml Dimethylformamid und 20 ml Dimethylsulfat (frisch destilliert) werden zugesetzt, worauf man 5 Stunden unter Rückfluss erwärmt. Anschliessend werden bei 20° 300 ml 1N-Natronlauge zugefügt. Das Reaktionsgemisch wird 30 Minuten gerührt und mit 200 ml Aether versetzt. Die organische Phase wird abgetrennt; die wässrige Phase wird noch zweimal mit 100 ml Aether extrahiert; die vereinigten Aetherphasen werden über Natriumsulfat getrocknet und eingedampft, und der Rückstand wird an 70 g Kieselgel mit Methylenchlorid chromatographiert. Man erhält nach Umkristallisieren aus Methylenchlorid/Hexan 3,4-Dimethoxy-5-nitro-4'-(trifluoromethyl)benzophenon vom Schmelzpunkt 115°.
b) Zu 16,0 g 3,4-Dimethoxy-5-nitro-4'-(trifluoromethyl)benzophenon (gelöst in 300 ml Aethanol) werden 49,5 g Zinndichlorid-Dihydrat zugefügt, worauf man 30 Minuten bei 75° rührt. Hierauf wird das Reaktionsgemisch auf 800 ml Eiswasser gegossen. Es wird mit 28-proz. Natriumhydroxidlösung neutralisiert und dreimal mit 600 ml Methylenchlorid extrahiert. Die vereinigten Methylenchloridphasen werden mit Wasser gewaschen, über Natriumsulfat getrocknet und eingedampft. Man erhält nach Umkristallisieren aus Methylenchlorid/Hexan 5-Amino-3,4-dimethoxy-4'-(trifluoromethyl)benzophenon vom Schmelzpunkt 95-96°.
c) 3,25 g 5-Amino-3,4-dimethoxy-4'-(trifluoromethyl)benzophenon (gelöst in 50 ml Aceton) werden nach Zugabe von 15 ml 2N-Schwefelsäure unter reduziertem Druck eingedampft. Der so erhaltene Rückstand wird in 20 ml Essigsäure suspendiert, mit 100 ml Wasser verdünnt und bei 5° mit einer Lösung von 700 mg Natriumnitrit in 10 ml Wasser versetzt. Es wird eine Stunde bei 5° gerührt. Hierauf wird die Diazoniumsalzlösung filtriert, bei 5° zu einer Lösung von 2,0 g Natriumcyanid und 1,0 g Kupfer(I)cyanid in 20 ml Wasser zugegeben und 1 Stunde bei 5° gerührt. Hierauf werden 200 ml Methylenchlorid hinzugefügt. Unlösliche Anteile werden abfiltriert. Die Phasen werden getrennt; die wässrige Phase wird noch zweimal mit 100 ml Methylenchlorid extrahiert. Die vereinigten Methylenchloridphasen werden mit Wasser gewaschen, über Natriumsulfat getrocknet und eingedampft. Der Rückstand wird an 30 g Kieselgel mit Methylenchlorid chromatographiert. Man erhält nach Umkristallisieren aus Methylenchlorid/Hexan 5-Cyano-3,4-dimethoxy-4'-(trifluoromethyl)benzophenon vom Schmelzpunkt 130°.
d) 1,5 g 5-Cyano-3,4-dimethoxy-4'-(trifluoromethyl)benzophenon (gelöst in 75 ml Methylenchlorid) werden bei 5° mit 2,18 ml Bortribromid versetzt, worauf man 18 Stunden bei Raumtemperatur rührt. Anschliessend wird das Reaktionsgemisch mit 50 ml Methylenchlorid verdünnt. Man erhitzt weitere 4 Stunden unter Rückfluss, versetzt bei -70° mit 15 ml Methanol, rührt 2 Stunden bei Raumtemperatur, dampft ein, trocknet den Rückstand im Vakuum und verteilt zwischen Aethylacetat und Eiswasser. Die wässrige Phase wird noch zweimal mit Aethylacetat ausgezogen. Die vereinigten Aethylacetatphasen werden über Natriumsulfat getrocknet und eingedampft. Das so erhaltene Material wird mit 10 ml Essigsäureanhydrid und 1 ml Pyridin während 6 Stunden bei 130° gerührt. Es wird eingedampft und der Rückstand wird zwischen Eiswasser und Methylenchlorid verteilt. Die Methylenchloridphase wird über Natriumsulfat getrocknet und eingedampft, und der Rückstand wird an 30 g Kieselgel mit Methylenchlorid chromatographiert. Das so erhaltene Diacetat wird in 10 ml Methanol gelöst. Es wird mit 4,2 ml 1N-Natronlauge versetzt, 1 Stunde bei 0° gerührt, mit Essigsäure neutralisiert, eingedampft und zwischen Aethylacetat und gesättigter Kochsalzlösung verteilt. Die Aethylacetatphasen werden über Natriumsulfat getrocknet und eingedampft, und der Rückstand wird aus Methylenchlorid umkristallisiert. Man erhält 5-Cyano-3,4-dihydroxy-4'-(trifluoromethyl)benzophenon vom Schmelzpunkt 204-206°.
   In analoger Weise erhält man:
   a1) Aus 2'-Fluor-4-hydroxy-3-methoxy-5-nitrobenzophenon das 3,4-Dimethoxy-2'-fluor-5-nitrobenzophenon vom Smp. 86-88° (aus Aether/Petroläther, ts.),
   b1) aus 3,4-Dimethoxy-2'-fluor-5-nitrobenzophenon das 5-Amino-3,4-dimethoxy-2'-fluorbenzophenon vom Smp. 93-95° (aus Aether/Petroläther, ts.),
   c1) aus 5-Amino-3,4-dimethoxy-2'-fluorbenzophenon das 5-Benzoyl-2,3-dimethoxy-2'-fluorbenzonitril vom Smp. 132-134° (aus Methylenchlorid/Petroläther, ts) und
   d1) aus 5-Benzoyl-2,3-dimethoxy-2'-fluorbenzonitril das 5-Benzoyl-2,3-dihydroxy-2'-fluorbenzonitril vom Smp. 228-230° (aus Aether/Petroläther, ts.).
      In analoger Weise erhält man:
   b2) Aus 3,4-Dimethoxy-5-nitrobenzophenon das 5-Amino-3,4-dimethoxybenzophenon als amorphen Festkörper,
   c2) aus 5-Amino-3,4-dimethoxybenzophenon das 5-Benzoyl-2,3-dimethoxybenzonitril vom Smp. 98-100° (aus Aether/Hexan) und
   d2) aus 5-Benzoyl-2,3-dimethoxybenzonitril das 5-Benzoyl -2,3-dihydroxybenzonitril vom Smp. 212-214° (aus Essigester/Aether).

### Beispiel 67

a) Zu einer Lösung von 2,68 g (32,64 mMol) 1-Methylimidazol und 6,6 g (65,14 mMol) Triäthylamin in 80 ml Pyridin wird eine Lösung von 16,0 g (65,14 mMol) 3,4-Dimethoxy-5-nitrobenzoylchlorid in 80 ml Pyridin zugetropft, worauf man während 3 Stunden bei 60° rührt. Nach Versetzen mit 1,70 ml 3N-Natronlauge wird während 1 Stunde weitergerührt und anschliessend auf Eiswasser gegossen. Die ausgefallenen grauen Kristalle werden abgesaugt und in Essigester aufgenommen, worauf man die organische Phase über Magnesiumsulfat trocknet und das Lösungsmittel abdestilliert. Nach Umkristallisieren aus Essigester/Hexan erhält man 3,4-Dimethoxy-5-nitrophenyl (1-methylimidazol-2-yl)keton in Form farbloser Kristalle vom Smp. 144-145°.
b) 5,0 g (17,17 mMol) 3,4-Dimethoxy-5-nitrophenyl (1-methylimidazol-2-yl)keton werden mit 50 ml Bromwasserstoffsäure (48%) versetzt, worauf man während 2 Stunden unter Rückflusstemperatur rührt. Nach dem Abkühlen wird der ausgefallene Niederschlag abgesaugt, mit Eiswasser gewaschen und aus Aethanol umkristallisiert. Man erhält 3,4-Dihydroxy-5-nitrophenyl (1-methylimidazol-2-yl)keton-Hydrobromid in Form gelber Kristalle vom Zersetzungspunkt >240°.

### Beispiel 68

In Analogie zu Beispiel 67 erhält man aus 3,4-Dimethoxy-5-nitrobenzoylchlorid und 1-Benzylimidazol das 1-Benzylimidazol-2-yl (3,4-dimethoxy-5-nitrophenyl)keton in Form farbloser Kristalle vom Smp. 134-135° (aus Methylenchlorid/Hexan) und daraus mit Bromwasserstoffsäure das 1-Benzylimidazol-2-yl (3,4-dihydroxy-5-nitrophenyl)keton-Hydrobromid als gelbe Kristalle vom Smp. 218-219° (Zers.).

### Beispiel 69

a) Zu einer Lösung von 13,0 g (53,13 mMol) 3,4-Dimethoxy-5-nitrobenzoylchlorid und 5,4 g (53,13 mMol) Triäthylamin in 80 ml Acetonitril wird innert 10 Minuten eine Lösung von 10,0 g (53,13 mMol) 1-[(Benzyloxy)methyl]imidazol in 50 ml Acetonitril unter Eiskühlen so zugetropft, dass die Temperatur 25° nicht übersteigt. Anschliessend wird 3 Stunden weitergerührt, worauf man das Lösungsmittel abdestilliert. Nach Versetzen mit Wasser wird mit Essigester extrahiert. Nach zweimaligem Waschen mit Wasser wird die organische Phase über Natriumsulfat getrocknet und eingedampft. Das erhaltene Oel wird an 600 g Kieselgel mit Toluol/Essigester (95:5) chromatographiert. Man erhält 1-[(Benzyloxy)methyl)]imidazol-2-yl (3,4-dimethoxy-5-nitrophenyl)keton als gelbliches Oel.
b) In Analogie zu Beispiel 67b) erhält man nach Behandeln mit Bromwasserstoffsäure das 3,4-Dihydroxy-5-nitrophenyl (imidazol-2-yl)keton-Hydrobromid als gelbe Kristalle vom Smp. 247-248°.

### Beispiel 70

a) Eine Lösung von 25,0 g (120,16 mMol) 3-Bromchinolin werden in 200 ml trockenem Aether gelöst und auf -60° abgekühlt. Bei dieser Temperatur werden innert 15 Minuten 75,1 ml (120,16 mMol) einer 1,6-molaren Lösung von n-Butyllithium zugetropft, worauf man während 10 Minuten rührt. Dazu tropft man bei -60° eine Lösung von 26,5 g (109,2 mMol) Vanillinbenzyläther in 250 ml trockenem Aether, rührt anschliessend während 3 Stunden bei Raumtemperatur, giesst auf ca. 1,5 l Eiswasser und extrahiert dreimal mit je 600 ml Essigester. Die organische Phase wird mit Wasser gewaschen, über Natriumsulfat getrocknet und eingedampft. Das erhaltene Oel wird an 1 kg Kieselgel mit Methylenchlorid/Essigester (1:1) chromatographiert. Das erhaltene kristalline Rohprodukt wird aus Essigester umgelöst Man erhält α-[4-(Benzyloxy)-3-methoxyphenyl]-3-chinolinmethanol in Form farbloser Kristalle vom Smp. 124-125°.
b) Eine Lösung von 6,2 g (16,7 mMol) α-[4-(Benzyloxy)-3-methoxyphenyl]-3-chinolinmethanol in 200 ml Methylenchlorid wird nach Versetzen mit 62 g Mangandioxyd während 2 Stunden bei der Rückflusstemperatur gerührt. Nach dem Abkühlen wird der Niederschlag abgesaugt und mit Methylenchlorid gewaschen. Das Filtrat wird eingedampft und das erhaltene Oel wird in heissem Aether gelöst, worauf man mit wenig Pentan versetzt. Die ausgefallenen Kristalle werden abgesaugt. Man erhält 4-(Benzyloxy)-3-methoxyphenyl (3-chinolin)keton in Form farbloser Kristalle vom Smp. 110-111°.
c) 11,0 g (29,78 mMol) 4-(Benzyloxy)-3-methoxyphenyl (3-chinolin)keton werden mit 50 ml Trifluoressigsäure versetzt, worauf man während 2 Stunden bei Raumtemperatur rührt. Nach Abdestillieren der Trifluoressigsäure wird zweimal mit je 50 ml Aethanol versetzt und das Lösungsmittel jeweils abdestilliert. Das erhaltene Oel kristallisiert beim Versetzen mit Aethanol. Nach Umkristallisieren aus Aethanol erhält man 4-Hydroxy-3-methoxyphenyl (3-chinolinyl)keton in Form gelblicher Kristalle vom Smp. 196-197°.
d) Zu einer Lösung von 5,5 g (19,69 mMol) 4-Hydroxy-3-methoxyphenyl (3-chinolinyl)keton in 300 ml Eisessig tropft man bei 15° eine Lösung von 1,91 ml 65-proz. Salpetersäure in 20 ml Eisessig und rührt dann während 2 Stunden bei dieser Temperatur weiter. Das Ganze wird dann auf Eiswasser gegossen und dreimal mit je 300 ml Essigester extrahiert. Die organische Phase wird fünfmal mit je 100 ml Wasser gewaschen, über Natriumsulfat getrocknet und eingedampft. Nach Versetzen des Rückstandes mit Essigester erhält man 4-Hydroxy-3-methoy-5-nitrophenyl (3-chinolinyl)keton in Form gelber Kristalle vom Smp. 220-221° (Zers.).
e) 820 mg (2,53 mMol) 4-Hydroxy-3-methoxy-5-nitrophenyl (3-chinolinyl)keton werden mit 50 ml 48-proz. Bromwasserstoffsäure versetzt und während 3 Stunden bei der Rückflusstemperatur gehalten. Nach Abdestillieren der Bromwasserstoffsäure bei 50° wird der Rückstand mit 70 ml heissem Wasser versetzt, und der unlösliche Teil wird abgesaugt. Man erhält 3,4-Dihydroxy-5-nitrophenyl (3-chinolinyl)keton-Hydrobromid in Form gelber Kristalle vom Smp. 270° (Zers.).

### Beispiel 71

In Analogie zu Beispiel 70 erhält man α-[4-(Benzyloxy)-3-methoxyphenyl]-4-chinolinmethanol als farblose Kristalle vom Smp. 117-118° (Essigester), daraus mit Mangandioxyd das 4-(Benzyloxy)-3-methoxyphenyl (4-isochinolinyl)keton als farblose Kristalle vom Smp. 126,5-127,5°, daraus mit Trifluoressigsäure das 4-Hydroxy-3-methoxyphenyl (4-isochinolinyl)keton als gelbe Kristalle vom Smp. 197,5-198,5° und daraus durch Nitrieren mit Salpetersäure in Eisessig und anschliessendem Behandeln mit Bromwasserstoffsäure das 3,4-Dihydroxy-5-nitrophenyl (4-isochinolinyl)keton-Hydrobromid als gelbe Kristalle vom Smp. 256° (Zers.).

### Beispiel 72

In Analogie zu Beispiel 70 erhält man α-[4-(Benzyloxy)-3-methoxyphenyl]-2-naphthalinmethanol als farblose Kristalle (Essigester/Hexan) vom Smp. 113-114°, daraus mit Mangandioxyd das 4-(Benzyloxy)-3-methoxyphenyl (2-naphthyl)keton als farblose Kristalle (Essigester/Hexan) vom Smp. 104-105°, daraus durch Behandeln mit Trifluoressigsäure und Nitrierung mit Salpetersäure in Eisessig das 4-Hydroxy-3-methoxy-5-nitrophenyl (2-naphthyl)keton als gelbe Kristalle vom Smp. 187-188° und daraus durch Behandeln mit Bromwasserstoffsäure das 3,4-Dihydroxy-5-nitrophenyl (2-naphthyl)keton als gelbe Kristalle vom Smp. 184-185°.

### Beispiel 73

a) Eine Lösung von 20,0 g (100,5 mMol) 3-Phenylpropylbromid in 300 ml Aether wird bei -60° unter Rühren innert 15 Minuten mit einer Lösung von 71,8 ml (100,5 mMol) tert.-Butyllithium (1,4 molar) in Pentan versetzt. Nach 10 Minuten wird bei dieser Temperatur eine Lösung von 22,14 g (91,36 mMol) Vanillinbenzyläther in 200 ml Aether innert 15 Minuten zugetropft, worauf man 2 Stunden weiterrührt. Der Ansatz wird auf 1 l Eiswasser gegossen und dreimal mit je 500 ml Essigester extrahiert. Die organische Phase wird zweimal mit je 200 ml Wasser gewaschen, über Natriumsulfat getrocknet und eingedampft. Das erhaltene Oel wird an 1 kg Kieselgel mit Methylenchlorid chromatographiert. Die erhaltenen Kristalle werden aus Aether/Pentan umgelöst. Man erhält 4-(Benzyloxy)-3-methoxy-α-(3-phenylpropyl)benzylalkohol in Form farbloser Kristalle vom Smp. 71-73°.
b) Eine Lösung von 9,0 g (24,83 mMol) 4-(Benzyloxy)-3-methoxy-α-(3-phenylpropyl)benzylalkohol in 250 ml Methylenchlorid wird mit 90 g Mangandioxyd versetzt und während 2 Stunden bei der Rückflusstemperatur gehalten. Nach dem Abkühlen wird der Niederschlag abgesaugt und mit Methylenchlorid gewaschen. Das Filtrat wird eingedampft, und der Rückstand wird aus Essigester/Aether umkristallisiert. Man erhält 4'-(Benzyloxy)-3'-methoxy-4-phenylbutyrophenon in Form farbloser Kristalle vom Smp. 81-82°.
c) Eine Lösung von 7,0 g (19,42 mMol) 4'-(Benzyloxy)-3'-methoxy-4-phenylbutyrophenon in 40 ml 33-proz. Bromwasserstoffsäure in Eisessig wird während 5 Stunden bei Raumtemperatur gerührt und anschliessend auf 500 ml Eiswasser gegossen. Durch Zugabe von konz. Ammoniak wird die Lösung auf pH 6,0 gestellt und dreimal mit je 250 ml Essigester extrahiert. Die organische Phase wird dreimal mit je 50 ml Wasser gewaschen, über Natriumsulfat getrocknet und eingedampft. Das erhaltene Oel wird in 20 ml Aether gelöst, worauf man bis zur Trübung mit Hexan versetzt und auskristallisieren lässt. Man erhält farbloses 4'-Hydroxy-3'-methoxy-4-phenylbutyrophenon vom Smp. 91-92°.
d) Zu einer Lösung von 2,2 g (8,14 mMol) 4'-Hydroxy-3'-methoxy-4-phenylbutyrophenon in 25 ml Eisessig tropft man eine Lösung von 0,79 ml 65-proz. Salpetersäure in 25 ml Eisessig zu und rührt während 2 Stunden bei Raumtemperatur weiter. Das Ganze wird auf 150 ml Eiswasser gegossen und nach Versetzen mit 20 ml 3N-Salzsäure dreimal mit je 75 ml Essigester extrahiert. Die organische Phase wird mit Wasser gewaschen, über Natriumsulfat getrocknet und eingedampft. Das erhaltene Rohprodukt wird in Essigester aufgenommen und über 75 g Kieselgel filtriert. Nach Umkristallisieren aus Acetonitril erhält man 4'-Hydroxy-3'-methoxy-5'-nitro-4-phenylbutyrophenon in Form gelber Kristalle vom Smp. 120-121°.
e) 1,0 g (3,2 mMol) 4'-Hydroxy-3'-methoxy-5'-nitro-4-phenylbutyrophenon werden mit 8 g Pyridin-Hydrochlorid während 1 Stunde bei 200° gehalten. Das Reaktionsgemisch wird noch warm auf Eiswasser gegossen und mit Essigester extrahiert. Die organische Phase wird mit 1N-Salzsäure und anschliessend mit Wasser gewaschen, über Natriumsulfat getrocknet und eingedampft. Der erhaltene dunkle Rückstand wird an der 30-fachen Menge Kieselgel mit Essigester chromatographiert. Aus Methylenchlorid/Hexan erhält man 3',4'-Dihydroxy-5'-nitro-4-phenylbutyrophenon in Form gelber Kristalle vom Smp. 118-119°.

### Beispiel 74

a) Zu einer Lösung von 10,0 g (47,4 mMol) 3,4-Dimethoxy-5-nitrobenzaldehyd in 100 ml Dioxan wird eine Lösung von 14,68 g (225,4 mMol) Kaliumcyanid in 20 ml Wasser zugegeben. Nun tropft man innert 30 Minuten 18,81 ml (190,76 mMol) 37-proz. Salzsäure unter kräftigem Rühren dazu. Nach Zugabe von 120 ml Aether wird das überschüssige Blausäuregas durch Ueberleiten von Argon ausgetrieben. Das Reaktionsgemisch wird über ein Filtrierhilfsmittel aus Kieselgur filtriert, und die organische Phase wird mit Wasser gewaschen, über Natriumsulfat getrocknet und eingedampft. Das gebildete α-Hydroxy-3,4-dimethoxyphenylacetonitril (gelbliches Oel) wird in 200 ml Aether gelöst, worauf man mit 20 ml Aethanol versetzt, auf 0° abkühlt und während 30 Minuten Salzsäuregas einleitet. Nach 3 Stunden wird der ausgefallene farblose Niederschlag abgesaugt und aus Aethanol/Aether umgelöst. Man erhält Aethyl (3,4-dimethoxy-5-nitrophenyl)hydroxyacetimidat-Hydrochlorid.
b) 19,7 g (61,46 mMol) Aethyl (3,4-dimethoxy-5-nitrophenyl)hydroxyacetimidat-Hydrochlorid werden in 500 ml Aethanol gelöst, worauf man nach Zugabe von 6,73 g (61,46 mMol) o-Phenylendiamin während 2 Stunden bei Raumtemperatur rührt und anschliessend über Nacht bei der Rückflusstemperatur hält. Nach Abdestillieren des Lösungsmittels wird mit 50 ml Wasser versetzt, mit Sodalösung alkalisch gestellt und zweimal mit je 250 ml Methylenchlorid extrahiert. Die organische Phase wird mit Wasser gewaschen, über Natriumsulfat getrocknet und eingedampft. Der erhaltene orange Rückstand wird an 400 g Kieselgel mit Methylenchlorid/Essigester (1:1) chromatographiert. Aus Aether/Hexan erhält man α-(3,4-Dimethoxy-5-nitrophenyl)-2-benzimidazolmethanol in Form gelblicher Kristalle vom Smp. 50° (Zers.).
c) 13,2 g (40,1 mMol) α-(3,4-Dimethoxy-5-nitrophenyl)-2-benzimidazolmethanol werden in 200 ml Methylenchlorid gelöst, worauf man nach Versetzen mit 130 g Mangandioxyd während 2 Stunden bei der Rückflusstemperatur rührt. Nach Filtration wird das Lösungsmittel abdestilliert. Man erhält 2-Benzimidazolyl (3,4-dimethoxy-5-nitrophenyl)keton in Form gelblicher Kristalle vom Smp. 212-213°.
d) 1,0 g (3.05 mMol) 2-Benzimidazolyl (3,4-dimethoxy-5-nitrophenyl)keton und 8,0 g Pyridin-Hydrochlorid werden während 60 Minuten bei 200° gehalten. Die dunkle Lösung wird noch warm auf Eiswasser gegossen und dreimal mit je 100 ml Essigester extrahiert. Die organische Phase wird mit Wasser gewaschen über Natriumsulfat getrocknet und eingedampft. Nach Umkristallisieren aus Essigester/Hexan erhält man 2-Benzimidazolyl (3,4-dihydroxy-5-nitrophenyl)keton in Form gelber Kristalle vom Smp. 249-250°.

### Beispiel 75

a) 30,0 g (132 mMol) 3,4-Dimethoxy-5-nitrobenzoesäure werden in 250 ml Tetrahydrofuran gelöst, worauf man nach Zugabe von 21,85 g (135 mMol) 1,1'-Carbonyldiimidazol während 2 Stunden bei der Rückflusstemperatur rührt. Das Ganze wird auf 300 ml Eiswasser gegossen, und die ausgefallenen Kristalle werden nach 30-minütigem Rühren abgesaugt. Letztere werden in Methylenchlorid aufgenommen, worauf die organische Phase mit Wasser gewaschen, über Natriumsulfat getrocknet und eingedampft wird. Nach Kristallisieren aus Methylenchlorid/Hexan erhält man 1-(3,4-Dimethoxy-5-nitrobenzoyl)imidazol in Form farbloser Kristalle vom Smp. 136-137°.
b) 10,0 g (36,1 mMol) 1-(3,4-Dimethoxy-5-nitrobenzoyl)imidazol in 50 ml Dimethylformamid werden mit 6,95 g (93,8 mMol) Acetamidoxim versetzt, worauf man während 1 Stunde bei 70° rührt. Das Ganze wird nach dem Abkühlen auf 500 ml Eiswasser gegossen und während 30 Minuten gerührt. Die ausgefallene Kristalle werden abgesaugt und mit Wasser gewaschen. Nach Kristallisieren aus Essigester erhält man N'-[(3,4-Dimethoxy-5-nitrobenzoyl)oxy]acetamidin in Form farbloser Kristalle vom Smp. 165-166°.
c) 2,0 g (7,2 mMol) N'-[(3,4-Dimethoxy-5-nitrobenzoyl)oxy]acetamidin werden in 20 ml Eisessig während 1 Stunde bei der Rückflusstemperatur gehalten. Nach Abdestillieren des Eisessigs wird der kristalline Rückstand aus Aether/Hexan umgelöst. Man erhält 5-(3,4-Dimethoxy-5-nitrophenyl)-3-methyl-1,2,4-oxadiazol in Form farbloser Kristalle vom Smp. 111°.
d) 2,5 g (9,43 mMol) 5-(3,4-Dimethoxy-5-nitrophenyl)-5-methyl-1,2,4-oxadiazol werden in 70 ml Methylenchlorid gelöst. Nach Abkühlen auf -60° wird innert 20 Minuten unter Rühren eine Lösung von 23,62 g (94,3 mMol) Bortribromid in 50 ml Methylenchlorid dazugetropft, worauf man während 48 Stunden bei der Rückflusstemeratur hält. Nach Abkühlen auf -60° wird mit 60 ml Aethanol versetzt und anschliessend während 30 Minuten bei Raumtemperatur gerührt. Die gelbe Lösung wird zur Trockene eingedampft, worauf der Rückstand dreimal mit je 100 ml Toluol/Aethanol (1:1) versetzt und das Lösungsmittel jeweils abdestilliert wird. Nach Kristallisieren aus Aethanol erhält man 5-(3-Methyl-1,2,4-oxadiazol-5-yl)-3-nitropyrocatechol in Form gelber Kristalle vom Smp. 201-202°.

### Beispiel 76

a) Zu 35.0 g 1-Brom-2-fluorbenzol (gelöst in 600 ml Tetrahydrofuran) werden bei -70° innert 30 Minuten 143,8 ml n-Butyllithiumlösung (1,53M in Hexan) zugetropft. Nach 60 Minuten Rühren bei -70° werden 48,5 g 3-Methoxy-4-benzyloxybenzaldehyd (gelöst in 450 ml Tetrahydrofuran) während 30 Minuten zugetropft. Das Reaktionsgemisch wird 2 Stunden bei -70° und 30 Minuten bei 0° gerührt, auf ein Gemisch von Eis und 150 ml 2N-Schwefelsäure gegossen und dreimal mit 500 ml Aether extrahiert. Die vereinigten Aetherphasen werden mit gesättigter Kochsalzlösung gewaschen, über Natriumsulfat getrocknet und eingedampft. Man erhält 4-(Benzyloxy)-2'-fluor-3-methoxybenzhydrol, als gelbliches Oel, das direkt in die anschliessende Reaktionsstufe eingesetzt werden kann.
   In analoger Weise erhält man:
   a1) Aus 3-Methoxy-4-benzyloxybenzaldehyd und 1-Brom-3-fluorbenzol das 4-(Benzyloxy)-3'-fluor-3-methoxybenzhydrol als Oel:
   a2) aus 3-Methoxy-4-benzyloxybenzaldehyd und 1-Brom-4-fluorbenzol das 4-(Benzyloxy)-4'-fluor-3-methoxybenzhydrol als Oel;
   a3) auf 3-Methoxy-4-benzyloxybenzaldehyd und 1-Brom-2,6-difluorbenzol das 4-(Benzyloxy)-2',6'-difluor-3-methoxybenzhydrol als Oel;
   a4) aus 3-Methoxy-4-benzyloxybenzaldehyd und 1-Brom-2-chlorbenzol das 4-(Benzyloxy)-2'-chlor-3-methoxybenzhydrol als Oel:
   a5) aus 3-Methoxy-4-benzyloxybenzaldehyd und 1-Brom-3-chlorbenzol das 4-(Benzyloxy)-3'-chlor-3-methoxybenzhydrol als Oel;
   a6) aus 3-Methoxy-4-benzyloxybenzaldehyd und 1-Brom-4-chlorbenzol das 4-(Benzyloxy)-4'-chlor-3-methoxybenzhydrol als Oel;
   a7) aus 4-Benzyloxy-3-methoxybenzaldehyd und 2-Bromtoluol das 4-(Benzyloxy)-3-methoxy-2'-methylbenzhydrol als Oel;
   a8) aus 3-Methoxy-4-benzyloxybenzaldehyd und 4-Bromtoluol das 4-(Benzyloxy)-3-methoxy-4'-methylbenzhydrol als Oel;
   a9) aus 3-Methoxy-4-benzyloxybenzaldehyd und 1-Brombenzonitril das 4-(Benzyloxy)-2'-cyano-3-methoxybenzhydrol als Oel und
   a10) aus 3-Methoxy-4-benzyloxybenzaldehyd und 1-Brom-2-trifluormethylbenzol das 4-(Benzyloxy)-3-methoxy-2'-(trifluormethyl)benzhydrol als Oel.
b) 69,8 g 4-(Benzyloxy)-2'-fluor-3-methoxybenzhydrol (gelöst in 600 ml Methylenchlorid) werden innert 30 Minuten bei 20° mit 45,3 g Pyridiniumchlorochromat versetzt und 3 Stunden bei 20° gerührt. Anschliessend wird der gebildete Niederschlag abfiltriert und mit Methylenchlorid gewaschen. Das Filtrat wird eingedampf, und der Rückstand wird an 100 g Kieselgel mit Aether filtriert. Man erhält nach Umkristallisieren aus Aether 4-(Benzyloxy)-2'-fluor-3-methoxybenzophenon vom Smp. 118-120°.
   In analoger Weise erhält man:
   b1) Aus 4-(Benzyloxy)-3'-fluor-3-methoxybenzhydrol das 4-(Benzyloxy)-3'-fluor-3-methoxybenzophenon als amorphen Festkörper;
   b2) aus 4-(Benzyloxy)-4'-fluor-3-methoxybenzhydrol das 4-(Benzyloxy)-4'-fluor-3-methoxybenzophenon vom Smp. 99-101° (aus Aether/Hexan);
   b3) aus 4-(Benzyloxy)-2',6'-difluor-3-methoxybenzhydrol das 4-(Benzyloxy)-2',6'-difluor-3-methoxybenzophenon vom Smp. 139-141° (aus Methylenchlorid/Aether):
   b4) aus 4-(Benzyloxy)-2'-chlor-3-methoxybenzhydrol das 4-(Benzyloxy)-2'-chlor-3-methoxybenzophenon vom Smp. 128-130° (aus Aether);
   b5) aus 4-(Benzyloxy)-3'-chlor-3-methoxybenzhydrol das 4-(Benzyloxy)-3'-chlor-3-methoxybenzophenon als amorphen Festkörper;
   b6) aus 4-(Benzyloxy)-4'-chlor-3-methoxybenzhydrol das 4-(Benzyloxy)-4'-chlor-3-methoxybenzophenon vom Smp. 106-108° (aus Methylenchlorid/Hexan);
   b7) aus 4-(Benzyloxy)-3-methoxy-2'-methylbenzhydrol das 4-(Benzyloxy)-3-methoxy-2'-methylbenzophenon vom Smp. 86-88° (aus Isopropyläther);
   b8) aus 4-(Benzyloxy)-3-methoxy-4'-methylbenzhydrol das 4-(Benzyloxy)-3-methoxy-4'-methylbenzophenon vom Smp. 79-81° (aus Aether(Hexan);
   b9) aus 4-(Benzyloxy)-2'-cyano-3-methoxybenzhydrol das 4-(Benzyloxy)-2'-cyano-3-methoxybenzophenon als amorphen Festkörper und
   b10) aus 4-(Benzyloxy)-3-methoxy-2'-(trifluormethyl)benzhydrol das 4-(Benzyloxy)-3-methoxy-2'-(trifluormethyl)benzophenon vom Smp. 103-105° (aus Aether).
c) Zu 42,4 g 4-(Benzyloxy)-2'-fluor-3-methoxybenzophenon (gelöst in 450 ml Methylenchlorid) werden bei 20-25° 170 ml 33-proz. Bromwasserstoffsäure in Eisessig innert 20 Minuten zugegeben. Nach 1,5 Stunden Rühren bei 20° wird das Reaktionsgemisch auf 750 ml Eiswasser gegossen; die Methylenchloridphase wird abgetrennt, und die wässrige Phase wird noch zweimal mit 200 ml Methylenchlorid extrahiert. Die vereinigten Methylenchloridphasen werden mit 1200 ml Wasser gewaschen, über Natriumsulfat getrocknet und eingedampft. Um das entstandene Benzylbromid zu entfernen, wird der ölige Rückstand mit Hexan versetzt und abdekantiert. Man erhält 2'-Fluor-4-hydroxy-3-methoxybenzophenon, als gelbliches Oel, das direkt in die anschliessende Reaktionsstufe eingesetzt werden kann.
   In analoger Weise erhält man:
   c1) Aus 4-(Benzyloxy)-3'-fluor-3-methoxybenzophenon das 3'-Fluor-4-hydroxy-3-methoxybenzophenon vom Smp. 133-135° (aus Methylenchlorid/Petroläther ts.);
   c2) aus 4-(Benzyloxy)-4'-fluor-3-methoxybenzophenon das 4'-Fluor-4-hydroxy-3-methoxybenzophenon vom Smp. 139-141° (aus Aether);
   c3) aus 4-(Benzyloxy)-2',6'-difluor-3-methoxybenzophenon das 2',6'-Difluor-4-hydroxy-3-methoxybenzophenon vom Smp. 130-132° (aus Methylenchlorid/Petroläther ts.);
   c4) aus 4-(Benzyloxy)-2'-chlor-3-methoxybenzophenon das 2'-Chlor-4-hydroxy-3-methoxybenzophenon als amorphen Festkörper;
   c5) aus 4-(Benzyloxy)-3'-chlor-3-methoxybenzophenon das 3'-Chlor-4-hydroxy-3-methoxybenzophenon vom Smp. 136-138° (aus Methylenchlorid);
   c6) aus 4-(Benzyloxy)-4'-chlor-3-methoxybenzophenon das 4'-Chlor-4-hydroxy-3-methoxybenzophenon vom Smp. 114-116° (aus Methylenchlorid/Petroläther ts.);
   c7) aus 4-(Benzyloxy)-3-methoxy-2'-methylbenzophenon das 4-Hydroxy-3-methoxy-2'-methylbenzophenon vom Smp. 103-105° (aus Isopropyläther);
   c8) aus 4-(Benzyloxy)-3-methoxy-4'-methylbenzophenon das 4-Hydroxy-3-methoxy-4'-methylbenzophenon vom Smp. 103-105° (aus Aether/Petroläther ts);
   c9) aus 4-(Benzyloxy)-2'-cyano-3-methoxybenzophenon das 2'-Cyano-4-hydroxy-3-methoxybenzophenon von Smp. 124-126° (aus Aether/n-Hexan) und
   c10) aus 4-(Benzyloxy)-3-methoxy-2'-(trifluormethyl)benzophenon das 4-Hydroxy-3-methoxy-2'-(trifluormethyl)benzophenon vom Smp. 115-117° (aus Aether).
d) Zu 29,4 g 2'-Fluor-4-hydroxy-3-methoxybenzophenon (gelöst in 450 ml Essigsäure) werden innert 20 Minuten bei 20° 7,8 ml 65-proz. Salpetersäure zugetropft. Nach 1,5 Stunden Rühren wird das Reaktionsgemisch auf 2 l Eiswasser gegossen, und der gebildete Niederschlag wird abfiltriert, mit Wasser gewaschen und in Methylenchlorid gelöst. Die Methylenchloridlösung wird mit Wasser gewaschen, über Natriumsulfat getrocknet und eingedampft. Der Rückstand wird aus Methanol umkristallisiert. Man erhält 2'-Fluor-4-hydroxy-3-methoxy-5-nitrobenzophenon vom Smp. 127-129°.
   In analoger Weise erhält man:
   d1) Aus 3'-Fluor-4-hydroxy-3-methoxybenzophenon das 3'-Fluor-4-hydroxy-3-methoxy-5-nitrobenzophenon vom Smp. 168-170° (aus Methanol);
   d2) aus 4'-Fluor-4-hydroxy-3-methoxybenzophenon das 4'-Fluor-4-hydroxy-3-methoxy-5-nitrobenzophenon vom Smp. 126-128° (aus Aether);
   d3) aus 2',6'-Difluor-4-hydroxy-3-methoxybenzophenon das 2',6'-Difluor-4-hydroxy-3-methoxy-5-nitrobenzophenon vom Smp. 147-149° (aus Methanol):
   d4) aus 2'-Chlor-4-hydroxy-3-methoxybenzophenon das 2'-Chlor-4-hydroxy-3-methoxy-5-nitrobenzophenon vom Smp. 123-125° (aus Aether);
   d5) aus 3'-Chlor-4-hydroxy-3-methoxybenzophenon das 3'-Chlor-4-hydroxy-3-methoxy-5-nitrobenzophenon vom Smp. 152-154° (aus Methanol);
   d6) aus 4'-Chlor-4-hydroxy-3-methoxybenzophenon das 4'-Chlor-4-hydroxy-3-methoxy-5-nitrobenzophenon vom Smp. 129-131° (aus Methylenchlorid/Petroläther ts);
   d7) aus 4-Hydroxy-3-methoxy-2'-methylbenzophenon das 4-Hydroxy-3-methoxy-2'-methyl-5-nitrobenzophenon vom Smp. 125-127° (aus Aethanol);
   d8) aus 4-Hydroxy-3-methoxy-4'-methylbenzophenon das 4-Hydroxy-3-methoxy-4'-methyl-5-nitrobenzophenon vom Smp. 137-139° (aus Methylenchlorid/Aether);
   d9) aus 2'-Cyano-4-hydroxy-3-methoxybenzophenon das 2'-Cyano-4-hydroxy-3-methoxy-5-nitrobenzophenon vom Smp. 163-164° (aus Methanol);
   d10) aus 4-Hydroxy-3-methoxy-2'-(trifluormethyl)benzophenon das 4-Hydroxy-3-methoxy-5-nitro-2'-(trifluormethyl)benzophenon vom Smp. 138-140° (aus Methylenchlorid/Petroläther ts);
   d11) aus 4-Hydroxy-3,4'-dimethoxybenzophenon das 4-Hydroxy-3,4'-dimethoxy-5-nitrobenzophenon vom Smp. 134-136° (aus Methanol) und
   d12) aus 4-Hydroxy-3,3',4'-trimethoxybenzophenon das 4-Hydroxy-5-nitro-3,3',4'-trimethoxybenzophenon vom Smp. 178-180° (aus Methanol).
e) 24,8 g 2'-Fluor-4-hydroxy-3-methoxy-5-nitrobenzophenon (gelöst in 120 ml Eisessig, 100 ml 33-proz. Bromwasserstoffsaure in Eisessig und 68 ml 48-proz. wässriger Bromwasserstoffsäure) werden 4 Stunden unter Rückfluss gekocht. Anschliessend wird das Reaktionsgemisch unter vermindertem Druck eingedampft, und mit Toluol abdestilliert. Der Rückstand wird in Methylenchlorid gelöst, mit Wasser gewaschen, über Natriumsulfat getrocknet, filtriert und eingedampft. Das Produkt wird aus Methylenchlorid/Petroläther ts. kristallisiert. Man erhält 2'-Fluor-3,4-dihydroxy-5-nitrobenzophenon vom Smp. 169-171°.
   In analoger Weise erhält man:
   e1) Aus 3'-Fluor-4-hydroxy-3-methoxy-5-nitrobenzophenon das 3'-Fluor-3,4-dihydroxy-5-nitrobenzophenon vom Smp. 124-126° (aus Methylenchlorid);
   e2) aus 4'-Fluor-4-hydroxy-3-methoxy-5-nitrobenzophenon das 4'-Fluor-3,4-dihydroxy-5-nitrobenzophenon vom Smp. 171-173° (aus Methylenchlorid);
   e3) aus 2',6'-Difluor-4-hydroxy-3-methoxy-5-nitrobenzophenon das 2',6'-Difluor-3,4-dihydroxy-5-nitrobenzophenon vom Smp. 194-196° (aus Methanol);
   e4) aus 2'-Chlor-4-hydroxy-3-methoxy-5-nitrobenzophenon das 2'-Chlor-3,4-dihydroxy-5-nitrobenzophenon vom Smp. 129-131° (aus Methylenchlorid/Petroläther ts);
   e5) aus 3'-Chlor-4-hydroxy-3-methoxy-5-nitrobenzophenon das 3'-Chlor-3,4-dihydroxy-5-nitrobenzophenon vom Smp. 143-145° (aus Methylenchlorid/Petroläther ts);
   e6) aus 4'-Chlor-4-hydroxy-3-methoxybenzophenon das 4'-Chlor-3,4-dihydroxybenzophenon vom Smp. 174-176° (aus Methylenchlorid);
   e7) aus 4-Hydroxy-3-methoxy-2'-methyl-5-nitrobenzophenon das 3,4-Dihydroxy-2'-methyl-5-nitrobenzophenon vom Smp. 164-166° (aus Methylenchlorid);
   e8) aus 4-Hydroxy-3-methoxy-4'-methyl-5-nitrobenzophenon das 3,4-Dihydroxy-4'-methyl-5-nitrobenzophenon vom Smp. 146-148° (aus Methylenchlorid);
   e9) aus 2'-Cyano-4-hydroxy-3-methoxy-5-nitrobenzophenon das 2'-Cyano-3,4-dihydroxy-5-nitrobenzopheonon vom Smp. 159-161° (aus Methanol);
   e10) aus 4-Hydroxy-3-methoxy-5-nitro-2'-(trifluormethyl)benzophenon das 3,4-Dihydroxy-5-nitro-2'-(trifluormethyl)benzophenon vom Smp. 146-148° (aus Methanol);
   e11) aus 4-Hydroxy-3,4'-dimethoxy-5-nitrobenzophenon das 5-Nitro-3,4,4'-trihydroxybenzophenon vom Smp. 212-214° (aus Methanol/Methylenchlorid) und
   e12) aus 4-Hydroxy-5-nitro-3,3',4'-trimethoxybenzophenon das 5-Nitro-3,3',4,4'-tetrahydroxybenzophenon vom Smp. 222-224° (aus Aether).

### Beispiel 77

Man versetzt eine Suspension von 13,8 g 2-Brom-3',4'-dihydroxy-5'-nitroacetophenon mit 9,0 g 1-(Phenäthyl)-2-thioharnstoff in 150 ml n-Butanol und erhitzt 3 Stunden unter Rückfluss zum Sieden. Nach dem Abkühlen auf Raumtemperatur nutscht man die Kristalle ab und kristallisiert sie aus n-Butanol um. Man erhält 3-Nitro-5-[2-(phenäthylamino)-4-thiazolyl]pyrocatechol-hydrobromid vom Smp. 249-251°.

### Beispiel 78

In Analogie zu Beispiel 38 erhält man aus 2-Brom-3',4'-dihydroxy-5'-nitroacetophenon und 2-Aminobenzophenon das 2-(3,4-Dihydroxy-5-nitrobenzoyl)-3-phenylindol vom Smp. 196-198° (aus Isopropanol).

### Beispiel 79

Man versetzt eine Suspension von 8.3 g 2-Brom-3',4'-dihydroxy-5'-nitroacetophenon mit 1-(1-Adamantyl)-2-thioharnstoff in 90 ml n-Butanol und erhitzt während 4 Stunden unter Rückfluss zum Sieden. Nach dem Abkühlen auf Raumtemperatur nutscht man die Kristalle ab und kristallisiert sie aus n-Butanol um. Man erhält 5-[2-(1-Adamantylamino)-5-thiazolyl]-3-nitropyrocatechol-hydrobromid vom Smp. 245-247°.

### Beispiel 80

Eine Suspension von 2,6 g (3,4-Dihydroxy-5-nitrobenzoyl)methylacetat in 20 ml Aethanol und 20 ml 1N-Salzsäure wird während 5 Stunden unter Rückfluss zum Sieden erhitzt. Dann dampft man das Reaktionsgemisch ein, destilliert noch mit Toluol ab und kristallisiert den Rückstand aus Aethanol um. Man erhält 2,3',4'-Trihydroxy-5'-nitroacetophenon vom Smp. 208-210°.

### Beispiel 81

Eine Lösung von 4,0 g 3,4-Dihydroxy-5-nitrophenylglyoxylsäure-n-hexylester und 1,5 g Diaminomaleonitril in 35 ml Aethanol wird 24 Stunden unter Rückfluss zum Sieden erhitzt. Dann wird der Alkohol abdestilliert, der Rückstand in Aether gelöst, mit Wasser gewaschen, über Natriumsulfat getrocknet, filtriert und eingedampft. Man erhält 6-Hydroxy-5-(3,4-dihydroxy-5-nitrophenyl)-2,3-pyrazindicarbonitril vom Smp. >300° (aus Aether/Methylenchlorid).

### Beispiel 82

a) Zu 4,2 g 5-(Bromacetyl)-2,3-dimethoxybenzonitril, gelöst in 150 ml Methylenchlorid, werden mit 8,9 ml Bortribromid versetzt. Das Reaktionsgemisch wird während 18 Stunden bei 20° gerührt. Anschliessend wird es auf 220 ml gesättigter Natriumhydrogencarbonatlösung und 100 g Eis gegossen, mit Eisessig auf pH 6 gestellt und mit Essigester extrahiert. Die organische Phase wird mit Wasser gewaschen, über Natriumsulfat getrocknet und eingedampft. Man erhält 5-(Bromacetyl)-2,3-dihydroxybenzonitril als amorphen Festkörper.
b) 3,8 g 5-(Bromacetyl)-2,3-dihydroxybenzonitril, gelöst in 25 ml N,N-Dimethylformamid, werden mit N-Phenylthioharnstoff versetzt und 5 Stunden bei 100° gerührt. Nachher wird das Lösungsmittel abgedampft. Der Rückstand wird mit 200 ml 1N-Natriumcarbonatlösung versetzt und dreimal mit je 100 ml Methylenchlorid extrahiert. Die vereinigten organischen Phasen werden mit Wasser gewaschen, über Natriumsulfat getrocknet und eingedampft. Der Rückstand wird an 30 g Kieselgel mit Essigester chromatographiert. Das so erhaltene Rohprodukt wird mit 40 ml 1N-Salzsäure versetzt, eingedampft und aus Aceton kristallisiert. Man erhält 5-(2-Anilino-4-thiazolyl)-2,3-dihydroxybenzonitril-Hydrochlorid vom Smp. 245-247°.

### Beispiel 83

a) Zu 10 g 4-(Benzyloxy)-3-methoxy-brombenzol, gelöst in 100 ml Tetrahydrofuran, werden bei -70° innert 10 Minuten 25 ml tert.-Butyllithiumlösung (1,4M in Hexan) zugetropft. Nach 1 Stunde Rühren bei -70° werden 5 g Chinolin-4-carbaldehyd, gelöst in 50 ml Tetrahydrofuran, innert 30 Minuten zugetropft. Das Reaktionsgemisch wird 1 Stunde bei -40° und 1 Stunde bei -5° gerührt, auf 200 ml Wasser gegossen und mit Eisessig auf pH 4 gestellt. Es wird dreimal mit je 50 ml Aether extrahiert. Die vereinigten Aetherphasen werden mit Wasser gewaschen, über Natriumsulfat getrocknet und eingedampft. Man erhält α-[4-(Benzyloxy)-3-methoxyphenyl]-4-chinolinmethanol als amorphen Festkörper.
b) 9,4 g α-[4-(Benzyloxy)-3-methoxyphenyl]-4-chinolinmethanol, gelost in 200 ml Methylenchlorid, werden mit 6.5 g Pyridiniumchlorochromat versetzt, worauf man während 3 Stunden bei Raumtemperatur rührt. Anschliessend werden die unlöslichen Bestandteile abfiltriert. Das Filtrat wird eingedampft, und der Rückstand wird an 150 g Kieselgel mit Essigester chromatographiert. Dabei erhält man 4-(Benzyloxy)-3-methoxyphenyl-4-chinolylketon als amorphen Festkörper.
c) Zu 7,5 g 4-(Benzylox)-3-methoxyphenyl-4-chinolylketon, gelöst in 150 ml Methylenchlorid, werden bei Raumtemperatur 15 ml 33-proz. Bromwasserstoffsäure in Eisessig innert 5 Minuten zugetropft. Nach 4,5 Stunden Rühren bei 20° wird das Reaktionsgemisch portionenweise auf 250 ml gesättigter Natriumbicarbonatlösung gegossen. Die Methylenchloridphase wird abgetrennt; die wässrige Phase wird noch zweimal mit je 100 ml Methylenchlorid extrahiert. Die vereinigten Methylenchloridphasen werden über Natriumsulfat getrocknet und eingedampft. Der Rückstand wird aus Methylenchlorid/Hexan umkristallisiert. Man erhält 4-Hydroxy-3-methoxyphenyl-4-chinolylketon vom Smp. 190-192°.
d) Zu 1,3 g 4-Hydroxy-3-methoxyphenyl-4-chinolylketon, gelöst in 25 ml Eisessig, werden 0,37 ml 65-proz. Salpetersäure bei Raumtemperatur zugetropft. Nach 3 Stunden Rühren wird das Reaktionsgemisch auf Eiswasser gegossen, worauf man mit konz. Ammoniak auf pH 6 stellt und den gebildeten Niederschlag abfiltriert. Der so erhaltene Rückstand wird in 20 ml Acetonitril unter Rückfluss erwärmt, worauf man bei 0° die Kristalle abfiltriert. Man erhält 4-Hydroxy-3-methoxy-5-nitrophenyl-4-chinolylketon vom Smp. 246-248°.
e) 1 g 4-Hydroxy-3-methoxy-5-nitrophenyl-4-chinolylketon, gelöst in 30 ml 48-proz. wässriger Bromwasserstoffsäure, wird während 18 Stunden bei 100° gerührt. Nach Abkühlen auf Raumtemperatur wird das Reaktionsgut mit 30 ml Wasser verdünnt, und der Niederschlag wird abgenutscht. Man erhält 3,4-Dihydroxy-5-nitrophenyl-4-chinolylketon-Hydrobromid vom Smp. 273-275° (aus Acetonitril).

### Beispiel 84

a) Zu 4,03 g Thiophen, gelöst in 40 ml Tetrahydrofuran, werden bei -50° innert 10 Minuten 18,8 ml n-Butyllithiumlösung (1,6M in Hexan) zugetropft. Nach 30 Minuten Rühren bei -50° werden 6,3 g 3,4-Dimethoxy-5-nitrobenzaldehyd, in 100 ml Tetrahydrofuran gelöst, innert 30 Minuten zugetropft. Das Reaktionsgemisch wird 1 Stunde bei -50° und 30 Minuten bei 0° gerührt und auf 100 ml 2N-Schwefelsäure gegossen. Es wird dreimal mit je 100 ml Aether extrahiert; die vereinigten Aetherphasen werden mit Kochsalzlösung gewaschen, über Natriumsulfat getrocknet, filtriert und eingedampft. Man erhält α-(3,4-Dimethoxy-5-nitrophenyl)-2-thiophenmethanol vom Smp. 79-81° (aus Methylenchlorid/Hexan).
b) 9,9 g α-(3,4-Dimethoxy-5-nitrophenyl)-2-thiophenmethanol, gelöst in 300 ml Aceton, werden mit 90 g Braunstein versetzt und während 4 Stunden unter Rückfluss erhitzt. Nachher wird vom Braunstein abgenutscht, und das Filtrat wird eingedampft. Man erhält 3,4-Dimethoxy-5-nitrophenyl-2-thienylketon vom Smp. 102-104° (aus Methylenchlorid/Hexan).
c) 2 g 3,4-Dimethoxy-5-nitrophenyl-2-thienylketon werden in einem Gemisch von 20 ml 30-33-proz. Bromwasserstoffsäure in Eisessig und 20 ml 48-proz. wässriger Bromwasserstoffsäure während 8 Stunden bei 100° gerührt. Anschliessend wird das Reaktionsgemisch zur Trockene eingedampft. Der Rückstand wird in Essigester aufgenommen, mit Wasser gewaschen, über Natriumsulfat getrocknet und filtriert, und das Filtrat wird eingedampft. Nach Umkristallisieren aus Essigester/Hexan erhält man 3,4-Dihydroxy-5-nitrophenyl-2-thienylketon vom Smp. 155-157°.

### Beispiel A

Die Gelatine-Steckkapseln nachfolgender Zusammensetzung können in an sich bekannter Weise hergestellt werden:

| Bestandteile | Menge in mg/Kapsel |
|---|---|
| L-Dopa | 100 |
| Benserazid-Hydrochlorid | 29,3 |
| 3,4-Dihydroxy-5-nitrophenyl-4-pyridylketon | 25 |
| Gelatine | 1 |
| Mg-stearat | 1 |
| Avicel | 93.7 |
| Mannit | 100 |
| Kapselfüllgewicht | 3̅5̅0̅ mg |

## Patentansprüche (Patentansprüche für folgende(n) Vertragsstaat(en): BE, CH, DE, FR, GB, IT, LI, LU, NL, SE)

1. Verbindungen der allgemeinen Formel 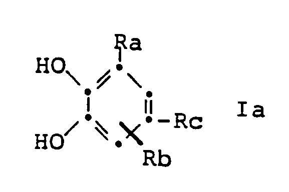 worin Ra Nitro oder Cyano, Rb Wasserstoff oder Halogen, Rc Halogen, Nitro, Cyano oder die Gruppe -(A)ₙ-(Q)ₘ-R¹ oder -(A)ₙ-Q-R², A gegebenenfalls durch niederes Alkyl substituiertes Vinylen, n die Zahl 0 oder 1, m die Zahl 0 oder 1, R¹ die Gruppe -COR³, Aryl oder Heteroaryl, R² Wasserstoff oder einen unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten, gesättigten oder partiell ungesättigten, niederen Kohlenwasserstoffrest, R³ Hydroxy, Amino, einen über ein Sauerstoffatom oder eine Imino- oder niedere Alkyliminogruppe gebundenen, unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten, gesättigten oder partiell ungesättigten, niederen Kohlenwasserstoffrest oder eine unsubstituierte oder durch niederes Alkyl, Hydroxy, niederes Alkoxy, niederes Alkanoyloxy, niederes Hydroxyalkyl, niederes Alkoxyalkyl, niederes Alkanoyloxyalkyl, niederes Alkoxycarbonyl, niederes Alkanoyl, Carbamoyl, mono- oder di(niederes Alkyl)carbamoyl, Oxo und/oder niederes Alkylendioxy mono- oder disubstituierte 4-Morpholinyl-, 1-Pyrrolidinyl- oder 1-Azetidinylgruppe, Q die Gruppe -CO- oder >C=N-(Z)ₚ-R⁴, Z ein Sauerstoffatom oder eine Iminogruppe, p die Zahl 0 oder 1 und R⁴ Wasserstoff oder einen unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten und gegebenenfalls über eine Carbonylgruppe gebundenen, gesättigten oder partiell ungesättigten, niederen Kohlenwasserstoffrest bedeuten, wobei Aryl eine unsubstituierte oder durch Halogen, Trifluormethyl, Nitro, Amino, mono- oder di(niederes Alkyl)amino, niederes Alkyl, niederes Alkoxy, niederes Alkylthio, niederes Alkanoyl, niederes Alkoxycarbonyl, Carboxy, Hydroxy, Cyano, niederes Alkanoyloxy, Carbamoyl, mono- oder di(niederes Alkyl)carbamoyl, niederes Alkylendioxy, niederes Alkanoylamino oder niederes Alkoxycarbonylamino mono- oder disubstituierte Phenyl- oder Naphthylgruppe, Heteroaryl eine über ein Kohlenstoffatom gebundene, unsubstituierte oder durch Halogen, Trifluormethyl, Nitro, Carboxy, Amino, Arylamino, niederes Alkyl, niederes Alkoxy, Hydroxy, niederes Alkoxycarbonyl, niederes Alkanoyl, niederes Alkanoyloxy, Oxo, niederes Alkylendioxy, Mercapto, niederes Alkylthio, niederes Alkylamino, di(niederes Alkyl)amino, C₃₋₇-Cycloalkylamino, C₈₋₁₀-Bicycloalkylamino, niederes Alkanoylamino, niederes Alkoxycarbonylamino, Carbamoyl, mono- oder di(niederes Alkyl)carbamoyl, Cyano, Aryl, Aryl-niederes Alkyl, Aryl-niederes Alkylamino, Heteroraryl, Heteroaryl-niederes Alkyl, Heteroarylamino oder C₃₋₇-Cycloalkyl mono-, di- oder trisubstituierte heterocyclische Gruppe ausgewählt aus der Gruppe, bestehend aus Pyridyl, Pyrazinyl, Triazinyl, Thiadiazinyl, Thiazolyl, Oxazolyl, Oxadiazolyl, Pyrazolyl, Tetrazolyl, Imidazolyl, Thienyl, Chinolinyl, Isochinolinyl, Dihydroisochinolinyl, Benzoxazinyl, Chinoxalinyl, Benzopyranyl, Benzimidazolyl, Indolyl, Imidazothiazolyl, Imidazothiadiazolyl, Imidiazopyridyl, Benzothiazinyl, Benzochinoxalinyl und Imidazobenzothiazolyl, und nieder Reste mit bis zu sieben Kohlenstoffatomen bezeichnen, unter physiologischen Bedingungen hydrolysierbare Ester- und Aetherderivate und pharmazeutisch annehmbare Salze davon zur Anwendung als therapeutische Wirkstoffe.

2. Verbindungen der allgemeinen Formel 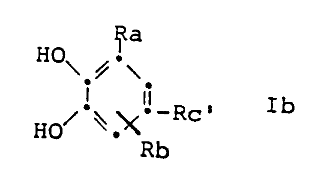 worin Ra Nitro oder Cyano, Rb Wasserstoff oder Halogen, Rc' Nitro, Cyano oder die Gruppe -(A)ₙ-(Q)ₘ-R¹¹ oder -(A)ₙ-(Q)-R²¹, A gegebenenfalls durch niederes Alkyl substituiertes Vinylen, n die Zahl 0 oder 1, m die Zahl 0 oder 1, R¹¹ die Gruppe -COR³¹, Aryl oder Heteroaryl, R²¹ einen unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten, gesättigten oder partiell ungesättigten, niederen Kohlenwasserstoffrest, R³¹ Hydroxy, Amino, einen über ein Sauerstoffatom oder eine Imino- oder niedere Alkyliminogruppe gebundenen, unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten, gesättigten oder partiell ungesättigten, niederen Kohlenwasserstoffrest oder eine unsubstituierte oder durch niederes Alkyl, Hydroxy, niederes Alkoxy, niederes Alkanoyloxy, niederes Hydroxyalkyl, niederes Alkoxyalkyl, niederes Alkanoyloxyalkyl, niederes Alkoxycarbonyl, niederes Alkanoyl, Carbamoyl, mono- oder di(niederes Alkyl)carbamoyl, Oxo und/oder niederes Alkylendioxy mono oder disubstituierte 4-Morpholinyl-, 1-Pyrrolidinyl- oder 1-Azetidinylgruppe, Q die Gruppe -CO- oder >C=N-(Z)ₚ-R⁴, Z ein Sauerstoffatom oder eine Iminogruppe, p die Zahl 0 oder 1 und R⁴ Wasserstoff oder einen unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten und gegebenfalls über eine Carbonylgruppe gebundenen, gesättigten oder partiell ungesättigten, niederen Kohlenwasserstoffrest bedeuten, wobei Ra Cyano bedeutet, wenn Rc' Cyano oder Nitro bedeutet, und R³¹ eine von Hydroxy verschieden Bedeutung hat, wenn m die Zahl 0 bedeutet, und wobei Aryl eine unsubstituierte oder durch Halogen, Trifluormethyl, Nitro, Amino, mono- oder di(niederes Alkyl)amino, niederes Alkyl, niederes Alkoxy, niederes Alkylthio, niederes Alkanoyl, niederes Alkoxycarbonyl, Carboxy, Hydroxy, Cyano, niederes Alkanoyloxy, Carbamoyl, mono- oder di(niederes Alkyl)carbamoyl, niederes Alkylendioxy, niederes Alkanoylamino oder niederes Alkoxycarbonylamino mono- oder disubstituierte Phenyl- oder Naphthylgruppe, Heteroaryl eine über ein Kohlenstoffatom gebundene, unsubstituierte oder durch Halogen, Trifluormethyl, Nitro, Carboxy, Amino, Arylamino, niederes Alkyl, niederes Alkoxy, Hydroxy, niederes Alkoxycarbonyl, niederes Alkanoyl, niederes Alkanoyloxy, Oxo, niederes Alkylendioxy, Mercapto, niederes Alkylthio, niederes Alkylamino, di(niederes Alkyl)amino, C₃₋₇-Cycloalkylamino, C₈₋₁₀-Bicycloalkylamino, niederes Alkanoylamino, niederes Alkoxycarbonylamino, Carbamoyl, mono- oder di(niederes Alkyl)carbamoyl, Cyano, Aryl, Aryl-niederes Alkyl, Aryl-niederes Alkylamino, Heteroaryl, Heteroaryl-niederes Alkyl, Heteroarylamino oder C₃₋₇-Cycloalkyl mono-, di- oder trisubstituierte heterocyclische Gruppe ausgewählt aus der Gruppe, bestehend aus Pyridyl, Pyrazinyl, Triazinyl, Thiadiazinyl, Thiazolyl, Oxazolyl, Oxadiazolyl, Pyrazolyl, Tetrazolyl, Imidazolyl, Thienyl, Chinolinyl, Isochinolinyl, Dihydroisochinolinyl, Benzoxazinyl, Chinoxalinyl, Benzopyranyl, Benzimidazolyl, Indolyl, Imidazothiazolyl, Imidazothiadiazolyl, Imidazopyridyl, Benzothiazinyl, Benzochinoxalinyl und Imidazobenzothiazolyl, und nieder Reste mit bis zu sieben Kohlenstoffatomen bezeichnen, unter physiologischen Bedingungen hydrolysierbare Ester- und Aetherderivate und pharmazeutisch annehmbare Salze davon.

3. Verbindungen nach Anspruch 2, worin sich Rb in der p-Stellung zu Ra befindet.

4. Verbindungen nach Anspruch 2 oder 3, worin Ra Nitro bedeutet.

5. Verbindungen nach einem der Ansprüche 2-4, worin Rb Wasserstoff, Chlor oder Fluor bedeutet.

6. Verbindungen nach Anspruch 5, worin Rb Wasserstoff bedeutet.

7. Verbindungen nach einem der Ansprüche 2-6, worin Rc' die Gruppe -CO-R¹¹ und R¹¹ eine unsubstituierte oder durch Halogen, Trifluormethyl, Nitro, Amino, mono- oder di(niederes Alkyl)amino, niederes Alkyl, niederes Alkoxy, niederes Alkylthio, niederes Alkynoyl, niederes Alkoxy, niederes Alkylthio, niederes Alkanoyl, niederes Alkoxycarbonyl, Carboxy, Hydroxy, Cyano, niederes Alkanoyloxy, Carbamoyl, mono- oder di(niederes Alkyl)carbamoyl, niederes Alkylendioxy, niederes Alkanoylamino oder niederes Alkoxycarbonylamino mono- oder disubstituierte Phenylgruppe oder eine über ein Kohlenstoffatom gebundene, unsubstituierte oder durch Halogen, Trifluormethyl, Nitro, Carboxy, Amino, Arylamino, niederes Alkyl, niederes Alkoxy, Hydroxy, niederes Alkoxycarbonyl, niederes Alkanoyl, niederes Alkanoyloxy, Oxo, niederes Alkylendioxy, Mercapto, niederes Alkylthio, niederes Alkylamino, di(niederes Alkyl)amino, C₃₋₇-Cycloalkylamino, C₈₋₁₀-Bicycloalkylamino, niederes Alkanoylamino, niederes Alkoxycarbonylamino, Carbamoyl, mono- oder di(niederes Alkyl)carbamoyl, Cyano, Aryl, Aryl-niederes Alkyl, Aryl-niederes Alkylamino, Heteroaryl, Heteroaryl-niederes Alkyl, Heteroarylamino oder C₃₋₇-Cycloalkyl mono-, di- oder trisubstituierte heterocyclische Gruppe ausgewählt aus der Gruppe, bestehend aus Pyridyl, Pyrazinyl, Triazinyl, Pyrazolyl, und Imidazolyl, bedeuten.

8. Verbindungen nach Anspruch 7, worin R¹¹ eine gegebenenfalls durch Halogen, Trifluormethyl, Cyano, Hydroxy oder niederes Alkyl mono- oder disubstituierte Phenylgruppe oder eine Pyridylgruppe bedeutet.

9. 3,4-Dihydroxy-4'-methyl-5-nitrobenzophenon.

10. 2'-Fluor-3,4-dihydroxy-5-nitrobenzophenon.

11. 3,4-Dihydroxy-5-nitrophenyl-4-pyridylketon.

12. Verbindungen nach einem der Ansprüche 2-11 zur Anwendung als therapeutische Wirkstoffe.

13. Verbindungen nach einem der Ansprüche 1-11 zur Anwendung als die Catechol-O-Methyl-Transferase hemmende Wirkstoffe.

14. Verfahren zur Herstellung von Verbindungen nach einem der Ansprüche 2-11, von unter physiologischen Bedingungen hydrolysierbaren Ester- und Aetherderivaten und von pharmazeutisch annehmbaren Salzen davon, dadurch gekennzeichnet, dass man
a) in einer Verbindung der allgemeinen Formel 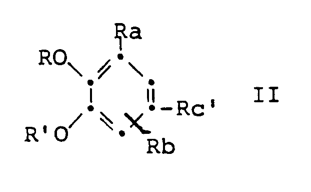 worin eines der Symbole R und R' niederes Alkyl und das andere Wasserstoff oder niederes Alkyl bedeuten, und Ra, Rb und Rc' die in Anspruch 2 angegebene Bedeutung besitzen,
die niedere(n) Alkyläthergruppe(n) spaltet, oder
b) eine Verbindung der allgemeinen Formel 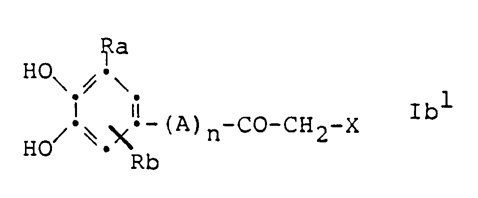 worin X eine Abgangsgruppe bedeutet, und Ra, Rb, A und n die in Anspruch 2 angegebene Bedeutung besitzen,
mit einem Thioamid, Thioharnstoff, Thiocarbonsäurehydrazid, Thiosemicarbazid, Amidin, Guanidin, Amidrazon, Aminoguanidin, cyclischen Amidin, 1,2-Diamin, 1,2-Aminothiol oder einem 1,2-Aminoalkohol umsetzt und das erhaltene Cyclokondensationsprodukt erwünschtenfalls dehydriert, oder
c) eine Verbindung der allgemeinen Formel 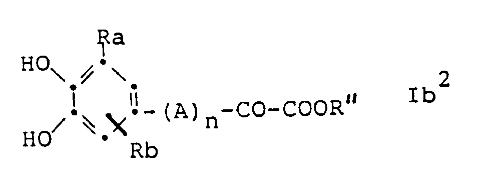 worin R'' niederes Alkyl bedeutet, und Ra, Rb, A und n die in Anspruch 2 angegebene Bedeutung besitzen,
mit einem 1,2-Diamin, 1,2-Aminothiol, 1,2-Aminoalkohol, Semicarbazid, Thiosemicarbazid, Amidrazon oder einem Aminoguanidin umsetzt und das erhaltene Cyclokondensationsprodukt erwünschtenfalls dehydriert, oder
d) eine Verbindung der obigen Formel Ib¹ mit einer β-Aminocarbonylverbindung umsetzt, oder
e) in einer Verbindung der allgemeinen Formel 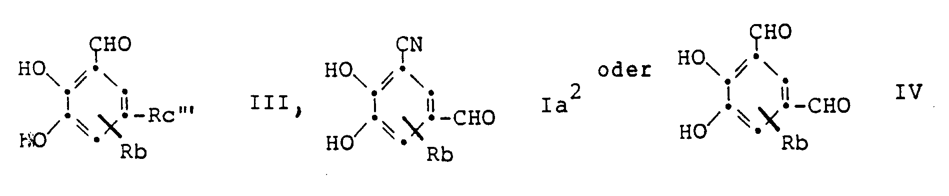 worin Rc''' Nitro, Cyano oder die Gruppe -(A)ₙ-R¹² und R¹² die Gruppe -COR³¹, Aryl oder Heteroaryl bedeuten, und Ra, Rb, A, n und
R³¹ die in Anspruch 2 angegebene Bedeutung besitzen, oder in einem Di-O-niederen Alkanoylderivat davon die Carboxaldehydgruppe(n) in die Cyanogruppe(n) überführt, oder
f) ein Di-O-niederes Alkanoylderivat einer Carbonsäure der allgemeinen Formel 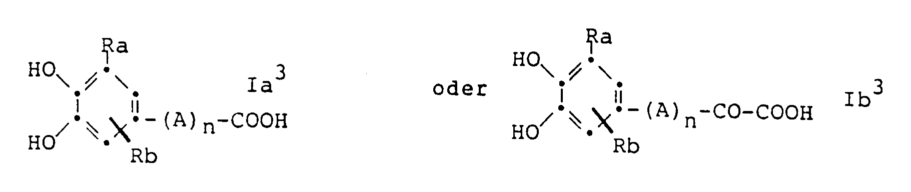 worin Ra, Rb, A und n die in Anspruch 2 angegebene Bedeutung besitzen,
in Gegenwart eines Kondensationsmittels oder ein reaktives Derivat eines Di-O-niederen Alkanoylderivates einer Carbonsäure der Formel Ia³ oder Ib³ mit einer Verbindung der allgemeinen Formel
HO-R⁵ V oder HNR⁶R⁷ VI
worin R⁵einen unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten,gesättigten oder partiell ungesättigten, niederen Kohlenwasserstoffrest, R⁶ Wasserstoff oder niederes Alkyl und R⁷Wasserstoff oder einen unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten gesättigten oder partiell ungesättigten, niederen Kohlenwasserstoffrest oder R⁶ und R₇ zusammen mit dem Stickstoffatom eine unsubstituierte oder durch niederes Alkyl, Hydroxy, niederes Alkoxy, niederes Alkanoyloxy, niederes Hydroxyalkyl, niederes Alkoxyalkyl, niederes Alkynoyloxyalkyl, niederes Alkoxycarbonyl, niederes Alkanoyl, Carbamoyl, mono- oder di(niederes Alkyl)carbamoyl, Oxo und/oder niederes Alkylendioxy mono- oder disubstituierte 4-Morpholinyl-, 1-Pyrrolidinyl- oder 1-Azetidinylgruppe bedeuten, umsetzt, oder
g) eine Verbindung der obigen Formel Ib² oder der allgemeinen Formel 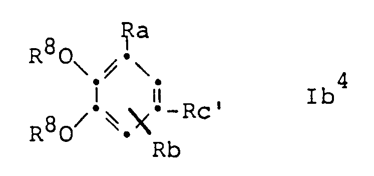 worin R⁸ niederes Alkanoyl beduetet, und Ra, Rb und Rc' die in Anspruch 2 angegebene Bedeutung besitzen, hydrolysiert, oder
h) eine Verbindung der allgemeinen Formel 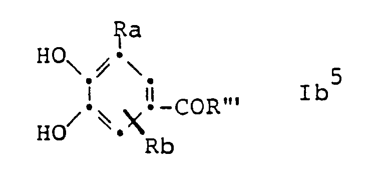 worin Ra und Rb die in Anspruch 2 angegebene Bedeutung besitzen, und R''' Wasserstoff oder niederes Alkyl bedeutet,
oder ein Di-O-niederes Alkanoylderivat davon in Gegenwart eines sekundären Amins mit einer Verbindung der allgemeinen Formel
CH₃CO-R²³ VII
worin R²³ einen
unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten gesättigten oder partiell ungesättigten niederen Kohlenwasserstoffrest bedeutet,
umsetzt, oder
i) eine Verbindung der allgemeinen Formel 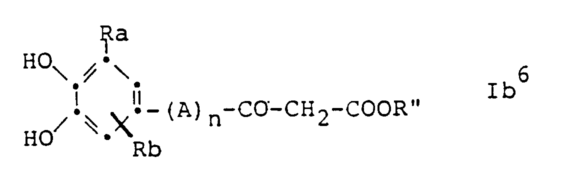 worin Ra, Rb, A und n die in Anspruch 2 angegebene und R'' obige Bedeutung besitzen,
mit einem Hydrazin oder einem Amidin umsetzt, oder
j) eine Verbindung der obigen Formel Ib, worin m die Zahl 1 und Q die Gruppe -CO- bedeuten,
mit einer Verbindung der allgemeinen Formel
H₂N-(Z)ₚ-R⁴ VIII
worin Z, p und R⁴ die in Anspruch 2 angegebene Bedeutung besitzen,
umsetzt, und erwünschtenfalls
k) eine Verbindung der obigen Formel Ib in ein unter physiologischen Bedingungen hydrolysierbares Ester- oder Aetherderivat oder in ein pharmazeutisch annehmbares Salz davon überführt.

15. Arzneimittel, enthaltend eine Verbindung nach einem der Ansprüche 1-11 und ein therapeutisch inertes Trägermaterial.

16. Mittel zur Hemmung der Catechol-O-Methyl-Transferase, enthaltend eine Verbindung nach einem der Ansprüche 1-11 und ein therapeutisch inertes Trägermaterial.

17. Mittel zur Behandlung der Parkinson-Krankheit und von Parkinsonismus, enthaltend L-Dopa, einen peripheren Decarboxylase-Hemmer, eine Verbindung nach einem der Ansprüche 1-11 und ein therapeutisch inertes Trägermaterial,

18. Arzneimittelgarnitur zur Behandlung der Parkinson-Krankheit und von Parkinsonismus, bestehend aus einem Mittel nach Anspruch 16 und einem Mittel, enthaltend L-Dopa, einen peripheren Decarboxylase-Hemmer und ein therapeutisch inertes Trägermaterial.

19. Verwendung von Verbindungen nach einem der Ansprüche 1-11 zur Herstellung von Mitteln, welche die Catechol-O-Methyl-Transferase hemmen.

20. Verwendung von Verbindungen nach einem der Ansprüche 1-11 zur Herstellung von Mitteln zur Behandlung der Parkinson-Krankheit und von Parkinsonismus in Kombination mit L-Dopa und einem peripheren Decarboxylase-Hemmer.

21. Verwendung von Verbindungen nach einem der Ansprüche 1-11, zur Herstellung von Arzneimittelgarnituren nach Anspruch 18.

## Patentansprüche (Patentansprüche für folgende(n) Vertragsstaat(en): AT, ES, GR)

1. Verfahren zur Herstellung von Verbindungen der allgemeinen Formel 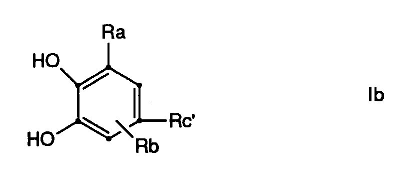 worin Ra Nitro oder Cyano, Rb Wasserstoff oder Halogen, Rc' Nitro, Cyano oder die Gruppe -(A)ₙ-(Q)ₘ-R¹¹ oder -(A)ₙ-Q-R²¹, A gegebenenfalls durch niederes Alkyl substituiertes Vinylen, n die Zahl 0 oder 1, m die Zahl 0 oder 1, R¹¹ die Gruppe -COR³¹, Aryl oder Heteroaryl, R²¹ einen unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten, gesättigten oder partiell ungesättigten, niederen Kohlenwasserstoffrest, R³¹ Hydroxy, Amino, einen über ein Sauerstoffatom oder eine Imino- oder niedere Alkyliminogruppe gebundenen, unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten, gesättigten oder partiell ungesättigten, niederen Kohlenwasserstoffrest oder eine unsubstituierte oder durch niederes Alkyl, Hydroxy, niederes Alkoxy, niederes Alkanoyloxy, niederes Hydroxyalkyl, niederes Alkoxyalkyl, niederes Alkanoyloxyalkyl, niederes Alkoxycarbonyl, niederes Alkanoyl, Carbamoyl, mono- oder di(niederes Alkyl)carbamoyl, Oxo und/oder niederes Alkylendioxy mono- oder disubstituierte 4-Morpholinyl-, 1-Pyrrolidinyl- oder 1-Azetidinylgruppe, Q die Gruppe -CO- oder >C=N-(Z)ₚ-R⁴, Z ein Sauerstoffatom oder eine Iminogruppe, p die Zähl 0 oder 1 und R⁴ Wasserstoff oder einen unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten und gegebenenfalls über eine Carbonylgruppe gebundenen, gesättigten oder partiell ungesättigten, niederen Kohlenwasserstoffrest bedeuten, wobei Ra Cyano bedeutet, wenn Rc' Cyano oder Nitro bedeutet und R³¹ eine von Hydroxy verschiedene Bedeutung hat, wenn m die Zahl 0 bedeutet, und wobei Aryl eine unsubstituierte oder durch Halogen, Trifluormethyl, Nitro, Amino, mono- oder di(niederes Alkyl)amino, niederes Alkyl, niederes Alkoxy, niederes Alkylthio, niederes Alkanoyl, niederes Alkoxycarbonyl, Carboxy, Hydroxy, Cyano, niederes Alkanoyloxy, Carbamoyl, mono- oder di(niederes Alkyl)carbamoyl, niederes Alkylendioxy, niederes Alkanoylamino oder niederes Alkoxycarbonylamino mono- oder disubstituierte Phenyl- oder Naphthylgruppe, Heteroaryl eine über ein Kohlenstoffatom gebundene unsubstituierte oder durch Halogen, Trifluormethyl, Nitro, Carboxy, Amino, Arylamino, niederes Alkyl, niederes Alkoxy, Hydroxy, niederes Alkoxycarbonyl, niederes Alkanoyl, niederes Alkanyoloxy, Oxo, niederes Alkylendioxy, Mercapto, niederes Alkylthio, niederes Alkylamino, di(niederes Alkyl)amino, C₃₋₇-Cycloalkylamino, C₈₋₁₀-Bicycloalkylamino, niederes Alkanoylamino, niederes Alkoxycarbonylamino, Carbamoyl, mono-oder di(niederes Alkyl)carbamoyl, Cyano, Aryl, Aryl-niederes Alkyl, Aryl-niederes Alkylamino, Heteroaryl, Heteroaryl-niederes Alkyl, Heteroarylamino oder C₃₋₇-Cycloalkyl mono-, di- oder trisubstituierte heterocyclische Gruppe ausgewählt aus der Gruppe, bestehend aus Pyridyl, Pyrazinyl, Triazinyl, Thiadiazinyl, Thiazolyl, Oxazolyl, Oxadiazolyl, Pyrazolyl, Tetrazolyl, Imidazolyl, Thienyl, Chinolinyl, Isochinolinyl, Dihydroisochinolinyl, Benzoxazinyl, Chinoxalinyl, Benzopyranyl, Benzimidazolyl, Indolyl, Imidazothiazolyl, Imidazothiadiazolyl, Imidiazopyridyl, Benzothiazinyl, Benzochinoxalinyl und Imidazobenzothiazinyl, und "nieder" Reste mit bis zu sieben Kohlenstoffatomen bezeichnen, von unter physiologischen Bedingungen hydrolysierbaren Ester- und Aetherderivaten und von pharmazeutisch annehmbaren Salzen davon, dadurch gekennzeichnet, dass man
a) in einer Verbindung der allgemeinen Formel 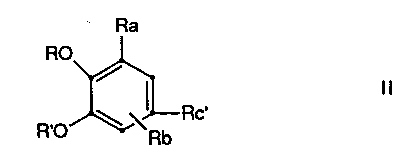 worin eines der Symbole R und R' niederes Alkyl und das andere Wasserstoff oder niederes Alkyl bedeuten, und Ra, Rb und Rc' obige Bedeutung besitzen,
die niedere(n) Alkyläthergruppe(n) spaltet, oder
b) eine Verbindung der allgemeinen Formel 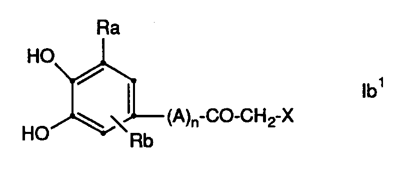 worin X eine Abgangsgruppe bedeutet, und Ra, Rb A und n obige Bedeutung besitzen,
mit einem Thioamid, Thioharnstoff, Thiocarbonsäurehydrazid, Thiosemicarbazid, Amidin, Guanidin, Amidrazon, Aminoguanidin, cyclischen Amidin, 1,2-Diamin, 1,2-Aminothiol oder einem 1,2-Aminoalkohol umsetzt und das erhaltene Cyclokondensationsprodukt erwünschtenfalls dehydriert, oder
c) eine Verbindung der allgemeinen Formel 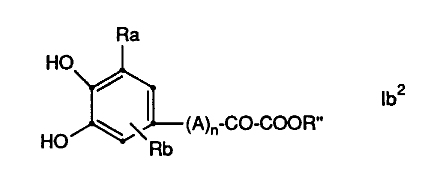 worin R'' niederes Alkyl bedeutet, und Ra, Rb, A und n obige Bedeutung besitzen,
mit einem 1,2-Diamin, 1,2-Aminothiol, 1,2-Aminoalkohol, Semicarbazid, Thiosemicarbazid, Amidrazon oder einem Aminoguanidin umsetzt und das erhaltene Cyclokondensationsprodukt erwünschtenfalls dehydriert, oder
d) eine Verbindung der obigen Formel Ib¹ mit einer β-Aminocarbonylverbindung umsetzt, oder
e) in einer Verbindung der allgemeinen Formel 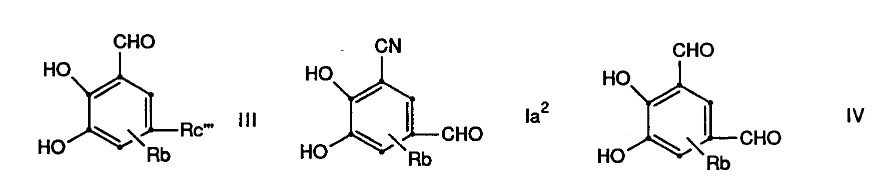 worin Rc''' Nitro, Cyano oder die Gruppe -(A)ₙ-R¹² und R¹² die Gruppe -COR³¹, Aryl oder Heteroaryl bedeuten, und Ra, Rb, A, n und R³¹ obige Bedeutung besitzen,
oder in einem Di-O-niederen Alkanoylderivat davon die Carboxaldehydgruppe(n) in die Cyanogruppe(n) überführt, oder
f) ein Di-O-niederes Alkanoylderivat einer Carbonsäure der allgemeinen Formel 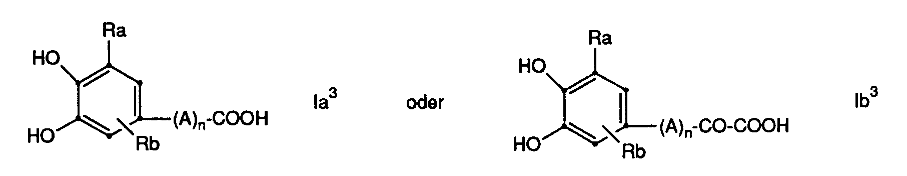 worin Ra, Rb, A und n obige Bedeutung besitzen, in Gegenwart eines Kondensationsmittels oder ein reaktives Derivat eines Di-O-niederen Alkanoylderivates einer Carbonsäure der Formel Ia³ oder Ib³ mit einer Verbindung der allgemeinen Formel
HO-R⁵ V oder HNR⁶R⁷ VI
worin R⁵ einen unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten gesättigten oder partiell ungesättigten, niederen Kohlenwasserstoffrest, R⁶ Wasserstoff oder niederes Alkyl und R⁷ Wasserstoff oder einen unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten gesättigten oder partiell ungesättigten, niederen Kohlenwasserstoffrest oder R⁶ und R⁷ zusammen mit dem Stickstoffatom eine unsubstituierte oder durch niederes Alkyl, Hydroxy, niederes Alkoxy, niederes Alkanoyloxy, niederes Hydroxyalkyl, niederes Alkoxyalkyl, niederes Alkanoyloxyalkyl, niederes Alkoxycarbonyl, niederes Alkanoyl, Carbamoyl, mono- oder di(niederes Alkyl)carbamoyl, Oxo und/oder niederes Alkylendioxy mono- oder disubstituierte 4-Morpholinyl-, 1-Pyrrolidinyl- oder 1-Azetidinylgruppe bedeuten,
umsetzt, oder
g) eine Verbindung der obigen Formel Ib² oder der allgemeinen Formel 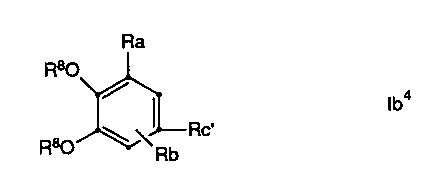 worin R⁸ niederes Alkanoyl bedeutet, und Ra, Rb und Rc' obige Bedeutung besitzen, hydrolysiert, oder
h) eine Verbindung der allgemeinen Formel 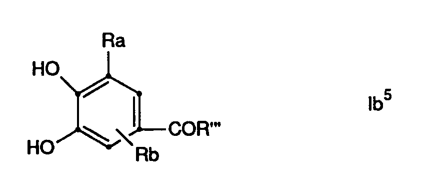 worin Ra und Rb obige Bedeutung besitzen, und R''' Wasserstoff oder niederes Alkyl bedeutet, oder ein Di-O-niederes Alkanoylderivat davon in Gegenwart eines sekundären Amins mit einer Verbindung der allgemeinen Formel
CH₃CO-R²³ VII
worin R²³ einen unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten gesättigten oder partiell ungesättigten, niederen Kohlenwasserstoffrest bedeutet, umsetzt, oder
i) eine Verbindung der allgemeinen Formel 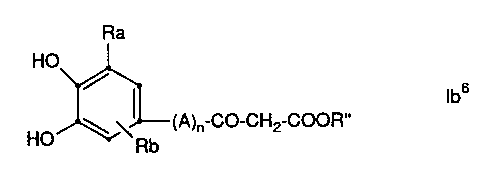 worin Ra, Rb, A, n und R'' obige Bedeutung besitzen, mit einem Hydrazin oder einem Amidin umsetzt, oder
j) eine Verbindung der obigen Formel Ib, worin m die Zahl 1 und Q die Gruppe -CO- bedeuten, mit einer Verbindung der allgemeinen Formel
H₂N-(Z)ₚ-R⁴ VIII
worin Z, p und R⁴ obige Bedeutung besitzen, umsetzt und erwünschtenfalls
k) eine Verbindung der obigen Formel Ib in ein unter physiologischen Bedingungen hydrolysierbares Ester- oder Aetherderivat oder in ein pharmazeutisch annehmbares Salz davon überführt.

2. Verfahren nach Anspruch 1, worin sich Rb in der p-Stellung zu Ra befindet.

3. Verfahren nach Anspruch 1 oder 2, worin Ra Nitro bedeutet.

4. Verfahren nach einem der Ansprüche 1-3, worin Rb Wasserstoff, Chlor oder Fluor bedeutet.

5. Verfahren nach Anspruch 4, worin Rb Wasserstoff bedeutet.

6. Verfahren nach einem der Ansprüche 1-5, worin Rc' die Gruppe -CO-R¹¹ und R¹¹ eine unsubstituierte oder durch Halogen, Trifluormethyl, Nitro, Amino, mono- oder di(niederes Alkyl)amino, niederes Alkyl, niederes Alkoxy, niederes Alkylthio, niederes Alkynoyl, niederes Alkoxy, niederes Alkylthio, niederes Alkanoyl, niederes Alkoxycarbonyl, Carboxy, Hydroxy, Cyano, niederes Alkanoyloxy, Carbamoyl, mono- oder di(niederes Alkyl)carbamoyl, niederes Alkylendioxy, niederes Alkanoylamino oder niederes Alkoxycarbonylamino mono- oder disubstituierte Phenylgruppe oder eine über ein Kohlenstoffatom gebundene, unsubstituierte oder durch Halogen, Trifluormethyl, Nitro, Carboxy, Amino, Arylamino, niederes Alkyl, niederes Alkoxy, Hydroxy, niederes Alkoxycarbonyl niederes Alkanoyl, niederes Alkanoyloxy, Oxo, niederes Alkylendioxy, Mercapto, niederes Alkylthio, niederes Alkylamino, di(niederes Alkyl)amino, C₃₋₇-Cycloalkylamino, C₈₋₁₀-Bicycloalkylamino, niederes Alkanoylamino, niederes Alkoxycarbonylamino, Carbamoyl, mono- oder di(niederes Alkyl)carbamoyl, Cyano, Aryl, Aryl-niederes Alkyl, Aryl-niederes Alkylamino, Heteroaryl, Heteroaryl-niederes Alkyl, Heteroarylamino oder C₃₋ ₇-Cycloalkyl mono-, di- oder trisubstituierte heterocyclische Gruppe ausgewählt aus der Gruppe bestehend aus Pyridyl, Pyrazinyl, Triazinyl, Pyrazolyl und Imidazolyl, bedeuten.

7. Verfahren nach Anspruch 6, worin R¹¹ eine gegebenenfalls durch Halogen, Trifluormethyl, Cyano, Hydroxy oder niederes Alkyl mono- oder disubstituierte Phenylgruppe oder eine Pyridylgruppe bedeutet.

8. Verfahren nach Anspruch 1, dadurch gekennzeichnet, dass man 3,4-Dihydroxy-4'-methyl-5-nitrobenzophenon herstellt.

9. Verfahren nach Anspruch 1, dadurch gekennzeichnet, dass man 2'-Fluor-3,4-dihydroxy-5-nitrobenzophenon herstellt.

10. Verfahren nach Anspruch 1, dadurch gekennzeichnet, dass man 3,4-Dihydroxy-5-nitrophenyl-4-pyridylketon herstellt.

11. Verwendung von Verbindungen der allgemeinen Formel 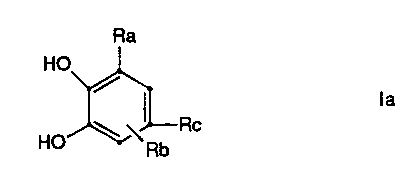 worin Ra Nitro oder Cyano, Rb Wasserstoff oder Halogen, Rc Halogen, Nitro, Cyano oder die Gruppe -(A)ₙ-(Q)ₘ-R¹ oder -(A)ₙ-Q-R², A gegebenenfalls durch niederes Alkyl substituiertes Vinylen, n die Zahl 0 oder 1, m die Zahl 0 oder 1, R¹ die Gruppe -COR³, Aryl oder Heteroaryl, R² Wasserstoff oder einen unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten, gesättigten oder partiell ungesättigten, niederen Kohlenwasserstoffrest, R³ Hydroxy, Amino, einen über ein Sauerstoffatom oder eine Imino- oder niedere Alkyliminogruppe gebundenen, unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten, gesättigten oder partiell ungesättigten, niederen Kohlenwasserstoffrest oder eine unsubstituierte oder durch niederes Alkyl, Hydroxy, niederes Alkoxy, niederes Alkanoyloxy, niederes Hydroxyalkyl, niederes Alkoxyalkyl, niederes Alkanoyloxyalkyl, niederes Alkoxycarbonyl, niederes Alkanoyl, Carbamoyl, mono- oder di(niederes Alkyl)carbamoyl, Oxo und/oder niederes Alkylendioxy mono- oder disubstituierte 4-Morpholinyl-, 1-Pyrrolidinyl- oder 1-Azetidinylgruppe, Q die Gruppe -CO- oder >C=N-(Z)ₚ-R⁴, Z ein Sauerstoffatom oder eine Iminogruppe, p die Zahl 0 oder 1 und R⁴ Wasserstoff oder einen unsubstituierten oder durch Hydroxy, Cyano, Nitro, Halogen, Amino, niederes Alkylamino, di(niederes Alkyl)amino, niederes Alkoxy, niederes Alkoxycarbonyl, Aryl, Arylaminocarbonyl, Arylcarbonyl, Arylcarbonylamino, niederes Alkanoyloxy, niederes Alkanoyl, Carbamoyl, mono- oder di(niederes Alkyl)carbamoyl, niederes Alkylendioxy, Trifluormethyl, Carboxy, niederes Alkanoylamino, niederes Alkoxycarbonylamino oder niederes Alkylthio mono- oder disubstituierten und gegebenenfalls über eine Carbonylgruppe gebundenen, gesättigten oder partiell ungesättigten, niederen Kohlenwasserstoffrest bedeuten, wobei Aryl eine unsubstituierte oder durch Halogen, Trifluormethyl, Nitro, Amino, mono- oder di(niederes Alkyl)amino, niederes Alkyl, niederes Alkoxy, niederes Alkylthio, niederes Alkanoyl, niederes Alkoxycarbonyl, Carboxy, Hydroxy, Cyano, niederes Alkanoyloxy, Carbamoyl, mono- oder di(niederes Alkyl)carbamoyl, niederes Alkylendioxy, niederes Alkanoylamino oder niederes Alkoxycarbonylamino mono- oder disubstituierte Phenyl- oder Naphthylgruppe, Heteroaryl eine über ein Kohlenstoffatom gebundene unsubstituierte oder durch Halogen, Trifluormethyl, Nitro, Carboxy, Amino, Arylamino, niederes Alkyl, niederes Alkoxy, Hydroxy, niederes Alkoxycarbonyl, niederes Alkanoyl, niederes Alkanyoloxy, Oxo, niederes Alkylendioxy, Mercapto, niederes Alkylthio, niederes Alkylamino, di(niederes Alkyl)amino, C₃₋₇-Cycloalkylamino, C₈₋₁₀-Bicycloalkylamino, niederes Alkanoylamino, niederes Alkoxycarbonylamino, Carbamoyl, mono-oder di(niederes Alkyl)carbamoyl, Cyano, Aryl, Aryl-niederes Alkyl, Aryl-niederes Alkylamino, Heteroraryl, Heteroaryl-niederes Alkyl, Heteroarylamino oder C₃₋₇-Cycloalkyl mono-, di- oder trisubstituierte heterocyclische Gruppe ausgewählt aus der Gruppe, bestehend aus Pyridyl, Pyrazinyl, Triazinyl, Thiadiazinyl, Thiazolyl, Oxazolyl, Oxadiazolyl, Pyrazolyl, Tetrazolyl, Imidazolyl, Thienyl, Chinolinyl, Isochinolinyl, Dihydroisochinolinyl, Benzoxazinyl, Chinoxalinyl, Benzopyranyl, Benzimidazolyl, Indolyl, Imidazothiazolyl, Imidazothiadiazolyl, Imidiazopyridyl, Benzothiazinyl, Benzochinoxalinyl und Imidazobenzothiazinyl, und "nieder" Reste mit bis zu sieben Kohlenstoffatomen bezeichnen, und unter physiologischen Bedingungen hydrolysierbaren Ester- und Aetherderivaten und pharmazeutisch annehmbaren Salzen davon zur Herstellung von Mitteln, welche die Catechol-O-Methyl-Transferase hemmen.

12. Verwendung von Verbindungen der in Anspruch 11 definierten Formel Ia zur Herstellung von Mitteln zur Behandlung der Parkinson-Krankheit und von Parkinsonismus in Kombination mit L-Dopa und einem peripheren Decarboxylase-Hemmer.

13. Verwendung von Verbindungen der in Anspruch 11 definierten Formel Ia zur Herstellung von Arzneimittelgarnituren, bestehend aus einem Mittel, enthaltend eine Verbindung der in Anspruch 11 definierten Formel Ia und ein therapeutisch inertes Trägermaterial und einem Mittel, enthaltend L-Dopa, einen peripheren Decarboxylase-Hemmer und ein therapeutisch inertes Trägermaterial.

## Claims (Claims for the following Contracting State(s): BE, CH, DE, FR, GB, IT, LI, LU, NL, SE)

1. Compounds of the general formula 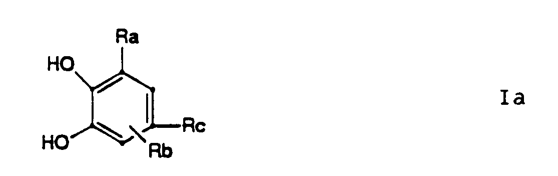 wherein Ra signifies nitro or cyano, Rb signifies hydrogen or halogen, Rc signifies halogen, nitro, cyano or the group -(A)ₙ-(Q)ₘ-R¹ or -(A)ₙ-Q-R², A signifies vinylene optionally substituted by lower alkyl, n signifies the number O or 1, m signifies the number O or 1, R¹ signifies the group -COR³, aryl or heteroaryl, R² signifies hydrogen or a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio, R³ signifies hydroxy, amino, a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio and which is attached via an oxygen atom or an imino or lower alkylimino group, or a 4-morpholinyl, 1-pyrrolidinyl or 1-azetidinyl group which is unsubstituted or mono- or disubstituted by lower alkyl, hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo and/or lower alkylenedioxy, Q signifies the group -CO- or >C=N-(Z)ₚ-R⁴, Z signifies an oxygen atom or an imino group, p signifies the number O or 1 and R⁴ signifies hydrogen or a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio and which is optionally attached via a carbonyl group, whereby aryl denotes a phenyl or naphthyl group which is unsubstituted or mono- or disubstituted by halogen, trifluoromethyl, nitro, amino, mono- or di(lower alkyl)amino, lower alkyl, lower alkoxy, lower alkylthio, lower alkanoyl, lower alkoxycarbonyl, carboxy, hydroxy, cyano, lower alkanoyloxy, carbamoyl, mono- or di(lower alkyl)carbamoyl, lower alkylenedioxy, lower alkanoylamino or lower alkoxycarbonylamino, heteroaryl denotes a heterocyclic group selected from the group consisting of pyridyl, pyrazinyl, triazinyl, thiadiazinyl, thiazolyl, oxazolyl, oxadiazolyl, pyrazolyl, tetrazolyl, imidazolyl, thienyl, quinolinyl, isoquinolinyl, dihydroisoquinolinyl, benzoxazinyl, quinoxalinyl, benzopyranyl, benzimidazolyl, indolyl, imidazothiazolyl, imidazothiadiazolyl, imidiazopyridyl, benzothiazinyl, benzoquinoxalinyl and imidazobenzothiazolyl which is attached via a carbon atom and which is unsubstituted or mono-, di- or trisubstituted by halogen, trifluoromethyl, nitro, carboxy, amino, arylamino, lower alkyl, lower alkoxy, hydroxy, lower alkoxycarbonyl, lower alkanoyl, lower alkanoyloxy, oxo, lower alkylenedioxy, mercapto, lower alkylthio, lower alkylamino, di(lower alkyl)amino, C₃₋₇-cycloalkylamino, C₈₋₁₀-bicycloalkylamino, lower alkanoylamino, lower alkoxycarbonylamino, carbamoyl, mono- or di(lower alkyl)carbamoyl, cyano, aryl, aryl-lower alkyl, aryl-lower alkylamino, heteroaryl, heteroaryl-lower alkyl, heteroarylamino or C₃₋₇-cycloalkyl, and lower denotes residues with up to 7 carbon atoms,
ester and ether derivatives which are hydrolyzable under physiological conditions and pharmaceutically acceptable salts thereof for use as therapeutically active substances.

2. Compounds of the general formula 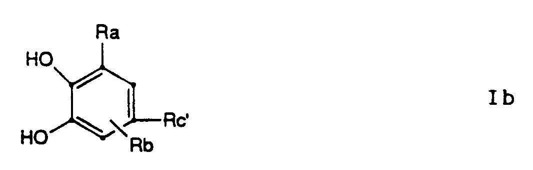 wherein Ra signifies nitro or cyano, Rb signifies hydrogen or halogen, Rc' signifies nitro, cyano or the group -(A)ₙ-(Q)ₘ-R¹¹ or -(A)ₙ-Q-R²¹, A signifies vinylene optionally substituted by lower alkyl, n signifies the number O or 1, m signifies the number O or 1, R¹¹ signifies the group -COR³¹, aryl or heteroaryl, R²¹ signifies a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio, R³¹ signifies hydroxy, amino, a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio and which is attached via an oxygen atom or an imino or lower alkylimino group, or a 4-morpholinyl, 1-pyrrolidinyl or 1-azetidinyl group which is unsubstituted or mono- or disubstituted by lower alkyl, hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo and/or lower alkylenedioxy, Q signifies the group -CO- or >C=N-(Z)ₚ-R⁴, Z signifies an oxygen atom or an imino group, p signifies the number O or 1 and R⁴ signifies hydrogen or a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio and which is optionally attached via a carbonyl group, whereby Ra signifies cyano when Rc' signifies cyano or nitro and R³¹ has a significance different from hydroxy when m signifies the number O, and whereby aryl denotes a phenyl or naphthyl group which is unsubstituted or mono- or disubstituted by halogen, trifluoromethyl, nitro, amino, mono- or di(lower alkyl)amino, lower alkyl, lower alkoxy, lower alkylthio, lower alkanoyl, lower alkoxycarbonyl, carboxy, hydroxy, cyano, lower alkanoyloxy, carbamoyl, mono- or di(lower alkyl)carbamoyl, lower alkylenedioxy, lower alkanoylamino or lower alkoxycarbonylamino, heteroaryl denotes a heterocyclic group selected from the group consisting of pyridyl, pyrazinyl, triazinyl, thiadiazinyl, thiazolyl, oxazolyl, oxadiazolyl, pyrazolyl, tetrazolyl, imidazolyl, thienyl, quinolinyl, isoquinolinyl, dihydroisoquinolinyl, benzoxazinyl, quinoxalinyl, benzopyranyl, benzimidazolyl, indolyl, imidazothiazolyl, imidazothiadiazolyl, imidiazopyridyl, benzothiazinyl, benzoquinoxalinyl and imidazobenzothiazolyl which is attached via a carbon atom and which is unsubstituted or mono-, di- or trisubstituted by halogen, trifluoromethyl, nitro, carboxy, amino, arylamino, lower alkyl, lower alkoxy, hydroxy, lower alkoxycarbonyl, lower alkanoyl, lower alkanoyloxy, oxo, lower alkylenedioxy, mercapto, lower alkylthio, lower alkylamino, di(lower alkyl)amino, C₃₋₇-cycloalkylamino, C₈₋₁₀-bicycloalkylamino, lower alkanoylamino, lower alkoxycarbonylamino, carbamoyl, mono- or di(lower alkyl)carbamoyl, cyano, aryl, aryl-lower alkyl, aryl-lower alkylamino, heteroaryl, heteroaryl-lower alkyl, heteroarylamino or C₃₋₇-cycloalkyl, and lower denotes residues with up to 7 carbon atoms,
and ester and ether derivatives which are hydrolyzable under physiological conditions and pharmaceutically acceptable salts thereof.

3. Compounds according to claim 2, wherein Rb is situated in the p-position to Ra.

4. Compounds according to claim 2 or 3, wherein Ra signifies nitro.

5. Compounds according to any one of claims 2-4, wherein Rb signifies hydrogen, chlorine or fluorine.

6. Compounds according to claim 5, wherein Rb signifies hydrogen.

7. Compounds according to any one of claims 2-6, wherein Rc' signifies the group -CO-R¹¹ and R¹¹ signifies a phenyl group which is unsubstituted or mono- or disubstituted by halogen, trifluoromethyl, nitro, amino, mono- or di(lower alkyl)amino, lower alkyl, lower alkoxy, lower alkylthio, lower alkynoyl, lower alkoxy, lower alkylthio, lower alkanoyl, lower alkoxycarbonyl, carboxy, hydroxy, cyano, lower alkanoyloxy, carbamoyl, mono- or di(lower alkyl)carbamoyl, lower alkylenedioxy, lower alkanoylamino or lower alkoxycarbonylamino or a heterocyclic group selected from the group consiting of pyridyl, pyrazinyl, triazinyl, pyrazolyl and imidazolyl which is unsubstituted or mono-, di- or trisubstituted by halogen, trifluoromethyl, nitro, carboxy, amino, arylamino, lower alkyl, lower alkoxy, hydroxy, lower alkoxycarbonyl, lower alkanoyl, lower alkanoyloxy, oxo, lower alkylenedioxy, mercapto, lower alkylthio, lower alkylamino, di(lower alkyl)amino, C₃₋₇-cycloalkylamino, C₈₋₁₀-bicycloalkylamino, lower alkanoylamino, lower alkoxycarbonylamino, carbamoyl, mono- or di(lower alkyl)carbamoyl, cyano, aryl, aryl-lower alkyl, aryl-lower alkylamino, heteroaryl, heteroaryl-lower alkyl, heteroarylamino or C₃₋₇-cycloalkyl and which is attached via a carbon atom.

8. Compounds according to claim 7, wherein R¹¹ signifies a phenyl group optionally mono- or disubstituted by halogen, trifluoromethyl, cyano, hydroxy or lower alkyl or a pyridyl group.

9. 3,4-Dihydroxy-4'-methyl-5-nitrobenzophenone.

10. 2'-Fluoro-3,4-dihydroxy-5-nitrobenzophenone.

11. 3,4-Dihydroxy-5-nitrophenyl 4-pyridyl ketone.

12. Compounds according to any one of claims 2-11 for use as therapeutically active substances.

13. Compounds according to any one of claims 1-11 for use as active substances which inhibit catechol-O-methyl-transferase.

14. A process for the manufacture of compounds according to any one of claims 2-11, of ester and ether derivatives which are hydrolyzable under physiological conditions and of pharmaceutically acceptable salts thereof, characterized by
a) cleaving off the lower alkyl ether group(s) in a compound of the general formula 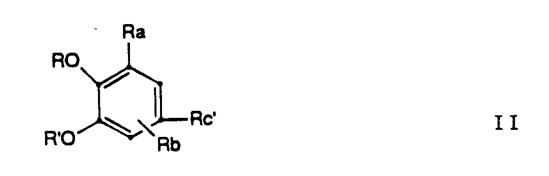 wherein one of the symbols R and R' signifies lower alkyl and the other signifies hydrogen or lower alkyl and Ra, Rb and Rc' have the significance given in claim 2,
or
b) reacting a compound of the general formula 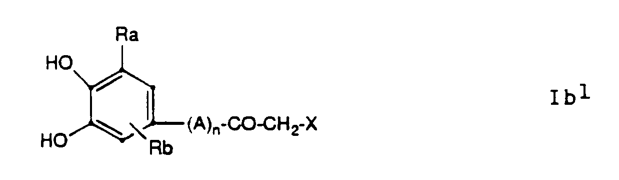 wherein X signifies a leaving group and Ra, Rb, A and n have the significance given in claim 2,
with a thioamide, thiourea, thiocarboxylic acid hydrazide, thiosemicarbazide, amidine, quanidine, amidrazone, aminoguanidine, cyclic amidine, 1,2-diamine, 1,2-aminothiol or a 1,2-aminoalcohol and, if desired, dehydrogenating the cyclocondensation product obtained, or
c) reacting a compound of the general formula 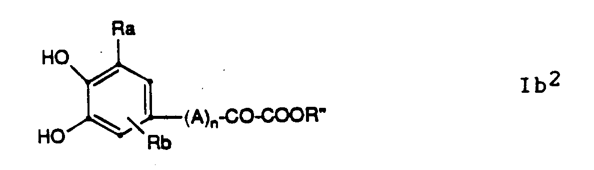 wherein R'' signifies lower alkyl and Ra, Rb, A and n have the significance given in claim 2,
with a 1,2-diamine, 1,2-aminothiol, 1,2-aminoalcohol, semicarbazide, thiosemicarbazide, amidrazone or an aminoguanidine and, if desired, dehydrogenating the cyclocondensation product obtained, or
d) reacting a compound of formula Ib¹ above with a β-aminocarbonyl compound, or
e) converting the carboxaldehyde group(s) in a compound of the general formula 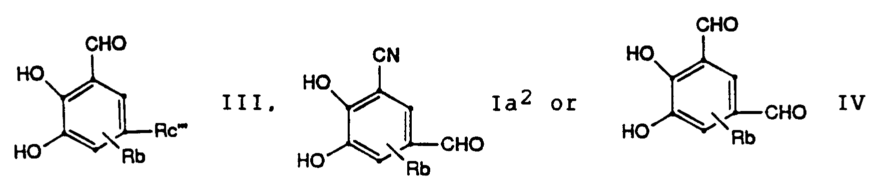 wherein Rc''' signifies nitro, cyano or the group -(A)ₙ-R¹² and R¹² signifies the group -COR³¹, aryl or heteroaryl and Ra, Rb, A, n and R³¹ have the significance given in claim 2,
or in a di-O-lower alkanoyl derivative thereof into the cyano group(s), or
f) reacting a di-O-lower alkanoyl derivative of a carboxylic acid of the general formula 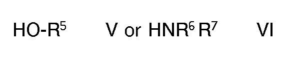 wherein Ra, Rb, A and n have the significance given in claim 2,
in the presence of a condensation agent, or a reactive derivative or a di-O-lower alkanoyl derivative of a carboxylic acid of formula Ia³ or Ib³, with a compound of the general formula
HO-R⁵ V or HNR⁶R⁷ VI
wherein R⁵ signifies a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio, R⁶ signifies hydrogen or lower alkyl and R⁷ signifies hydrogen or a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio or R⁶ and R⁷ together with the nitrogen atom signify a 4-morpholinyl, 1-pyrrolidinyl or 1-azetidinyl group which is unsubstituted or mono- or disubstituted by lower alkyl, hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo and/or lower alkylenedioxy,
or
g) hydrolyzing a compound of formula Ib² above or a compound of the general formula 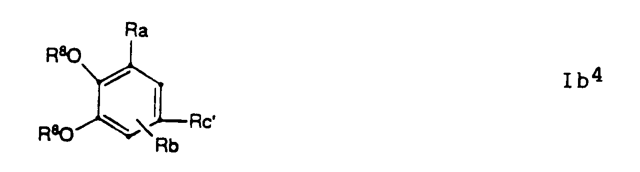 wherein R⁸ signifies lower alkanoyl and Ra, Rb and Rc' have the significance given in claim 2,
or
h) reacting a compound of the general formula 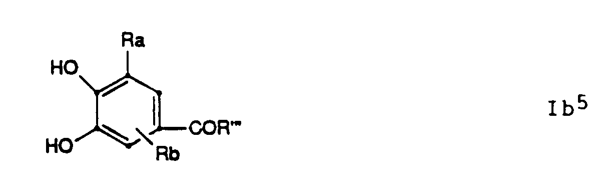 wherein Ra and Rb have the significance given in claim 2 and R''' signifies hydrogen or lower alkyl,
or a di-O-lower alkanoyl derivative thereof in the presence of a secondary amine with a compound of the general formula
CH₃CO-R²³ VII
wherein R²³ signifies a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio,
or
i) reacting a compound of the general formula 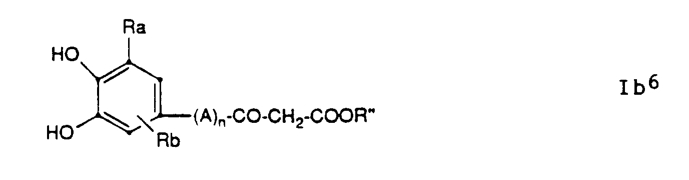 wherein Ra, Rb, A and n have the significance given in claim 2 and R'' has the above significance,
with a hydrazine or an amidine, or
j) reacting a compound of formula Ib above in which m signifies the number 1 and Q signifies the group -CO- with a compound of the general formula
H₂N-(Z)ₚ-R⁴ VIII
wherein Z, p and R⁴ have the significance give in claim 2,
and, if desired,
k) converting a compound of formula Ib above into an ester or ether derivative which is hydrolyzable under physiological conditions or into a pharmaceutically acceptable salt thereof.

15. A medicament containing a compound according to any one of claims 1-11 and a therapeutically inert carrier material.

16. A medicament for inhibiting catechol-O-methyl-transferase, containing a compound according to any one of claims 1-11 and a therapeutically inert carrier material.

17. A medicament for the treatment of Parkinson's disease and of parkinsonism, containing L-dopa, a peripheral decarboxylase inhibitor, a compound according to any one of claims 1-11 and a therapeutically inert carrier material.

18. A medicament pack for the treatment of Parkinson's disease and of parkinsonism, consisting of a medicament according to claim 16 and a medicament containing L-dopa, a peripheral decarboxylase inhibitor and a therapeutically inert carrier material.

19. The use of compounds according to any one of claims 1-11 for the manufacture of medicaments which inhibit catechol-O-methyltransferase.

20. The use of compounds according to any one of claims 1-11 for the manufacture of medicament for the treatment of Parkinson's disease and of parkinsonism in combination with L-dopa and a peripheral decarboxylase inhibitor.

21. The use of compounds according to any one of claims 1-11 for the manufacture of medicament packs according to claim 18.

## Claims (Claims for the following Contracting State(s): AT, ES, GR)

1. A process for the manufacture of compounds of the general formula 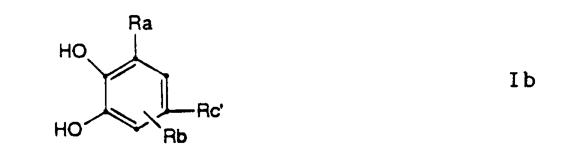 wherein Ra signifies nitro or cyano, Rb signifies hydrogen or halogen, Rc' signifies nitro, cyano or the group -(A)ₙ-(Q)ₘ-R¹¹ or -(A)ₙ-Q-R²¹, A signifies vinylene optionally substituted by lower alkyl, n signifies the number O or 1, m signifies the number O or 1, R¹¹ signifies the group -COR³¹, aryl or heteroaryl, R²¹ signifies a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio, R³¹ signifies hydroxy, amino, a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl lower alkylenedioxy, trifluoromethyl, carboxy, lower alkanoylamino, lower alkoxycarbonylamino or lower alkylthio, and which is attached via an oxygen atom or an imino or lower alkylimino group, or a 4-morpholinyl, 1-pyrrolidinyl or 1-azetidinyl group which is unsubstituted or mono- or disubstituted by lower alkyl, hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo and/or lower alkylenedioxy, Q signifies the group -CO- or >C=N-(Z)ₚ-R⁴, Z signifies an oxygen atom or an imino group, p signifies the number O or 1 and R⁴ signifies hydrogen or a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio and which is optionally attached via a carbonyl group, whereby Ra signifies cyano when Rc' signifies cyano or nitro and R³¹ has a significance different from hydroxy when m signifies the number O, and whereby aryl denotes a phenyl or naphthyl group which is unsubstituted or mono- or disubstituted by halogen, trifluoromethyl, nitro, amino, mono- or di(lower alkyl)amino, lower alkyl, lower alkoxy, lower alkylthio, lower alkanoyl, lower alkoxycarbonyl, carboxy, hydroxy, cyano, lower alkanoyloxy, carbamoyl, mono- or di(lower alkyl)carbamoyl, lower alkylenedioxy, lower alkanoylamino or lower alkoxycarbonylamino, heteroaryl denotes a heterocyclic group selected from the group consisting of pyridyl, pyrazinyl, triazinyl, thiadiazinyl, thiazolyl, oxazolyl, oxadiazolyl, pyrazolyl, tetrazolyl, imidazolyl, thienyl, quinolinyl, isoquinolinyl, dihydroisoquinolinyl, benzoxazinyl, quinoxalinyl, benzopyranyl, benzimidazolyl, indolyl, imidazothiazolyl, imidazothiadiazolyl, imidiazopyridyl, benzothiazinyl, benzoquinoxalinyl and imidazobenzothiazolyl which is attached via a carbon atom and which is unsubstituted or mono-, di- or trisubstituted by halogen, trifluoromethyl, nitro, carboxy, amino, arylamino, lower alkyl, lower alkoxy, hydroxy, lower alkoxycarbonyl, lower alkanoyl, lower alkanoyloxy, oxo, lower alkylenedioxy, mercapto, lower alkylthio, lower alkylamino, di(lower alkyl)amino, C₃₋₇-cycloalkylamino, C₈₋₁₀-bicycloalkylamino, lower alkanoylamino, lower alkoxycarbonylamino, carbamoyl, mono- or di(lower alkyl)carbamoyl, cyano, aryl, aryl-lower alkyl, aryl-lower alkylamino, heteroaryl, heteroaryl-lower alkyl, heteroarylamino or C₃₋₇-cycloalkyl, and lower denotes residues with up to 7 carbon atoms,
of ester and ether derivatives which are hydrolyzable under physiological conditions and of pharmaceutically acceptable salts thereof, characterized by
a) cleaving off the lower alkyl ether group(s) in a compound of the general formula 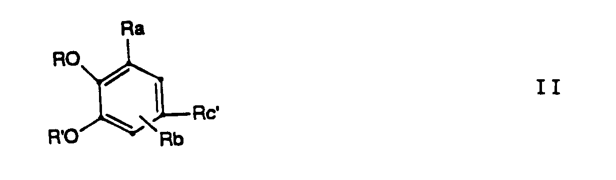 wherein one of the symbols R and R' signifies lower alkyl and the other signifies hydrogen or lower alkyl and Ra, Rb and Rc' have the above significance,
or
b) reacting a compound of the general formula 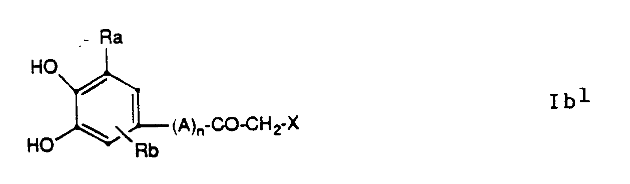 wherein X signifies a leaving group and Ra, Rb, A and n have the above significance,
with a thioamide, thiourea, thiocarboxylic acid hydrazide, thiosemicarbazide, amidine, guanidine, amidrazone, aminoguanidine, cyclic amidine, 1,2-diamine, 1,2-aminothiol or a 1,2-aminoalcohol and, if desired, dehydrogenating the cyclocondensation product obtained, or
c) reacting a compound of the general formula 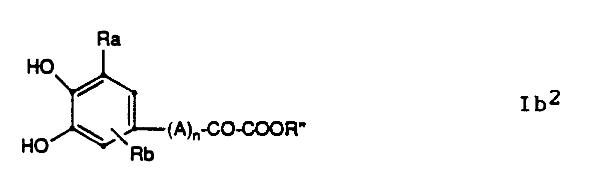 wherein R'' signifies lower alkyl and Ra, Rb, A and n have the above significance,
with a 1,2-diamine, 1,2-aminothiol, 1,2-aminoalcohol, semicarbazide, thiosemicarbazide, amidrazone or an aminoguanidine and, if desired, dehydrogenating the cyclocondensation product obtained, or
d) reacting a compound of formula Ib¹ above with a β-aminocarbonyl compound, or
e) converting the carboxaldehyde group(s) in a compound of the general formula 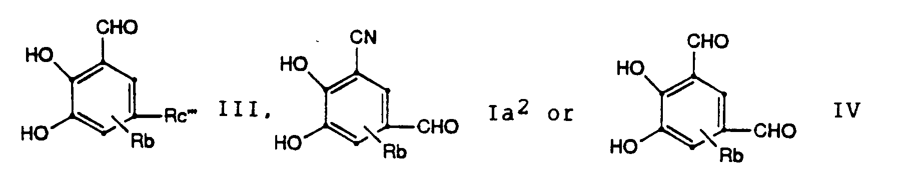 wherein Rc''' signifies nitro, cyano or the group -(A)ₙ-R¹² and R¹² signifies the group -COR³¹, aryl or heteroaryl and Ra, Rb, A, n and R³¹ have the above significance,
or in a di-O-lower alkanoyl derivative thereof into the cyano group(s), or
f) reacting a di-O-lower alkanoyl derivative of a carboxylic acid of the general formula 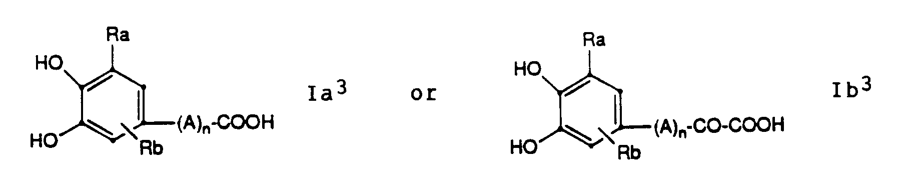 wherein Ra, Rb, A and n have the above significance, in the presence of a condensation agent, or a reactive derivative or a di-O-lower alkanoyl derivative of a carboxylic acid of formula Ia³ or Ib³, with a compound of the general formula
HO-R⁵ V or HNR⁶R⁷ VI
wherein R⁵ signifies a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio, R⁶ signifies hydrogen or lower alkyl and R⁷ signifies hydrogen or a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio or R⁶ and R⁷ together with the nitrogen atom signify a 4-morpholinyl, 1-pyrrolidinyl or 1-azetidinyl group which is unsubstituted or mono- or disubstituted by lower alkyl, hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo and/or lower alkylenedioxy,
or
g) hydrolyzing a compound of formula Ib² above or a compound of the general formula 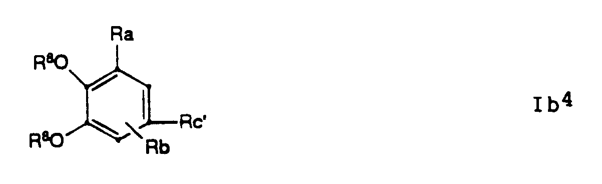 wherein R⁸ signifies lower alkanoyl and Ra, Rb and Rc' have the above significance,
or
h) reacting a compound of the general formula 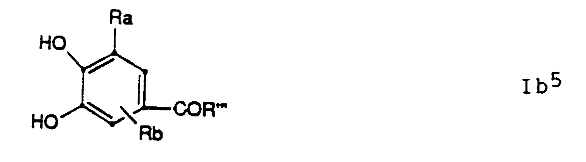 wherein Ra and Rb have the above significance and R''' signifies hydrogen or lower alkyl,
or a di-O-lower alkanoyl derivative thereof in the presence of a secondary amine with a compound of the general formula
CH₃CO-R²³ VII
wherein R²³ signifies a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio,
or
i) reacting a compound of the general formula 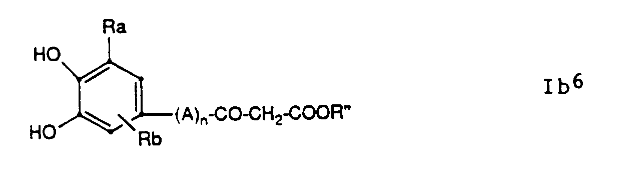 wherein Ra, Rb, A, n and R'' have the above significance,
with a hydrazine or an amidine, or
j) reacting a compound of formula Ib above in which m signifies the number 1 and Q signifies the group -CO- with a compound of the general formula
H₂N-(Z)ₚ-R⁴ VIII
wherein Z, p and R⁴ have the above significance, and, if desired,
k) converting a compound of formula Ib above into an ester or ether derivative which is hydrolyzable under physiological conditions or into a pharmaceutically acceptable salt thereof.

2. A process according to claim 1, wherein Rb is situated in the p-position to Ra.

3. A process according to claim 1 or 2, wherein Ra signifies nitro.

4. A process according to any one of claims 1-3, wherein Rb signifies hydrogen, chlorine or fluorine.

5. A process according to claim 4, wherein Rb signifies hydrogen.

6. A process according to any one of claims 1-5, wherein Rc' signifies the group -CO-R¹¹ and R¹¹ signifies a phenyl group which is unsubstituted or mono- or disubstituted by halogen, trifluoromethyl, nitro, amino, mono- or di(lower alkyl)amino, lower alkyl, lower alkoxy, lower alkylthio, lower alkynoyl, lower alkoxy, lower alkylthio, lower alkanoyl, lower alkoxycarbonyl, carboxy, hydroxy, cyano, lower alkanoyloxy, carbamoyl, mono- or di(lower alkyl)carbamoyl, lower alkylenedioxy, lower alkanoylamino or lower alkoxycarbonylamino or a heterocyclic group selected from the group consiting of pyridyl, pyrazinyl, triazinyl, pyrazolyl and imidazolyl which is unsubstituted or mono- or di- or trisubstituted by halogen, trifluoromethyl, nitro, carboxy, amino, arylamino, lower alkyl, lower alkoxy, hydroxy, lower alkoxycarbonyl, lower alkanoyl, lower alkanoyloxy, oxo, lower alkylenedioxy, mercapto, lower alkthythio, lower alkylamino, di(lower alkyl)amino, C₃₋₇-cycloalkylamino, C₈₋₁₀-bicycloalkylamino, lower alkanoylamino, lower alkoxycarbonylamino, carbamoyl, mono- or di(lower alkyl)carbamoyl, cyano, aryl, aryl-lower alkyl, aryl-lower alkylamino, heteroaryl, heteroaryl-lower alkyl, heteroarylamino or C₃₋₇-cycloalkyl.

7. A process according to claim 6, wherein R¹¹ signifies a phenyl group optionally mono- or disubstituted by halogen, trifluoromethyl, cyano, hydroxy or lower alkyl or a pyridyl group.

8. A process according to claim 1, characterized in that 3,4-dihydroxy-4'-methyl-5-nitrobenzophenone is manufactured.

9. A process according to claim 1, characterized in that 2'-fluoro-3,4-dihydroxy-5-nitrobenzophenone is manufactured.

10. A process according to claim 1, characterized in that 3,4-dihydroxy-5-nitrophenyl 4-pyridyl ketone is manufactured.

11. The use of compounds of the general formula 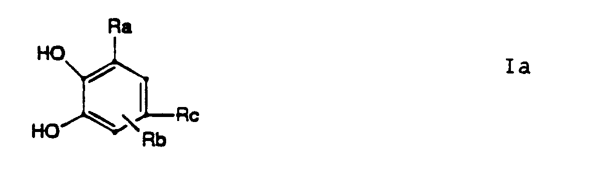 wherein Ra signifies nitro or cyano, Rb signifies hydrogen or halogen, Rc signifies halogen, nitro, cyano or the group -(A)ₙ-(Q)ₘ-R¹ or -(A)ₙ-Q-R². A signifies vinylene optionally substituted by lower alkyl, n signifies the number O or 1, m signifies the number O or 1, R¹ signifies the group -COR³, aryl or heteroaryl, R² signifies hydrogen or a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio, R³ signifies hydroxy, amino, a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio and which is attached via an oxygen atom or an imino or lower alkylimino group, or a 4-morpholinyl, 1-pyrrolidinyl or 1-azetidinyl group which is unsubstituted or mono- or disubstituted by lower alkyl, hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo and/or lower alkylenedioxy, Q signifies the group -CO- or >C=N-(Z)ₚ-R⁴, Z signifies an oxygen atom or an imino group, p signifies the number O or 1 and R⁴ signifies hydrogen or a saturated or partially unsaturated, lower hydrocarbon residue which is unsubstituted or mono- or disubstituted by hydroxy, cyano, nitro, halogen, amino, lower alkylamino, di(lower alkyl)amino, lower alkoxy, lower alkoxycarbonyl, aryl, arylaminocarbonyl, arylcarbonyl, arylcarbonylamino, lower alkanoyloxy, lower alkanoyl, carbamoyl, mono- or di(lower alkyl)carbamoyl, lower alkylenedioxy, trifluoromethyl, carboxy, lower alkanoylamino, lower alkoxycarbonylamino or lower alkylthio and which is optionally attached via a carbonyl group, whereby aryl denotes a phenyl or naphthyl group which is unsubstituted or mono- or disubstituted by halogen, trifluoromethyl, nitro, amino, mono- or di(lower alkyl)amino, lower alkyl, lower alkoxy, lower alkylthio, lower alkanoyl, lower alkoxycarbonyl, carboxy, hydroxy, cyano, lower alkanoyloxy, carbamoyl, mono- or di(lower alkyl)carbamoyl, lower alkylenedioxy, lower alkanoylamino or lower alkoxycarbonylamino, heteroaryl denotes a heterocyclic group selected from the group consisting of pyridyl, pyrazinyl, triazinyl, thiadiazinyl, thiazolyl, oxazolyl, oxadiazolyl, pyrazolyl, tetrazolyl, imidazolyl, thienyl, quinolinyl, isoquinolinyl, dihydroisoquinolinyl, benzoxazinyl, quinoxalinyl, benzopyranyl, benzimidazolyl, indolyl, imidazothiazolyl, imidazothiadiazolyl, imidiazopyridyl, benzothiazinyl, benzoquinoxalinyl and imidazobenzothiazolyl which is attached via a carbon atom and which is unsubstituted or mono-, di- or trisubstituted by halogen, trifluoromethyl, nitro, carboxy, amino, arylamino, lower alkyl, lower alkoxy, hydroxy, lower alkoxycarbonyl, lower alkanoyl, lower alkanoyloxy, oxo, lower alkylenedioxy, mercapto, lower alkylthio, lower alkylamino, di(lower alkyl)amino, C₃₋₇-cycloalkylamino, C₈₋₁₀-bicycloalkylamino, lower alkanoylamino, lower alkoxycarbonylamino, carbamoyl, mono- or di(lower alkyl)carbamoyl, cyano, aryl, aryl-lower alkyl, aryl-lower alkylamino, heteroaryl, heteroaryl-lower alkyl, heteroarylamino or C₃₋₇-cycloalkyl, and lower denotes residues with up to 7 carbon atoms,
and ester and ether derivatives which are hydrolyzable under physiological conditions and pharmaceutically acceptable salts thereof for the manufacture of medicaments which inhibit catechol-O-methyltransferase.

12. The use of compounds of formula Ia defined in claim 11 for the manufacture of medicaments for the treatment of Parkinson's disease and of parkinsonism in combination with L-dopa and a peripheral decarboxylase inhibitor.

13. The use of compounds of formula Ia defined in claim 11 for the manufacture of medicament packs consisting of a medicament containing a compound of formula Ia defined in claim 11 and a therapeutically inert carrier material and a medicament containing L-dopa, a peripheral decarboxylase inhibitor and a therapeutically inert carrier material.

## Revendications (Revendications pour l'(les) Etat(s) contractant(s) suivant(s): BE, CH, DE, FR, GB, IT, LI, LU, NL, SE)

1. Composés de formule générale 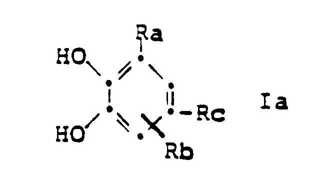 où Ra représente un nitro ou un cyano, Rb représente l'hydrogène ou un halogène, Rc représente un halogène, un nitro, un cyano ou le groupe -(A)ₙ-(Q)ₘ-R¹ ou -(A)ₙ-Q-R², A représentant le vinylène éventuellement substitué par un alkyle inférieur, n est égal à 0 ou 1, m est égal à 0 ou 1, R¹ représentant le groupe -COR³, un groupe aryle ou un hétéroaryle, R² représentant l'hydrogène ou un reste hydrocarboné inférieur, saturé ou partiellement insaturé, non substitué ou mono- ou disubstitué par un hydroxy, un cyano, un nitro, un halogène, un amino, un alkylamino inférieur, un di(alkyle inférieur) amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di-(alkyle inférieur)carbamoyle, un alkylène inférieur dioxy, le trifluorométhyle, un carboxy, un alcanoyle inférieur amino, un alcoxy inférieur carbonylamino ou un alkyle inférieur thio, R³ représentant un hydroxy, un amino, un reste hydrocarboné inférieur, saturé ou partiellement insaturé, lié par l'intermédiaire d'un atome d'oxygène ou d'un groupe imino ou d'un groupe alkylimino, ledit reste hydrocarboné étant non substitué ou mono- ou disubstitué par un hydroxy, un cyano, un nitro, un halogène, un amino, un alkyle inférieur amino, un di(alkyle inférieur)amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur)carbamoyle, un alkylène inférieur dioxy, le trifluorométhyle, un carboxy, un alcanoyle inférieur amino, un alcoxy inférieur carbonylamino ou un alkyle inférieur thio ou un groupe 4-morpholinyle, 1-pyrrolidinyle ou 1-azétidinyle non substitué ou mono- ou disubstitué par un alkyle inférieur, un hydroxy, un alcoxy inférieur, un alcanoyle inférieur oxy, un hydroxyalkyle inférieur, un alcoxy inférieur alkyle, un alcanoyle inférieur oxyalkyle, un alcoxy inférieur carbonyle, un alcanoyle inférieur, un carbamoyle, un mono- ou di(alkyle inférieur)carbamoyle, un oxo et/ou un alkylène inférieur dioxy, Q représentant le groupe -CO- ou 〉C=N-(Z)ₚ-R⁴, Z étant un atome d'oxygène ou un groupe imino, p étant égal à 0 ou 1, et R⁴ représentant l'hydrogène ou un reste hydrocarboné inférieur, saturé ou partiellement insaturé, éventuellement lié par un groupe carbonyle, ledit reste hydrocarboné étant non substitué ou mono- ou disubstitué par un hydroxy, un cyano, un nitro, un halogène, un amino, un alkyle inférieur amino, un di(alkyle inférieur)amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur)carbamoyle, un alkylène inférieur dioxy, le trifluorométhyle, un carboxy, un alcanoyle inférieur amino, un alcoxy inférieur carbonylamino ou un alkyle inférieur thio, un aryle signifiant un groupe phényle ou naphtyle non substitué ou mono- ou disubstitué par un halogène, le trifluorométhyle, un nitro, un amino, un mono- ou di(alkyle inférieur)amino, un alkyle inférieur, un alcoxy inférieur, un alkyle inférieur thio, un alcanoyle inférieur, un alcoxy inférieur carbonyle, un carboxy, un hydroxy, un cyano, un alcanoyle inférieur oxy, un carbamoyle, un mono- ou di(alkyle inférieur)carbamoyle, un alkylène inférieur dioxy, un alcanoyle inférieur amino ou un alcoxy inférieur carbonylamino, un hétéroaryle signifiant un groupe hétérocyclique lié par l'intermédiaire d'un atome de carbone, non substitué ou mono-, di- ou trisubstitué par un halogène, le trifluorométhyle, un nitro, un carboxy, un amino, un arylamino, un alkyle inférieur, un alcoxy inférieur, un hydroxy, un alcoxy inférieur carbonyle, un alcanoyle inférieur, un alcanoyle inférieur oxy, un oxo, un alkylène inférieur dioxy, un mercapto, un alkyle inférieur thio, un alkyle inférieur amino, un di(alkyle inférieur) amino, un cycloalkyle en C₃₋₇ amino, un bicycloalkyle en C₈₋₁₀ amino, un alcanoyle inférieur amino, un alcoxy inférieur carbonylamino, un carbamoyle, un mono- ou di(alkyle inférieur) carbamoyle, un cyano, un aryle, un aryle alkyle inférieur, un aryle alkyle inférieur amino, un hétéroaryle, un hétéroaryle alkyle inférieur, un hétéroarylamino ou un cycloalkyle en C₃₋₇, ledit groupe hétérocyclique étant choisi dans le groupe constitué par le pyridyle, le pyrazinyle, le triazinyle, le thiadiazinyle, le thiazolyle, l'oxazolyle, l'oxadiazolyle, le pyrazolyle, le tétrazolyle, l'imidazolyle, le thiényle, le quinoléinyle, l'isoquinoléinyle, le dihydroisoquinoléinyle, le benzoxazinyle, le quinoxalinyle, le benzopyranyle, le benzimidazolyle, l'indolyle, l'imidazothiazolyle, l'imidazothiadiazolyle, l'imidazopyridyle, le benzothiazinyle, le benzoquinoxalinyle et l'imidazobenzothiazolyle et des restes inférieurs ayant jusqu'à 7 atomes de carbone et leurs dérivés d'esters et d'éthers hydrolysables dans les conditions physiologiques et leurs sels pharmaceutiquement acceptables destinés à être utilisés comme substances actives thérapeutiques.

2. Composés de formule générale 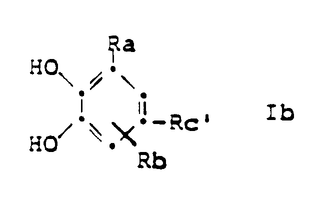 où Ra représente un nitro ou un cyano, Rb représente l'hydrogène ou un halogène, Rc' représente un nitro, un cyano ou le groupe -(A)ₙ-(Q)ₘ-R¹¹ ou -(A)ₙ-(Q)-R²¹ A représentant le vinylène éventuellement substitué par un alkyle inférieur, n étant égal à 0 ou 1, m étant égal à 0 ou 1, R¹¹ représentant le groupe -COR³¹, un aryle ou un hétéroaryle, R²¹ représentant un reste hydrocarboné inférieur, saturé ou partiellement insaturé, non substitué ou mono- ou disubstitué par un hydroxy, un cyano, un nitro, un halogène, un amino, un alkyle inférieur amino, un di(alkyle inférieur)amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur) carbamoyle, un alkylène inférieur dioxy, le trifluorométhyle, un carboxy, un alcanoyle inférieur amino, un alcoxy inférieur carbonylamino ou un alkyle inférieur thio, R³¹ représentant un hydroxy, un amino, un reste hydrocarboné inférieur, saturé ou partiellement insaturé, lié par l'intermédiaire d'un atome d'oxygène ou d'un groupe imino ou d'un groupe alkyle inférieur imino, non substitué ou mono- ou disubstitué par un hydroxy, un cyano, un nitro, un halogène, un amino, un alkyle inférieur amino, un di(alkyle inférieur)amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur) carbamoyle, un alkylène inférieur dioxy, le trifluorométhyle, un carboxy, un alcanoyle inférieur amino, un alcoxy inférieur carbonylamino ou un alkyle inférieur thio, ou un groupe 4-morpholinyle, 1-pyrrolidinyle ou 1-azétidinyle non substitué ou mono- ou disubstitué par un alkyle inférieur, un hydroxy, un alcoxy inférieur, un alcanoyle inférieur oxy, un hydroxyalkyle inférieur, un alcoxy inférieur alkyle, un alcanoyle inférieur oxyalkyle, un alcoxy inférieur carbonyle, un alcanoyle inférieur, un carbamoyle, un mono- ou di(alkyle inférieur) carbamoyle, un oxo et/ou un alkylène inférieur dioxy, Q représentant le groupe -CO- ou 〉C=N-(Z)ₚ-R⁴, Z représentant un atome d'oxygène ou un groupe imino, p étant égal à 0 ou 1 et R⁴ représentant l'hydrogène ou un reste hydrocarboné inférieur, saturé ou partiellement insaturé, et éventuellement lié par l'intermédiaire d'un groupe carbonyle, ledit reste hydrocarboné étant non substitué ou mono- ou disubstitué par un hydroxy, un cyano, un nitro, un halogène, un amino, un alkyle inférieur amino, un di(alkyle inférieur)amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur)carbamoyle, un alkylène inférieur dioxy, le trifluorométhyle, un carboxy, un alcanoyle inférieur amino, un alcoxy inférieur carbonylamino ou un alkyle inférieur thio, Ra représentant un cyano, lorsque Rc' représente un cyano ou un nitro et R³¹ ayant une signification différente de l'hydroxy, lorsque m est égal à 0 et un aryle signifiant un groupe phényle ou naphtyle non substitué ou mono- ou disubstitué par un halogène, le trifluorométhyle, un nitro, un amino, un mono- ou di(alkyle inférieur) amino, un alkyle inférieur, un alcoxy inférieur, un alkyle inférieur thio, un alcanoyle inférieur, un alcoxy inférieur carbonyle, un carboxy, un hydroxy, un cyano, un alcanoyle inférieur oxy, un carbamoyle, un mono- ou di(alkyle inférieur)carbamoyle un alkylène inférieur dioxy, un alcanoyle inférieur amino ou un alcoxy inférieur carbonylamino, un hétéroaryle signifiant un groupe hétérocyclique lié par l'intermédiaire d'un atome de carbone, non substitué ou mono-, di- ou trisubstitué par un halogène, le trifluorométhyle, un nitro, un carboxy, un amino, un arylamino, un alkyle inférieur, un alcoxy inférieur, un hydroxy, un alcoxy inférieur carbonyle, un alcanoyle inférieur, un alcanoyle inférieur oxy, un oxo, un alkylène inférieur dioxy, un mercapto, un alkyle inférieur thio, un alkyle inférieur amino, un di(alkyle inférieur)amino, un cycloalkyle en C₃₋₇ amino, un bicycloalkyle en C₈₋₁₀ amino, un alcanoyle inférieur amino, un alcoxy inférieur carbonylamino, un carbamoyle, un mono- ou di(alkyle inférieur) carbamoyle, un cyano, un aryle, un aryle alkyle inférieur, un aryle alkyle inférieur amino, un hétéroaryle, un hétéroaryle alkyle inférieur, un hétéroarylamino au un cycloalkyle en C₃₋₇, ledit groupe hétérocyclique étant choisi dans le groupe constitué par le pyridyle, le pyrazinyle, le triazinyle, le thiadiazinyle, le thiazolyle, l'oxazolyle, l'oxadiazolyle, le pyrazolyle, le tétrazolyle, l'imidazolyle, le thiényle, le quinoléinyle, l'isoquinoléinyle, le dihydroisoquinoléinyle, le benzoxazinyle, le quinoxalinyle, le benzopyranyle, le benzimidazolyle, l'indolyle, l'imidazothiazolyle, l'imidazothiadiazolyle, l'imidazopyridyle, le benzothiazinyle, le benzoquinoxalinyle et l'imidazobenzothiazolyle et les restes inférieurs ayant jusqu'à 7 atomes de carbone, leurs dérivés d'esters et d'éthers hydrolysables dans les conditions physiologiques et leurs sels pharmaceutiquement utilisables.

3. Composés selon la revendication 2 où Rb se trouve en position para par rapport à Ra.

4. Composés selon la revendication 2 ou 3 où Ra représente le nitro.

5. Composés selon l'une des revendications 2 à 4 où Rb représente l'hydrogène, le chlore ou le fluor.

6. Composés selon la revendication 5 où Rb représente l'hydrogène.

7. Composés selon l'une des revendications 2 à 6 où Rc' représente le groupe -CO-R¹¹ et R¹¹ représente un groupe phényle non substitué ou mono- ou disubstitué par un halogène, le trifluorométhyle, un nitro, un amino, un mono- ou di(alkyle inférieur) amino, un alkyle inférieur, un alcoxy inférieur, un alkyle inférieur thio, un alcynoyle inférieur, un alcoxy inférieur, un alkyle inférieur thio, un alcanoyle inférieur, un alcoxy inférieur carbonyle, un carboxy, un hydroxy, un cyano, un alcanoyle inférieur oxy, un carbamoyle, un mono- ou di(alkyle inférieur)carbamoyle, un alkylène inférieur dioxy, un alcanoyle inférieur amino ou un alcoxy inférieur carbonylamino ou un groupe hétérocyclique lié par l'intermédiaire d'un atome de carbone, non substitué ou mono-, di- ou trisubstitué par un halogène, le trifluorométhyle, un nitro, un carboxy, un amino, un arylamino, un alkyle inférieur, un alcoxy inférieur, un hydroxy, un alcoxy inférieur carbonyle, un alcanoyle inférieur, un alcanoyle inférieur oxy, un oxo, un alkylène inférieur dioxy, un mercapto, un alkyle inférieur thio, un alkyle inférieur amino, un di(alkyle inférieur) amino, un cycloalkyle en C₃₋₇ amino, un bicycloalkyle en C₈₋₁₀ amino, un alcanoyle inférieur amino, un alcoxy inférieur carbonylamino, un carbamoyle, un mono- ou di(alkyle inférieur) carbamoyle, un cyano, un aryle, un aryle alkyle inférieur, un aryle alkyle inférieur amino; un hétéroaryle, un hétéroaryle alkyle inférieur, un hétéroarylamino ou un cycloalkyle en C₃₋₇, ledit groupe hétérocyclique étant choisi dans le groupe constitué par le pyridyle, le pyrazinyle, le triazinyle, le pyrazolyle et l'imidazolyle.

8. Composés selon la revendication 7 où R¹¹ représente un groupe phényle éventuellement mono- ou disubstitué par un halogène, le trifluorométhyle, un cyano, un hydroxy ou un alkyle inférieur ou un groupe pyridyle.

9. La 3,4-dihydroxy-4'-méthyl-5-nitrobenzophénone.

10. La 2'-fluoro-3,4-dihydroxy-5-nitrobenzophénone.

11. La 3,4-dihydroxy-5-nitrophényl-4-pyridylcétone.

12. Composés selon l'une des revendications 2 à 11 destinés à être utilisés comme substances actives thérapeutiques.

13. Composés selon l'une des revendications 1 à 11 destinés à être utilisés comme substances actives inhibant la catéchol-O-méthyl-transférase.

14. Procédé pour la préparation des composés selon l'une des revendications 2 à 11, de leurs dérivés d'esters et d'éthers hydrolysables dans les conditions physiologiques et de leurs sels pharmaceutiquement acceptables, caractérisé
a) en ce que l'on clive, dans un composé de formule générale 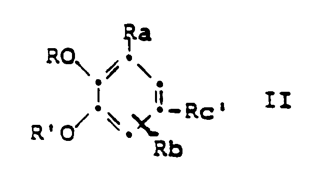 où l'un des symboles R et R' représente un alkyle inférieur et l'autre des symboles R et R' représente l'hydrogène ou un alkyle inférieur et Ra, Rb et Rc' ont la signification donnée dans la revendication 2,
le ou les groupe(s) alkyle(s) inférieurs éther, ou
b) en ce que l'on fait réagir un composé de formule générale 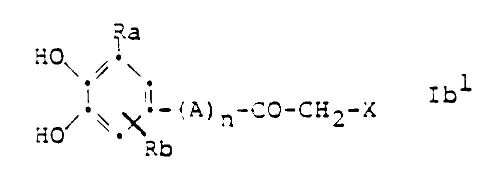 où X représente un groupe partant et Ra, Rb, A et n ont la signification donnée dans la revendication 2,
avec un thioamide, une thiourée, un hydrazide thiocarboxylique, un thiosemicarbazide, une amidine, une guanidine, une amidrazone, une aminoguanidine, une amidine cyclique, une 1,2-diamine, un 1,2-aminothiol ou un 1,2-aminoalcool et, si désiré, en ce que l'on déshydrogène le produit de condensation cyclique obtenu, ou
c) en ce que l'on fait réagir un composé de formule générale 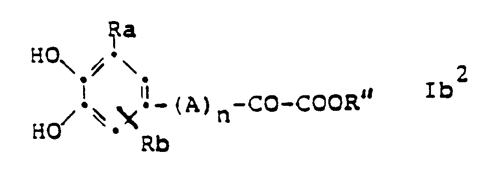 où R'' représente un alkyle inférieur et Ra, Rb, A et n ont la signification donnée dans la revendication 2,
avec une 1,2-diamine, un 1,2-aminothiol, un 1,2-aminoalcool, un semicarbazide, un thiosemicarbazide, une amidrazone ou une aminoguanidine et, si désiré, en ce que l'on déshydrogène le produit de condensation cyclique obtenu, ou
d) en ce que l'on fait réagir un composé de formule Ib¹ ci-dessus avec un composé β-aminocarbonyle, ou
e) en ce que l'on transforme dans un composé de formules générales 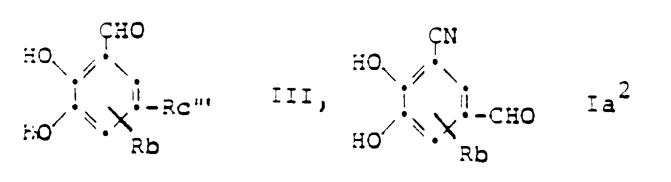 ou 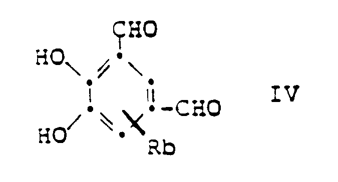 où Rc''' représente un nitro, un cyano ou le groupe -(A)ₙ-R¹² et R¹² le groupe -COR³¹, un aryle ou un hétéroaryle et Ra, Rb, A, n et R³¹ ont la signification donnée dans la revendication 2
ou dans l'un de ses dérivés di-O-alcanoyle inférieur le ou les groupe(s) carboxaldéhyde(s) en groupe(s) cyano, ou
f) en ce que l'on fait réagir un dérivé di-O-alcanoyle inférieur d'un acide carboxylique de formule générale 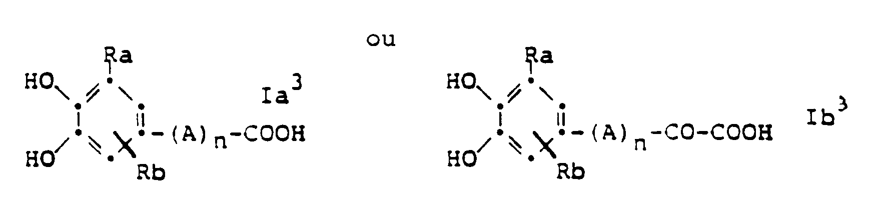 où Ra, Rb, A et n ont la signification donnée dans la revendication 2,
en présence d'un agent de condensation, ou un dérivé réactif d'un dérivé di-O-alcanoyle inférieur d'un acide carboxylique de formule Ia³ ou Ib³, avec un composé de formule générale
HO-R⁵ V ou HNR⁶R⁷ VI
où R⁵ représente un reste hydrocarboné inférieur, saturé ou partiellement insaturé, non substitué ou mono- ou disubstitué par un hydroxy, un cyano, un nitro, un halogène, un amino, un alkyle inférieur amino, un di(alkyle inférieur) amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur) carbamoyle, un alkylène inférieur dioxy, le trifluorométhyle, un carboxy, un alcanoyle inférieur amino, un alcoxy inférieur carbonylamino ou un alkyle inférieur thio, R⁶ représente l'hydrogène ou un alkyle inférieur et R⁷ représente l'hydrogène ou un reste hydrocarboné inférieur, saturé ou partiellement insaturé, non substitué ou mono- ou disubstitué par un hydroxy, un cyano, un nitro, un halogène, un amino, un alkyle inférieur amino, un di(alkyle inférieur) amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur) carbamoyle, un alkylène inférieur dioxy, le trifluorométhyle, un carboxy, un alcanoyle inférieur amino, un alcoxy inférieur carbonylamino ou un alkyle inférieur thio ou R⁶ et R⁷ représentent ensemble avec l'atome d'azote, un groupe 4-morpholinyle, 1-pyrrolidinyle ou 1-azétidinyle non substitué ou mono- ou disubstitué par un alkyle inférieur, un hydroxy, un alcoxy inférieur, un alcanoyle inférieur oxy, un hydroxyalkyle inférieur, un alcoxy inférieur alkyle, un alcynoyle inférieur oxyalkyle, un alcoxy inférieur carbonyle, un alcanoyle inférieur, un carbamoyle, un mono- ou di(alkyle inférieur) carbamoyle, un oxo et/ou un alkylène inférieur dioxy, ou
g) en ce que l'on hydrolyse un composé de formule Ib² ci-dessus ou de formule générale 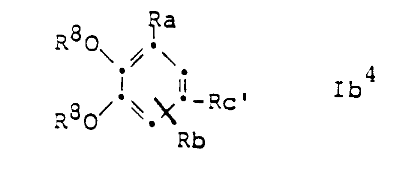 où R⁸ représente un alcanoyle inférieur et Ra, Rb et Rc' ont la signification donnée dans la revendication 2, ou
h) en ce que l'on fait réagir un composé de formule générale 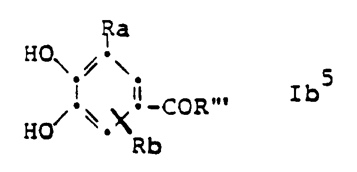 où Ra et Rb ont la signification donnée dans la revendication 2 et R''' représente l'hydrogène ou un alkyle inférieur
ou l'un de ses dérivés di-O-alcanoyle inférieur en présence d'une amine secondaire avec un composé de formule générale
CH₃CO-R²³ VII
où R²³ représente un reste hydrocarboné inférieur, saturé ou partiellement insaturé, non substitué ou mono- ou disubstitué par un hydroxy, un cyano, un nitro, un halogène, un amino, un alkyle inférieur amino, un di(alkyle inférieur) amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur) carbamoyle, un alkylène inférieur dioxy, le trifluorométhyle, un carboxy, un alcanoyle inférieur amino, un alcoxy inférieur carbonylamino ou un alkyle inférieur thio, ou
i) en ce que l'on fait réagir un composé de formule générale 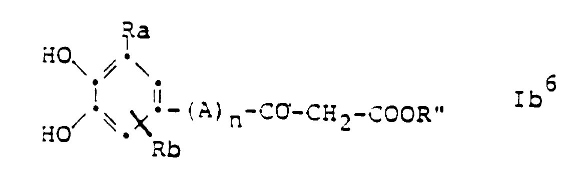 où Ra, Rb, A et n ont la signification donnée dans la revendication 2 et R'' a la signification donnée ci-dessus,
avec une hydrazine ou une amidine, ou
j) en ce que l'on fait réagir un composé de formule Ib ci-dessus où m est égal à 1 et Q représente le groupe -CO-, avec un composé de formule générale
H₂N-(Z)ₚ-R⁴ VIII
où Z, p et R⁴ ont la signification donnée dans la revendication 2
et, si désiré,
k) en ce que l'on transforme un composé de formule Ib ci-dessus en l'un de ses dérivés d'esters ou d'éthers hydrolysables dans les conditions physiologiques ou en l'un de ses sels pharmaceutiquement acceptables.

15. Médicament contenant un composé selon l'une des revendications 1 à 11 et un support thérapeutiquement inerte.

16. Médicament inhibant la catéchol-O-méthyltransférase, contenant un composé selon l'une des revendications 1 à 11 et un support thérapeutiquement inerte.

17. Médicament pour le traitement de la maladie de Parkinson et du parkinsonisme, contenant de la L-Dopa, un inhibiteur décarboxylase périphérique, un composé selon l'une des revendications 1 à 11 et un support thérapeutiquement inerte.

18. Kit de médicaments destiné au traitement de la maladie de Parkinson et du parkinsonisme, constitué d'un médicament selon la revendication 16 et d'un médicament contenant de la L-Dopa, un inhibiteur décarboxylase périphérique et un support thérapeutiquement inerte.

19. Utilisation des composés selon l'une des revendications 1 à 11 pour la préparation de médicaments inhibant la catéchol-O-méthyl-transférase.

20. Utilisation des composés selon l'une des revendications 1 à 11 pour la préparation de médicaments pour le traitement de la maladie de Parkinson et du parkinsonisme en combinaison avec la L-Dopa et un inhibiteur décarboxylase périphérique.

21. Utilisation des composés selon l'une des revendications 1 à 11 pour la préparation de kits de médicaments selon la revendication 18.

## Revendications (Revendications pour l'(les) Etat(s) contractant(s) suivant(s): AT, ES, GR)

1. Procédé pour la préparation des composés de formule générale 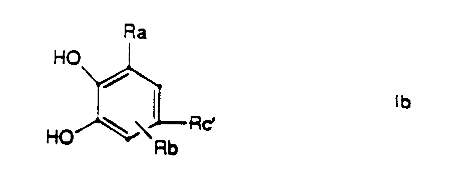 où Ra représente un nitro ou un cyano, Rb représente l'hydrogène ou un halogène, Rc' représente un nitro, un cyano ou le groupe -(A)ₙ-(Q)ₘ-R¹¹ où -(A')ₙ-Q-R²¹, A représentant le vinylène éventuellement substitué par un alkyle inférieur, n étant égal à O ou 1, m étant égal à 0 ou 1, R¹¹ représentant le groupe -COR³¹, un aryle ou un hétéroaryle, R²¹ représentant un reste hydrocarboné inférieur, saturé ou partiellement insaturé, non substitué ou mono- ou disubstitué par un hydroxy, un cyano, un nitro, un halogène, un amino, un alkyle inférieur amino, un di(alkyle inférieur)amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur) carbamoyle, un alkylène inférieur dioxy, le trifluorométhyle, un carboxy, un alcanoyle inférieur amino, un alcoxy inférieur carbonylamino ou un alkyle inférieur thio, R³¹ représentant un hydroxy, un amino, un reste hydrocarboné inférieur, saturé ou partiellement insaturé, lié par l'intermédiaire d'un atome d'oxygène ou d'un groupe imino ou d'un groupe alkyle inférieur imino, non substitué ou mono- ou disubstitué par un hydroxy, un cyano, un nitro, un halogène, un amino, un alkyle inférieur amino, un di(alkyle inférieur)amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur) carbamoyle, un alkylène inférieur dioxy, le trifluorométhyle, un carboxy, un alcanoyle inférieur amino, un alcoxy inférieur carbonylamino ou un alkyle inférieur thio ou un groupe 4-morpholinyle, 1-pyrrolidinyle ou 1-azétidinyle non substitué ou mono- ou disubstitué par un alkyle inférieur, un hydroxy, un alcoxy inférieur, un alcanoyle inférieur oxy, un hydroxyalkyle inférieur, un alcoxy inférieur alkyle, un alcanoyle inférieur oxyalkyle, un alcoxy inférieur carbonyle, un alcanoyle inférieur, un carbamoyle, un mono- ou di(alkyle inférieur) carbamoyle, un oxo et/ou un alkylène inférieur dioxy, Q représentant le groupe -CO- ou 〉C=N-(Z)ₚ-R⁴, Z représentant un atome d'oxygène ou un groupe imino, p étant égal à 0 ou 1 et R⁴ représentant l'hydrogène ou un reste hydrocarboné inférieur, saturé ou partiellement insaturé, et éventuellement lié par l'intermédiaire d'un groupe carbonyle, ledit reste hydrocarboné étant non substitué ou mono- ou disubstitué par un hydroxy, un cyano, un nitro, un halogène, un amino, un alkyle inférieur amino, un di(alkyle inférieur)amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur)carbamoyle, un alkylène inférieur dioxy, le trifluorométhyle, un carboxy, un alcanoyle inférieur amino, un alcoxy inférieur carbonylamino ou un alkyle inférieur thio, Ra représentant un cyano, lorsque Rc' représente un cyano ou un nitro et R³¹ ayant une signification différente de l'hydroxy, lorsque n est égal à 0 et un aryle signifiant un groupe phényle ou naphtyle non substitué ou mono- ou disubstitué par un halogène, le trifluorométhyle, un nitro, un amino, un mono- ou di(alkyle inférieur) amino, un alkyle inférieur, un alcoxy inférieur, un alkyle inférieur thio, un alcanoyle inférieur, un alcoxy inférieur carbonyle, un carboxy, un hydroxy, un cyano, un alcanoyle inférieur oxy, un carbamoyle, un mono- ou di(alkyle inférieur) carbamoyle, un alkylène inférieur dioxy, un alcanoyle inférieur amino ou un alcoxy inférieur carbonylamino, un hétéroaryle signifiant un groupe hétérocyclique lié par l'intermédiaire d'un atome de carbone, non substitué ou mono-, di- ou trisubstitué par un halogène, le trifluorométhyle, un nitro, un carboxy, un amino, un arylamino, un alkyle inférieur, un alcoxy inférieur, un hydroxy, un alcoxy inférieur carbonyle, un alcanoyle inférieur, un alcanoyle inférieur oxy, un oxo, un alkylène inférieur dioxy, un mercapto, un alkyle inférieur thio, un alkyle inférieur amino, un di(alkyle inférieur) amino, un cycloalkyle en C₃₋₇ amino, un bicycloalkyle en C₈₋₁₀ amino, un alcanoyle inférieur amino, un alcoxy inférieur carbonylamino, un carbamoyle, un mono- ou di(alkyle inférieur) carbamoyle, un cyano, un aryle, un aryle alkyle inférieur, un aryle alkyle inférieur amino, un hétéroaryle, un hétéroaryle alkyle inférieur, un hétéroarylamino ou un cycloalkyle en C₃₋₇, ledit groupe hétérocyclique étant choisi dans le groupe constitué par le pyridyle, le pyrazinyle, le triazinyle, lethiadiazinyle, le thiazolyle, l'oxazolyle, l'oxadiazolyle, le pyrazolyle, le tétrazolyle, l'imidazolyle, le thiényle, le quinoléinyle, l'isoquinoléinyle, le dihydroisoquinoléinyle, le benzoxazinyle, le quinoxalinyle, le benzopyranyle, le benzimidazolyle, l'indolyle, l'imidazothiazolyle, l'imidazothiadiazolyle, l'imidazopyridyle, le benzothiazinyle, le benzoquinoxalinyle et l'imidazobenzothiazolinyle et les restes "inférieurs" ayant jusqu'à 7 atomes de carbone, de leurs dérivés d'esters et d'éthers hydrolysables dans les conditions physiologiques et de leurs sels pharmaceutiquement utilisables, caractérisé
a) en ce que l'on clive, dans un composé de formule générale 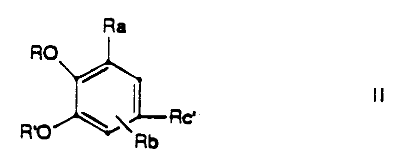 où l'un des symboles R et R' représente un alkyle inférieur et l'autre des symboles R et R' représente l'hydrogène ou un alkyle inférieur et Ra, Rb et Rc' ont la signification donnée ci-dessus,
le ou les groupe(s) alkyle(s) inférieurs éther, ou
b) en ce que l'on fait réagir un composé de formule générale 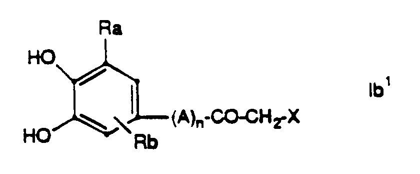 où X représente un groupe partant et Ra, Rb, A et n ont la signification donnée ci-dessus,
avec un thioamide, une thiourée, un hydrazide thiocarboxylique, un thiosemicarbazide, une amidine, une guanidine, une amidrazone, une aminoguanidine, une amidine cyclique, une 1,2-diamine, un 1,2-aminothiol ou un 1,2-aminoalcool et, si désiré, en ce que l'on déshydrogène le produit de condensation cyclique obtenu, ou
c) en ce que l'on fait réagir un composé de formule générale 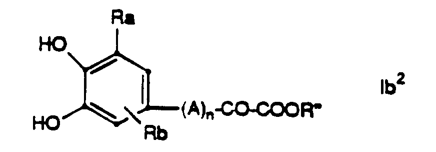 où R'' représente un alkyle inférieur et Ra, Rb, A et n ont la signification donnée ci-dessus,
avec une 1,2-diamine, un 1,2-aminothiol, un 1,2-aminoalcool, un semicarbazide, un thiosemicarbazide, une amidrazone ou une aminoguanidine et, si désiré, en ce que l'on déshydrogène le produit de condensation cyclique obtenu, ou
d) en ce que l'on fait réagir un composé de formule Ib¹ ci-dessus avec un composé β-aminocarbonyle, ou
e) en ce que l'on transforme dans un composé de formules générales 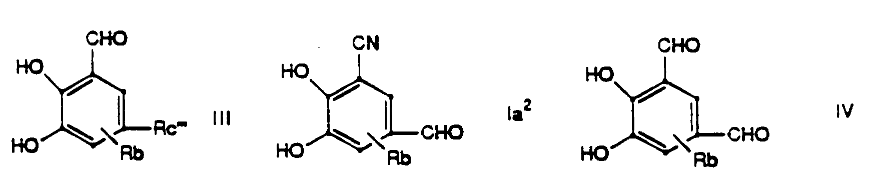 où Rc''' représente un nitro, un cyano ou le groupe -(A)ₙ-R¹² et R¹² le groupe -COR³¹, un aryle ou un hétéroaryle et Ra, Rb, A, n et R³¹ ont la signification donnée ci-dessus,
ou dans l'un de ses dérivés di-O-alcanoyle inférieur le ou les groupe(s) carboxaldéhyde(s) en groupe(s) cyano, ou
f) en ce que l'on fait réagir un dérivé di-O-alcanoyle inférieur d'un acide carboxylique de formule générale 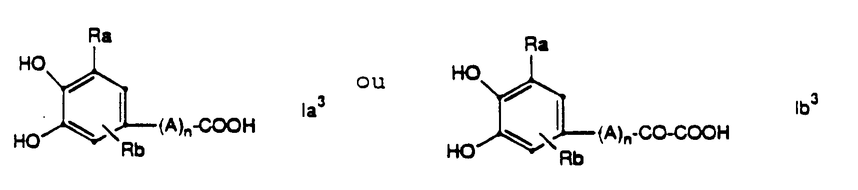 où Ra, Rb, A et n ont la signification donnée ci-dessus, en présence d'un agent de condensation, ou un dérivé réactif d'un dérivé di-O-alcanoyle inférieur d'un acide carboxylique de formule Ia³ ou Ib³, avec un composé de formule générale
HO-R⁵ V ou HNR⁶R⁷ VI
où R⁵ représente un reste hydrocarboné inférieur, saturé ou partiellement insature, non substitué ou mono- ou disubstitué par un hydroxy, un cyano, un nitro, un halogène, un amino, un alkyle inférieur amino, un di(alkyle inférieur) amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur) carbamoyle, un alkylène inférieur dioxy, le trifluorométhyle, un carboxy, un alcanoyle inférieur amino, un alcoxy inférieur carbonylamino ou un alkyle inférieur thio, R⁶ représente l'hydrogène ou un alkyle inférieur et R⁷ représente l'hydrogène ou un reste hydrocarboné inférieur, saturé ou partiellement insaturé, non substitué ou mono- ou disubstitué par un hydroxy, un cyano, un nitro, un halogène, un amino, un alkyle inférieur amino, un di(alkyle inférieur) amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur) carbamoyle, un alkylène inférieur dioxy, le trifluorométhyle, un carboxy, un alcanoyle inférieur amino, un alcoxy inférieur carbonylamino ou un alkyle inférieur thio ou R⁶ et R⁷ représentent ensemble avec l'atome d'azote, un groupe 4-morpholinyle, 1-pyrrolidinyle ou 1-azétidinyle non substitué ou mono- ou disubstitué par un alkyle inférieur, un hydroxy, un alcoxy inférieur, un alcanoyle inférieur oxy, un hydroxyalkyle inférieur, un alcoxy inférieur alkyle, un alcanoyle inférieur oxyalkyle, un alcoxy inférieur carbonyle, un alcanoyle inférieur, un carbamoyle, un mono- ou di(alkyle inférieur) carbamoyle, un oxo et/ou un alkylène inférieur dioxy, ou
g) en ce que l'on hydrolyse un composé de formule Ib² ci-dessus ou de formule générale 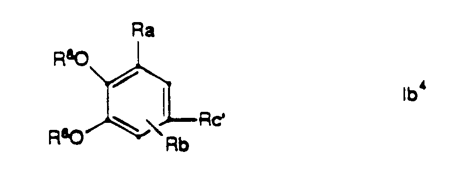 où R⁸ représente un alcanoyle inférieur et Ra, Rb et Rc' ont la signification donnée ci-dessus, ou
h) en ce que l'on fait réagir un composé de formule générale 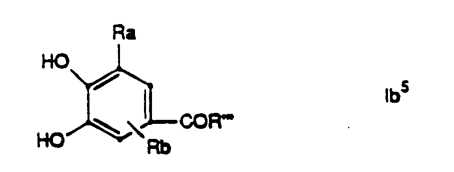 où Ra et Rb ont la signification donnée ci-dessus et R''' représente l'hydrogène ou un alkyle inférieur
ou l'un de ses dérivés di-O-alcanoyle inférieur en présence d'une amine secondaire avec un composé de formule générale
CH₃CO-R²³ VII
où R²³ représente un reste hydrocarboné inférieur, saturé ou partiellement insaturé, non substitué ou mono- ou disubstitué par un hydroxy, un cyano, un nitro, un halogène, un amino, un alkyle inférieur amino, un di(alkyle inférieur) amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyl inférieur) carbamoyle, un alkylène inférieur dioxy, le trifluorométhyle, un carboxy, un alcanoyle inférieur amino, un alcoxy inférieur carbonylamino ou un alkyle inférieur thio, ou
i) en ce que l'on fait réagir un composé de formule générale 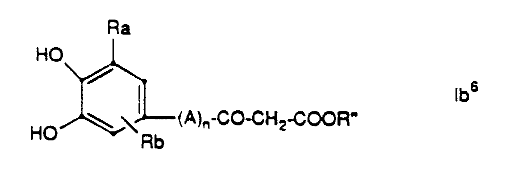 où Ra, Rb, A, n et R'' ont la signification donnée ci-dessus,
avec une hydrazine ou une amidine, ou
j) en ce que l'on fait réagir un composé de formule Ib ci-dessus où m est égal à 1 et Q représente le groupe -CO-, avec un composé de formule générale
H₂N-(Z)ₚ-R⁴ VIII
où Z, p et R⁴ ont la signification donnée ci-dessus
et, si désiré,
k) en ce que l'on transforme un composé de formule Ib ci-dessus en l'un de ses dérivés d'esters ou d'éthers hydrolysables dans les conditions physiologiques ou en l'un de ses sels pharmaceutiquement acceptables.

2. Procédé selon la revendication 1 où Rb se trouve en position para par rapport à Ra.

3. Procédé selon la revendication 1 ou 2 où Ra représente le nitro.

4. Procédé selon l'une des revendications 1 à 3 où Rb représente l'hydrogène, le chlore ou le fluor.

5. Procédé selon la revendication 4 où Rb représente l'hydrogène.

6. Procédé selon l'une des revendications 1 à 5 où Rc' représente le groupe -CO-R¹¹ et R¹¹ représente un groupe phényle non substitué ou mono- ou disubstitué par un halogène, le trifluorométhyle, un nitro, un amino, un mono- ou di(alkyle inférieur)amino, un alkyle inférieur, un alcoxy inférieur, un alkyle inférieur thio, un alcynoyle inférieur, un alcoxy inférieur, un alkyle inférieur thio, un alcanoyle inférieur, un alcoxy inférieur carbonyle, un carboxy, un hydroxy, un cyano, un alcanoyle inférieur oxy, un carbamoyle, un mono- ou di(alkyle inférieur)carbamoyle, un alkylène inférieur dioxy, un alcanoyle inférieur amino ou un alcoxy inférieur carbonylamino ou un groupe hétérocyclique lié par l'intermédiaire d'un atome de carbone, non substitué ou mono-, di- ou trisubstitué par un halogène, le trifluorométhyle, un nitro, un carboxy, un amino, un arylamino, un alkyle inférieur, un alcoxy inférieur, un hydroxy, un alcoxy inférieur carbonyle, un alcanoyle inférieur, un alcanoyle inférieur oxy, un oxo, un alkylène inférieur dioxy, un mercapto, un alkyle inférieur thio, un alkyle inférieur amino, un di(alkyle inférieur)amino, un cycloalkyle en C₃₋₇ amino, un bicycloalkyle en C₈₋₁₀ amino, un alcanoyle inférieur amino, un alcoxy inférieur carbonylamino, un carbamoyle, un mono- ou di(alkyle inférieur) carbamoyle, un cyano, un aryle, un aryle alkyle inférieur, un aryle alkyle inférieur amino, un hétéroaryle, un hétéroaryle alkyle inférieur, un hétéroarylamino ou un cycloalkyle en C₃₋₇, ledit groupé hétérocyclique étant choisi dans le groupe constitué par le pyridyle, le pyrazinyle, le triazinyle, le pyrazolyle et l'imidazolyle.

7. Procédé selon la revendication 6 où R¹¹ représente un groupe phényle éventuellement mono- ou disubstitué par un halogène, le trifluorométhyle, un cyano, un hydroxy ou un alkyle inférieur ou un groupe pyridyle.

8. Procédé selon la revendication 1, caractérisé en ce que l'on prépare la 3,4-dihydroxy-4'-méthyl-5-nitrobenzophénone.

9. Procédé selon la revendication 1, caractérisé en ce que l'on prépare la 2'-fluoro-3,4-dihydroxy-5-nitrobenzophénone.

10. Procédé selon la revendication 1, caractérisé en ce que l'on prépare la 3,4-dihydroxy-5-nitrophényle-4-pyridylcétone.

11. Utilisation des composés de formule générale 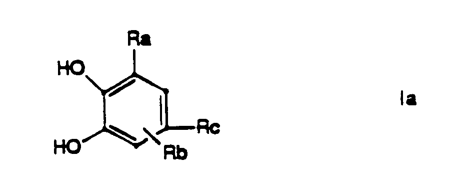 où Ra représente un nitro ou un cyano, Rb représente l'hydrogène ou un halogène, Rc représente un halogène, un nitro, un cyano ou le groupe -(A)ₙ-(Q)ₘ-R¹ ou -(A)ₙ-Q-R², A représentant le vinylène éventuellement substitué par un alkyle inférieur, n est égal à 0 ou 1, m est égal à 0 ou 1, R¹ représentant le groupe -COR³, un groupe aryle ou un hétéroaryle, R² représentant l'hydrogène ou un reste hydrocarboné inférieur, saturé ou partiellement insaturé, non substitué ou mono- ou disubstitué par un hydroxy, un cyano, un nitro, un halogène, un amino, un alkylamino inférieur, un di(alkyle inférieur) amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di (alkyle inférieur) carbamoyle, un alkylène inférieur dioxy, le trifluorométhyle, un carboxy, un alcanoyle inférieur amino, un alcoxy inférieur carbonylamino ou un alkyle inférieur thio, R³ représentant un hydroxy, un amino, un reste hydrocarboné inférieur, saturé ou partiellement insaturé, lié par l'intermédiaire d'un atome d'oxygène ou d'un groupe imino ou d'un groupe alkylimino, ledit reste hydrocarboné étant non substitué ou mono- ou disubstitué par un hydroxy, un cyano, un nitro, un halogène, un amino, un alkyle inférieur amino, un di(alkyle inférieur)amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur) carbamoyle, un alkylène inférieur dioxy, le trifluorométhyle, un carboxy, un alcanoyle inférieur amino, un alcoxy inférieur carbonylamino ou un alkyle inférieur thio ou un groupe 4-morpholinyle, 1-pyrrolidinyle ou 1-azétidinyle non substitué ou mono- ou disubstitué par un alkyle inférieur, un hydroxy, un alcoxy inférieur, un alcanoyle inférieur oxy, un hydroxyalkyle inférieur, un alcoxy inférieur alkyle, un alcanoyle inférieur oxyalkyle, un alcoxy inférieur carbonyle, un alcanoyle inférieur, un carbamoyle, un mono- ou di(alkyle inférieur) carbamoyle, un oxo et/ou un alkylène inférieur dioxy, Q représentant le groupe -CO- ou 〉C=N-(Z)ₚ-R⁴, Z étant un atome d'oxygène ou un groupe imino, p étant égal à 0 ou 1, et R⁴ représentant l'hydrogène ou un reste hydrocarboné inférieur, saturé ou partiellement insaturé, éventuellement lié par un groupe carbonyle, ledit reste hydrocarboné étant non substitué ou mono- ou disubstitué par un hydroxy, un cyano, un nitro, un halogène, un amino, un alkyle inférieur amino, un di(alkyle inférieur)amino, un alcoxy inférieur, un alcoxy inférieur carbonyle, un aryle, un arylaminocarbonyle, un arylcarbonyle, un arylcarbonylamino, un alcanoyle inférieur oxy, un alcanoyle inférieur, un carbamoyle, un mono- ou di(alkyle inférieur)carbamoyle, un alkylène inférieur dioxy, le trifluorométhyle, un carboxy, un alcanoyle inférieur amino, un alcoxy inférieur carbonylamino ou un alkyle inférieur thio, un aryle signifiant un groupe phényle ou naphtyle non substitué ou mono- ou disubstitué par un halogène, le trifluorométhyle, un nitro, un amino, un mono- ou di(alkyle inférieur)amino, un alkyle inférieur, un alcoxy inférieur, un alkyle inférieur thio, un alcanoyle inférieur, un alcoxy inférieur carbonyle, un carboxy, un hydroxy, un cyano, un alcanoyle inférieur oxy, un carbamoyle, un mono- ou di(alkyle inférieur) carbamoyle, un alkylène inférieur dioxy, un alcanoyle inférieur amino ou un alcoxy inférieur carbonylamino, un hétéroaryle signifiant un groupe hérérocyclique lié par l'intermédiaire d'un atome de carbone, non substitué ou mono-, di- ou trisubstitué par un halogène, le trifluorométhyle, un nitro, un carboxy, un amino, un arylamino, un alkyle inférieur, un alcoxy inférieur, un hydroxy, un alcoxy inférieur carbonyle, un alcanoyle inférieur, un alcanoyle inférieur oxy, un oxo, un alkylène inférieur dioxy, un mercapto, un alkyle inférieur thio, un alkyle inférieur amino, un di(alkyle inférieur)amino, un cycloalkyle en C₃₋₇ amino, un bicycloalkyle en C₈₋₁₀ amino, un alcanoyle inférieur amino, un alcoxy inférieur carbonylamino, un carbamoyle, un mono- ou di(alkyle inférieur) carbamoyle, un cyano, un aryle, un aryl alkyle inférieur, un aryle alkyle inférieur amino, un hétéroaryle, un hétéroaryle alkyle inférieur, un hétéroarylamino ou un cycloalkyle en C₃₋₇, ledit groupe hétérocyclique étant choisi dans le groupe constitué par le pyridyle, le pyrazinyle, le triazinyle, le thiadiazinyle, le thiazolyle, l'oxazolyle, l'oxadiazolyle, le pyrazolyle, le tétrazolyle, l'imidazolyle, le thiényle, le quinoléinyle, l'isoquinoléinyle, le dihydroisoquinoléinyle, le benzoxazinyle, le quinoxalinyle, le benzopyranyle, le benzimidazolyle, l'indolyle, l'imidazothiazolyle, l'imidazothiadiazolyle, l'imidazopyridyle, le benzothiazinyle, le benzoquinoxalinyle et l'imidazobenzothiazinyle et des restes "inférieurs" ayant jusqu'à 7 atomes de carbone et de leurs dérivés d'esters et d'éthers hydrolysables dans les conditions physiologiques et de leurs sels pharmaceutiquement acceptables pour la préparation de médicaments inhibant la catéchol-O-méthyl-transférase.

12. Utilisation des composés de formule Ia définie dans la revendication 11 pour la préparation de médicaments pour le traitement de la maladie de Parkinson et du parkinsonisme en combinaison avec la L-Dopa et un inhibiteur décarboxylase périphérique.

13. Utilisation des composés de formule Ia définie dans la revendication 11 pour la préparation de kits de médicaments constitués d'un médicament contenant un composé de formule Ia définie dans la revendication 11 et un support thérapeutiquement inerte et d'un médicament contenant de la L-Dopa, un inhibiteur décarboxylase périphérique et un support thérapeutiquement inerte.
